# EUROPEAN PATENT SPECIFICATION

(11) **EP 0 307 142 B1**
(45) Date of publication and mention of the grant of the patent: **14.09.1994**
(21) Application number: 88308127.5
(22) Date of filing: 01.09.1988
(51) Int. Cl.: C07D 417/04, C07D 453/02, C07D 471/08, C07D 487/08, A61K 31/41, A61K 31/44, A61K 31/435

(54) **Thiadiazoles useful in the treatment of senile dementia**
Thiadiazole, verwendbar zur Behandlung von altersbedingter Demenz
Thiadiazoles utilisables pour le traitement de la démence sénile

(30) Priority: 10.09.1987 GB 8721342; 27.01.1988 GB 8801758; 23.05.1988 GB 8812145; 20.07.1988 GB 8817311
(43) Date of publication of application: 15.03.1989
(73) Proprietor: MERCK SHARP & DOHME LTD., Hoddesdon Hertfordshire EN11 9NU (GB)
(72) Inventor: Baker, Raymond, Much Hadham Hertfordshire (GB); Saunders, John, Bishops Stortford Hertfordshire (GB); MacLeod, Angus M., Bishops Stortford Hertfordshire (GB); Merchant, Kevin, Bishops Stortford Hertfordshire (GB)
(74) Representative: Cole, William Gwyn

(56) References cited:
- EP-A- 0 020 161
- EP-A- 0 239 309
- EP-A- 0 261 763
- EP-A- 0 261 763
- EP-B- 0 018 497
- DE-A- 2 247 266
- DE-A- 3 038 636
- DE-A- 3 422 861
- US-A- 4 301 069
- CHEMICAL ABSTRACTS, Vol 75, No.19, May 8 1972 & Coll. Czech Chem.Commun. 1971 36 (12), 4087-91

## Description

The present invention relates to a class of substituted thiadiazole compounds which stimulate central muscarinic acetylcholine receptors and therefore are useful in the treatment of neurological and mental illnesses whose clinical manifestations are due to cholinergic deficiency. Such diseases include presenile and senile dementia (also known as Alzheimer's disease and senile dementia of the Alzheimer type respectively), Huntington's chorea, tardive dyskinesia, hyperkinesia, mania and Tourette Syndrome. Alzheimer's disease, the most common dementing illness, is a slowly progressive neurological disorder characterised by marked deficits in cognitive functions including memory, attention, language and visual perception capabilities. The compounds of this invention are also useful analgesic agents and therefore useful in the treatment of severe painful conditions such as rheumatism, arthritis and terminal illness.

Compounds capable of enhancing muscarinic cholinergic transmission in the cortex should be beneficial in reversing the cholinergic deficiency in Alzheimer's disease and other diseases related to cholinergic dysfunction. However, most muscarinic ligands, including acetylcholine itself, are quaternary ammonium compounds incapable of penetrating the blood-brain barrier to any clinically significant extent following peripheral (e.g. oral) administration. Such agents fail to stimulate the desired central sites but instead induce undesired side-effects mediated exclusively by peripherally-located muscarinic acetylcholine receptors.

The thiadiazole compounds of the present invention stimulate cholinergic transmission but, being either secondary or tertiary amines with physiochemical properties (lipophilicity and pKa) consistent with CNS penetrability, can stimulate those central sites implicated in neurodegenerative disorders. It is believed that the enhancement of cholinergic transmission demonstrated by the compounds of this invention is achieved either directly by stimulating postsynaptic receptors, or indirectly by potentiating acetylcholine release.

The present invention provides a thiadiazole, or a salt or prodrug thereof, which thiadiazole is substituted on one of the ring carbon atoms thereof with a non-aromatic azacyclic or azabicyclic ring system; and substituted on the other ring carbon with a substituent of low lipophilicity, or a hydrocarbon substituent.

Although a class of thiadiazole compounds having a pyridyl substituent on a ring carbon atom are disclosed in European Patent Specification No. 116515 as pesticides, there is no suggestion therein of any non-aromatic ring, or of any activity other than pesticidal activity.

In addition, EP-A-0261763, which was published on 30 March 1988, describes a class of compounds which includes thiadiazoles substituted on the thiadiazole ring by a group of formula 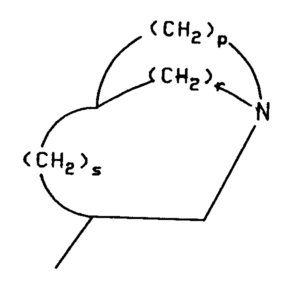
in which p represents an integer of 2 to 4; r represents an integer of 1 or 2; and s represents 0 or 1; such that when (p,r,s) is (2,2,0) or (2,2,1) the thiadiazole nucleus is optionally C-substituted by a methyl group, and when (p,r,s) is (2,1,0), (2,1,1) or (3,1,0) the thiadiazole nucleus is optionally C-substituted by C₁₋₂ alkyl; and wherein such thiadiazoles having two asymmetric centres have the exo stereochemical configuration, i.e. the configuration in which the thiadiazole ring and the (CH₂)ᵣ bridge are on the same side of the plane of the molecule which contains both bridgehead atoms and the ring carbon bonded to the thiadiazole ring. Example 7 of EP-A-0261763 purports to describe the preparation of (±)-3-(3-methyl-1,2,4-thiadiazol-5-yl)-1-azabicyclo[2.2.2]octane; none of the other Examples in this document purports to describe the preparation of a thiadiazole. However, the information in Example 7 of EP-A-0261763 does not in fact enable the title compound of Example 7 of EP-A-0261763 to be prepared and separated. From this it follows that the information contained in EP-A-0261763 does not enable any such thiadiazoles to be prepared and separated.

The novel compounds of this invention may be represented by structural formula IA, IB or IC: 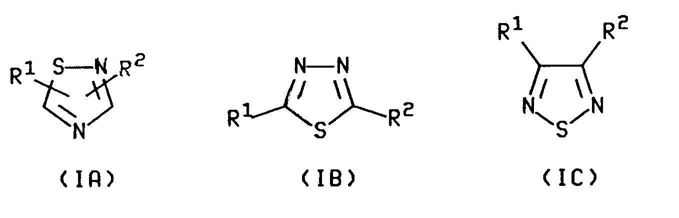
or a salt or prodrug thereof; wherein
R¹ represents a non-aromatic azacyclic or azabicyclic ring system; and R² represents hydrogen, halogen, -CF₃, -OR⁷, -SR⁷, -NR⁷R⁸, -NHOR⁷, -NHNH₂, -CN, -CO₂R⁷, -CONR⁷R⁸, or a substituted or unsubstituted, saturated or unsaturated hydrocarbon group; wherein R⁷ and R⁸ independently represent hydrogen or C₁₋₂ alkyl.

Preferably the thiadiazole ring is a 1,2,4-thiadiazole of formula IA.

The azacyclic or azabicyclic ring system is a non-aromatic ring system containing one nitrogen atom as the sole heteroatom. Suitably the ring system contains from 4 to 10 ring atoms, preferably from 5 to 8 ring atoms. Preferably, the ring system contains a tertiary amino nitrogen atom in a caged structure. The bicyclic systems may be fused, spiro or bridged. Preferably, the nitrogen atom is at a bridgehead in a bicyclic system. Examples of suitable ring systems for the group R¹ include the following: 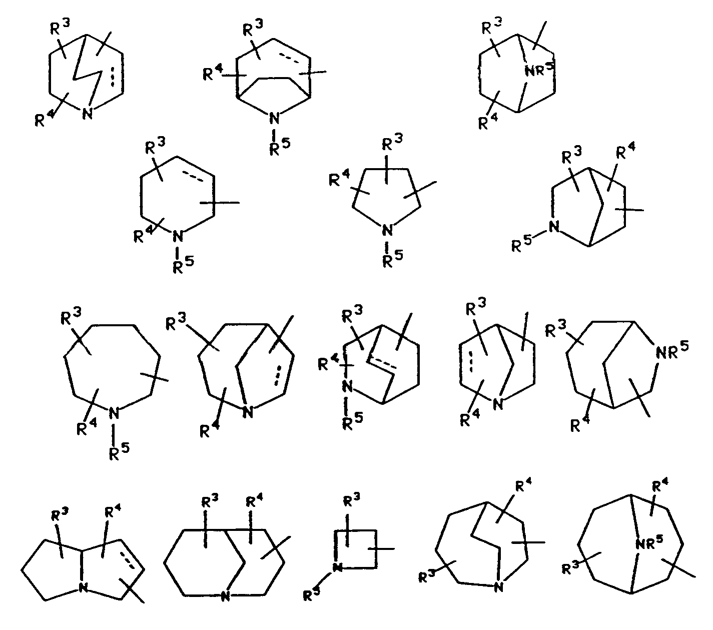
wherein the broken line represents an optional chemical bond;
the substituents R³ and R⁴ may be present at any position, including the point of attachment to the thiadiazole ring, and independently represent hydrogen, C₁₋₄ alkyl, halo, C₁₋₄ alkoxy, hydroxy or carboxy; or R³ and R⁴ together represent carbonyl; and
the group R⁵ represents hydrogen or C₁₋₄ alkyl.

It will be appreciated that the nitrogen atom in the azacyclic or azabicyclic ring system will carry a lone pair of electrons.

Suitably the group R³ is hydrogen or methyl; and R⁴ is hydrogen, methyl or hydroxy. Preferably one or both of R³ and R⁴ is hydrogen.

Preferably the group R⁵ represents hydrogen or methyl.

Suitably the azacyclic or azabicyclic ring system is a pyrrolidine, piperidine, tetrahydropyridine, azanorbornane, quinuclidine, isoquinuclidine or azabicyclo[3.2.1]octane ring system. Preferred values for the azacyclic or azabicyclic ring system are tetrahydropyridine, quinuclidine and 1-azanorbornane, in particular either unsubstituted or substituted with methyl or hydroxy.

The substituent R² on the thiadiazole ring may be a substituent of low lipophilicity. The term "low lipophilicity" is intended to indicate that the group has a Rekker f value (hydrophobic fragment constant; see R. F. Rekker, "The Hydrophobic Fragmental Constant", Elsevier, 1977) of not greater than 1.5. For example, the methyl group has a value of 0.7 and the ethyl group a value of 1.26.

Thus the substituent of low lipophilicity, represented by the group R² in formula IA, IB and IC, may be, for example, hydrogen, halogen, -CF₃, -OR⁷, -SR⁷, -NR⁷R⁸, -NHOR⁷, -NHNH₂, -CN, -CO₂R⁷, -CONR⁷R⁸, C₂₋₅ alkenyl, C₂₋₅ alkynyl, C₁₋₂ alkyl, or C₁₋₂ alkyl substituted with -OR⁷, -NR⁷R⁸, -SR⁷, -CO₂R⁷, -CONR⁷R⁸ or halogen; wherein R⁷ and R⁸ independently represent hydrogen or C₁₋₂ alkyl.

Alternatively the group R² may represent an optionally substituted saturated hydrocarbon group having at least three carbon atoms, or unsaturated hydrocarbon group having at least 6 carbon atoms.

Thus when the group R² is a hydrocarbon substituent, it may be C₁₋₁₅ alkyl, C₂₋₁₅ alkenyl, C₂₋₁₅ alkynyl, aryl or aralkyl. The alkyl, alkenyl or alkynyl groups may be straight, branched or cyclic groups. Suitably the alkyl group comprises from 1 to 6 carbon atoms. The hydrocarbon group may carry one or more substituents. Suitable substituent groups for the hydrocarbon group R² include halogen, -OR⁶, -CF₃, -NR⁶R⁹, -NO₂, optionally substituted aryl, optionally substituted heteroaryl, keto, -SR⁶, -SOR⁶, -SO₂R⁶, -CO₂R⁶ and -CONR⁶R⁹; wherein R⁶ is hydrogen or C₁₋₆ alkyl, and R⁹ is hydrogen, C₁₋₆ alkyl or -COCH₃.

Preferred substituents for the hydrocarbon group R² include phenyl, methylcarbonyloxy, hydroxy and methoxy.

Substituents most suitable for the aryl and heteroaryl groups include chloro, bromo, methoxy, C₁₋₆ alkyl, methoxycarbonyl, trifluoromethyl, nitro and -NR⁶R⁷.

Preferably the group R² is hydrogen, halogen, -CF₃, -OR⁷, -SR⁷, -NR⁷R⁸, -NHNH₂, -CN, -CO₂R⁷, -CONR⁷R⁸, phenyl(C₁₋₃)alkyl, C₃₋₆ cycloalkyl, adamantyl, C₂₋₆ alkenyl, C₂₋₆ alkynyl, C₁₋₆ alkyl, or C₁₋₆ alkyl substituted with -OR⁶, -NHR⁶, -SR⁶, -CO₂R⁶, -CON(R⁶)₂ or halogen. Particular values of the group R² are hydrogen, methyl, ethyl, n-propyl, isopropyl, cyclopropyl, t-butyl, phenyl, benzyl, 1-phenylethyl, adamantyl, amino, methylamino, ethylamino, dimethylamino, methoxy, methylthio, methoxycarbonyl and ethoxycarbonyl. Preferred values of the group R² are hydrogen, methyl, ethyl, cyclopropyl, amino and dimethylamino.

One class of groups R² comprises methyl, ethyl, n-propyl, isopropyl, cyclopropyl or ethenyl, optionally substituted by one or more substituents selected from phenyl, acetoxy, keto, hydroxy and methoxy. A preferred group R² has the structure: 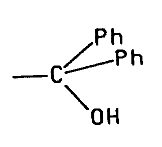
One group of prodrugs of compounds of this invention have a substituent on the thiadiazole ring which is hydrolysable in vivo to an amino group.

Groups which are hydrolysable in vivo to an amino group on the compounds of this invention may be readily ascertained by administering the compound to a human or animal and detecting, by conventional analytical techniques, the presence of the corresponding compound having an amino substituent in the urine of a human or animal. Examples of such groups include, for example, amid and urethane substituents, in particular a group of formula -NH.Q, wherein Q represents CHO, COR or CO₂R, and R represents an optionally substituted hydrocarbon group.

In this context, the hydrocarbon group R includes groups having up to 20 carbon atoms, suitably up to 10 carbon atoms, conveniently up to 6 carbon atoms. Suitable groups R include C₁₋₉ alkyl, C₂₋₆ alkenyl, C₂₋₆ alkynyl, C₃₋₇ cycloalkyl, C₃₋₇ cycloalkyl(C₁₋₆)alkyl, aryl, and aryl(C₁₋₆)alkyl. The alkyl group R may be straight or branched chain and may contain, for example, up to 12 carbon atoms, suitably from 1 to 6 carbon atoms. In particular the group may be substituted methyl, ethyl, n- or iso-propyl, n-, sec-, iso- or tert-butyl, n- or iso-heptyl, or n- or iso-octyl. Suitable cycloalkyl groups include cyclopentyl and cyclohexyl. The aryl group R includes phenyl and naphthyl optionally substituted with up to five, preferably up to three, substituent groups.

One sub-class of compounds within the scope of the present invention is represented by formula II: 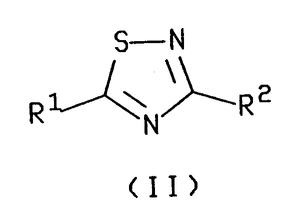
wherein R¹ and R² are as defined above; in particular wherein R¹ represents pyrrolidine, quinuclidine, tetrahydropyridine, piperidine, dehydrotropane, pyrrolizidine, azanorbornane or isoquinuclidine, any of which groups R¹ may be optionally substituted with C₁₋₃ alkyl, or hydroxy; and R² represents hydrogen, C₁₋₆ alkyl (preferably methyl or ethyl), C₃₋₆ cycloalkyl (preferably cyclopropyl), amino or dimethylamino. Preferably R¹ represents quinuclidine, tetrahydropyridine or 1-azanorbornane.

Specific compounds within the scope of the present invention include:
3-[5-(3-methyl-1,2,4-thiadiazol)-yl]pyrrolidine;
1-methyl-3-[5-(3-methyl-1,2,4-thiadiazol)-yl]pyrrolidine;
3-[5-(3-methyl-1,2,4-thiadiazol)-yl]quinuclidine;
3-[5-(3-methyl-1,2,4-thiadiazol)-yl]-1-azabicyclo[2.2.1]heptane;
3-[5-(3-phenyl-1,2,4-thiadiazol)-yl]quinuclidine;
3-[5-(3-cyclopropyl-1,2,4-thiadiazol)-yl]quinuclidine;
3-[5-(3-dimethylamino-1,2,4-thiadiazol)-yl]-1-azabicyclo[2.2.1]heptane;
3-[5-(3-cyclopropyl-1,2,4-thiadiazol)-yl]-1-azabicyclo[2.2.1]heptane;
3-[5-(3-benzyl-1,2,4-thiadiazol)-yl]quinuclidine;
3-[5-(3-t-butyl-1,2,4-thiadiazol)-yl]quinuclidine;
5-[5-(3-methyl-1,2,4-thiadiazol)-yl]-1-azabicyclo[2.2.1]heptan-3-ol;
3-[5-(3-isopropyl-1,2,4-thiadiazol)-yl]quinuclidine;
3-[5-(3-ethyl-1,2,4-thiadiazol)-yl]-1-azabicyclo[2.2.1]heptane;
1-methyl-3-[5-(3-methyl-1,2,4-thiadiazol)-yl]-1,2,5,6-tetrahydropyridine;
3-[5-(3-methyl-1,2,4-thiadiazol)-yl]-1,2,5,6-tetrahydropyridine;
3-[5-(3-(1-hydroxy-1-phenylmethyl)-1,2,4-thiadiazol)-yl]quinuclidine;
3-[5-(3-benzoyl-1,2,4-thiadiazol)-yl]quinuclidine;
3-[5-(3-(1,1-diphenyl-1-hydroxymethyl)-1,2,4-thiadiazol)-yl]quinuclidine;
3-[5-(3-(1,1-diphenyl-1-fluoromethyl)-1,2,4-thiadiazol)-yl]-quinuclidine;
6-[5-(3-methyl-1,2,4-thiadiazol)-yl]-1-azabicyclo[3.2.1]octane;
3-[5-(3-dimethylamino-1,2,4-thiadiazol)-yl]-1,2,5,6-tetrahydropyridine;
3-[5-(3-isopropyl-1,2,4-thiadiazol)-yl]-1-azabicyclo[2.2.1]heptane;
6-[5-(3-cyclopropyl-1,2,4-thiadiazol)-yl]-2-azabicyclo[2.2.2]octane;
3-[5-(3-n-propyl-1,2,4-thiadiazol)-yl]-1-azabicyclo[2.2.1]heptane;
3-[5-(3-methoxy-1,2,4-thiadiazol)-yl]-1-azabicyclo[2.2.1]heptane;
3-[5-(3-methylthio-1,2,4-thiadiazol)-yl]-1-azabicyclo[2.2.1]heptane;
3-[5-(3-n-propyl-1,2,4-thiadiazol)-yl]quinuclidine;
6-[5-(3-isopropyl-1,2,4-thiadiazol)-yl]-2-azabicyclo[2.2.2]octane;
5-[5-(3-isopropyl-1,2,4-thiadiazol)-yl]-1-azabicyclo[2.2.1]heptan-3-ol;
3-[5-(3-benzyl-1,2,4-thiadiazol)-yl]-1-azabicyclo[2.2.1]heptane;
1-methyl-3-[5-(3-amino-1,2,4-thiadiazol)-yl-pyrrolidine;
1-methyl-3-[5-(3-amino-1,2,4-thiadiazol)-yl]-1,2,5,6-tetrahydropyridine;
3-[5-(3-amino-1,2,4-thiadiazol)-yl]-1,2,5,6-tetrahydropyridine;
3-[5-(3-amino-1,2,4-thiadiazol)-yl]quinuclidine;
6-[5-(3-methyl-1,2,4-thiadiazol)-yl]-1-azabicyclo-[3.2.1]octane;
6-[5-(3-amino-1,2,4-thiadiazol)-yl]-1-azabicyclo-[3.2.1]octane;
3-[5-(3-amino-1,2,4-thiadiazol)-yl]-1-azabicyclo-[2.2.1]heptane;
5-[5-(3-methyl-1,2,4-thiadiazol)-yl]quinuclidin-3-ol;
5-[5-(3-amino-1,2,4-thiadiazol)-yl]quinuclidin-3-ol;
5-methyl-3-[5-(3-methyl-1,2,4-thiadiazol)-yl]-quinuclidine;
5-methyl-3-[5-(3-amino-1,2,4-thiadiazol)-yl]-quinuclidine;
5-[5-(3-amino-1,2,4-thiadiazol)-yl]-1-azabicyclo-[2.2.1]heptan-3-ol;
5-methyl-3-[5-(3-methyl-1,2,4-thiadiazol)-yl]-1-azabicyclo[2.2.1]heptane;
5-methyl-3-[5-(3-amino-1,2,4-thiadiazol)-yl]-1-azabicyclo[2.2.1]heptane;
3-[5-(3-ethoxy-1,2,4-thiadiazol)-yl]-1-azabicyclo-[2.2.1]heptane;
3-[5-(3-chloro-1,2,4-thiadiazol)-yl]-1-azabicyclo-[2.2.1]heptane;
3-[5-(3-methylamino-1,2,4-thiadiazol)-yl]-1-azabicyclo-[2.2.1]heptane;
3-[5-(3-ethylamino-1,2,4-thiadiazol)-yl]-1-azabicyclo-[2.2.1]heptane;
3-[5-(3-ethyl-1,2,4-thiadiazol)-yl]-1,2,5,6-tetrahydropyridine;
3-[5-(3-n-propyl-1,2,4-thiadiazol)-yl]-1,2,5,6-tetrahydropyridine;
3-[5-(3-cyclopropyl-1,2,4-thiadiazol)-yl]-1,2,5,6-tetrahydropyridine;
3-[5-(3-benzyl-1,2,4-thiadiazol)-yl]-1,2,5,6-tetrahydropyridine;
3-[5-(3-methoxy-1,2,4-thiadiazol)-yl]-1,2,5,6-tetrahydropyridine;
3-[5-(3-ethoxy-1,2,4-thiadiazol)-yl]-1,2,5,6-tetrahydropyridine;
3-[5-(3-chloro-1,2,4-thiadiazol)-yl]-1,2,5,6-tetrahydropyridine;
3-[5-(3-methylthio-1,2,4-thiadiazol)-yl]-1,2,5,6-tetrahydropyridine;
3-[5-(3-methylamino-1,2,4-thiadiazol)-yl]-1,2,5,6-tetrahydropyridine;
3-[5-(3-ethylamino-1,2,4-thiadiazol)-yl]-1,2,5,6-tetrahydropyridine;
1-methyl-3-[5-(3-n-propyl-1,2,4-thiadiazol)-yl]-1,2,5,6-tetrahydropyridine;
1-methyl-3-[5-(3-cyclopropyl-1,2,4-thiadiazol)-yl]-1,2,5,6-tetrahydropyridine;
1-methyl-3-[5-(3-benzyl-1,2,4-thiadiazol)-yl]-1,2,5,6-tetrahydropyridine;
1-methyl-3-[5-(3-isopropyl-1,2,4-thiadiazol)-yl]-1,2,5,6-tetrahydropyridine;
3-[5-(3-isopropyl-1,2,4-thiadiazol)-yl]-1,2,5,6-tetrahydropyridine;
5-[5-(3-cyclopropyl-1,2,4-thiadiazol)-yl]-1-azabicyclo[2.2.1]heptan-3-ol;
and salts and prodrugs thereof.

Most of the compounds of this invention have at least one asymmetric centre and often more than one; and can therefore exist as both enantiomers and diastereoisomers. In particular, those compounds possessing an unsymmetrical azabicyclic ring system may exist as exo and endo diastereoisomers. It is to be understood that the invention covers all such isomers and mixtures thereof.

Also included within the scope of the present invention are salts of the novel compounds. It will be appreciated that salts of the compounds for use in medicine will be non-toxic pharmaceutically acceptable salts. Other salts may, however, be useful in the preparation of the compounds of the invention or their non-toxic pharmaceutically acceptable salts. Acid addition salts, for example, may be formed by mixing a solution of the compound with a solution of a pharmaceutically acceptable non-toxic acid such as hydrochloric acid, fumaric acid, maleic acid, succinic acid, acetic acid, citric acid, tartaric acid, carbonic acid or phosphoric acid. Where the novel compound carries a carboxylic acid group the invention also contemplates salts thereof, preferably non-toxic pharmaceutically acceptable salts thereof, such as the sodium, potassium and calcium salts thereof.

Salts of amine groups may also comprise the quaternary ammonium salts in which the amino nitrogen atom carries an alkyl, alkenyl, alkynyl or aralkyl group. Such quaternary ammonium derivatives penetrate poorly into the central nervous system and are therefore useful as peripherally selective muscarinic agents, useful for example as antispasmodic agents, agents to reduce gastric acid secretion, agents to block the muscarinic actions of acetylcholinesterase inhibitors in the treatment of myasthenia gravis and as agents to co-administer with muscarinic agonists in Alzheimer's disease.

It is believed that those compounds of the invention which directly stimulate postsynaptic receptors are particularly useful as analgesic agents.

This invention therefore also provides a pharmaceutical composition comprising a compound of the invention and a pharmaceutically acceptable carrier.

It may, where appropriate, be advantageous, in order to reduce unwanted peripherally mediated side-effects, to incorporate into the composition a peripherally acting cholinergic antagonist (or anti-muscarinic agent). Thus the compounds of the invention may advantageously be administered together with a peripheral cholinergic antagonist such as N-methylscopolamine, N-methylatropine, propantheline, methantheline or glycopyrrolate.

The compounds of the invention can be administered orally, parenterally or rectally at a daily dose of about 0.01 to 10 mg/kg of body weight, preferably about 0.1 to 1 mg/kg, and may be administered on a regimen of 1-4 times a day. When a cholinergic antagonist is administered, it is incorporated at its conventional dose.

The pharmaceutical formulations of this invention preferably are in unit dosage forms such as tablets, pills, capsules, powders, granules, sterile parenteral solutions or suspensions, or suppositories for oral, parenteral or rectal administration. For preparing solid compositions such as tablets, the principal active ingredient is mixed with a pharmaceutical carrier, e.g. conventional tabletting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing from 0.1 to about 500 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids or mixtures of polymeric acids with such materials as shellac, cetyl alcohol and cellulose acetate.

The liquid forms in which the novel compositions of the present invention may be incorporated for administration orally or by injection include aqueous solutions, suitably flavoured syrups and flavoured emulsions with edible oils such as cottonseed oil, sesame oil, coconut oil and peanut oil, as well as elixirs and similar pharmaceutical vehicles. Suitable dispersing or suspending agents for aqueous suspension include synthetic and natural gums such as tragacanth, acacia, alginate, dextran, sodium carboxymethylcellulose, methylcellulose, polyvinyl-pyrrolidone and gelatin.

The 1,2,4-thiadiazoles of formula IA may be prepared by a process which comprises the cyclisation of a compound of formula III: 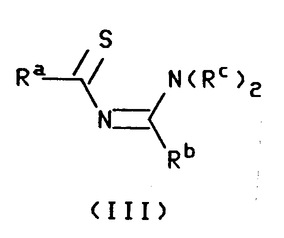
wherein one of R^{a} and R^{b} is a group R¹, and the other is a group R²; and R^{c} is hydrogen or an alkyl group.

Cyclisation of compound III can be achieved using an aminating agent such as hydroxylamine-O-sulphonic acid in a lower alkanol such as methanol, ethanol or propanol, in the presence of pyridine, at between -20°C and 50°C for about 1-6 hours.

Cyclisation of compounds of formula III (R^{c}=H) may also be achieved by use of an oxidising agent such as bromine, iodine, hydrogen peroxide or nitric acid.

The 1,2,4-thiadiazoles may also be prepared by cycloaddition of a nitrile sulphide R^{a}-C≡N⁺-S⁻ with a nitrile of formula R^{b}CN where R^{a} and R^{b} are as defined above.

A further method for the preparation of the 1,2,4-thiadiazoles of this invention comprises reaction of a thiadiazole of formula IV: 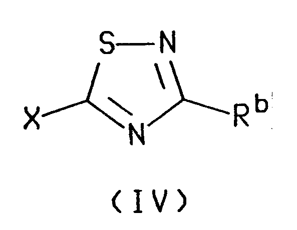
with a reagent which provides an anion ⁻R^{a}, where R^{a} and R^{b} are as previously defined and X represents halogen. Compound IV may be prepared by the general method described in Chem. Ber., 1957, 90, 182.

Reagents which may provide the anion ⁻R^{a} include a Grignard reagent R^{a}MgY (where Y = halogen); an organocuprate reagent such as LiR^{a}₂Cu; an organolithium reagent R^{a}Li; or a compound which stabilises the anion by means of an adjacent activating group such as an ester or enolisable ketone function. In this case, the adjacent ester or ketone function may be retained after the process is complete, or may be removed. For example, an ester moiety may be hydrolysed and decarboxylated.

1,2,5-Thiadiazoles of this invention may be prepared by reacting a diamine of the type 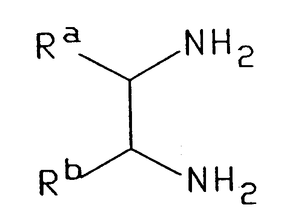
where R^{a} and R^{b} are as defined above, with a sulphur chloride such as thionyl chloride or sulphur dichloride.

1,3,4-Thiadiazoles of this invention may be prepared by dehydration of a thiosemicarbazide of formula R^{x}CSNHNHCONR^{p}R^{q} where R^{x} is an azacyclic or azabicyclic ring system and R^{p} and R^{q} are hydrogen or an alkyl group, with a dehydrating agent such as sulphuric acid, polyphosphoric acid or methanesulphonic acid.

The azacyclic or azabicyclic moiety may be introduced into the molecules concerned by methods known from the art, in particular by methods analogous to those described in EP-A-0239309.

After any of the above described processes is complete, one substituent of low lipophilicity can be converted to another. For example an amino group may be converted to chloro, or hydrazo, -NHNH₂, via the intermediacy of diazonium, -N². Similarly, a chloro substituent may be converted to methoxy by reaction with a nucleophile such as methoxide; and alkoxycarbonyl groups may be converted, via carboxy, to an amino substituent, -NH₂.

In any of the above reactions it may be necessary and/or desirable to protect any sensitive groups in the compounds. For example, if R^{a} and/or R^{b} include amino, carboxy, hydroxy or thiol groups, these may be protected in conventional manner. Thus, suitable protecting groups for hydroxy groups include silyl groups such as trimethylsilyl or t-butyldimethylsilyl, and etherifying groups such as tetrahydropyranyl; and for amino groups include benzyloxycarbonyl and t-butoxycarbonyl. Carboxy groups are preferably protected in a reduced form such as in the form of their corresponding protected alcohols, which may be subsequently oxidised to give the desired carboxy group. Thiol groups may be protected by disulphide formation, either with the thiol itself or with another thiol to form a mixed disulphide. The protecting groups may be removed at any convenient stage in the synthesis of the desired compound according to conventional techniques.

The following Examples illustrate the preparation of compounds according to the invention. Each of the compounds of the Examples demonstrates an affinity for the muscarinic receptor, having an IC₅₀ (concentration required to displace 50% of specific [³H]-N-methylscopolamine binding from rat cortical membrane preparations) significantly lower than 100µM. Penetrability into the central nervous system of compounds of this invention was assessed by a measurable displacement of radioligand binding using standard "ex-vivo" binding techniques (Ref: J. Neurosurg., 1985, 63, 589-592).

In the Examples, all temperatures are in °C; THF is tetrahydrofuran; and ether is diethyl ether.

### EXAMPLE 1

### 3-[5-(3-Methyl-1,2,4-thiadiazol)-yl]pyrrolidine Hydrochloride

### a) 3-Methoxycarbonylpyrrolidine

This was prepared by the method described by M. Joucla and J. Mortier (J. Chem. Soc., Chem. Commun., 1985, 1566) from formaldehyde, glycine and methyl acrylate and obtained as a crude oil which was used without further purification.

### b) 1-t-Butyloxycarbonyl-3-methoxycarbonyl pyrrolidine

To a solution of 3-methoxycarbonylpyrrolidine (10g, 0.077 mol) in dichloromethane (50 ml) at 4°C was added dropwise a solution of di-t-butyldicarbonate (16.9g, 0.077mol) in dichloromethane (50 ml). The solution was stirred at 20°C for 16 hours then evaporated under reduced pressure. The residue was purified by chromatography on silica (eluting with methanol/dichloromethane (1:100)) to give the title compound as a colourless oil (12.9g); νₘₐₓ (liquid film) 1740 cm⁻¹ (C=0); m/e 230 (M+1)⁺; δ(360 MHz, CDCl₃) 1.46 (9H,s,C(CH₃)₃, 2.10-2.15 (2H,m,4CH₂), 3.00-3.10 (1H,m) with 3.25-3.40 (1H,m) and 3.44-3.68 (3H,m) (2CH₂, 3CH and 5CH₂) and 3.71 (3H,s,OCH₃).

### c) 1-t-Butyloxycarbonyl-3-carboxamidopyrrolidine

A solution of 1-t-butyloxycarbonyl-3-methoxycarbonyl-pyrrolidine (8.2g. 36 mmol) in methanol (20ml) was stirred with water (20 ml) containing sodium hydroxide (1.7g, 43.2 mmol) for 15 minutes. The methanol was removed under reduced pressure and the remaining solution acidified with acetic acid and extracted (6x) with dichloromethane. The combined extracts were dried (sodium sulphate) and concentrated in vacuo to give a white solid. This solid was dissolved in dichloromethane (40 ml) containing triethylamine (1.96g, 19 mmol) and cooled to 0°C. Ethyl chloroformate (2.12g, 19 mmol) was added and the solution was allowed to warm to 20°C before bubbling ammonia through until the solution was basic. The reaction mixture was poured onto water and extracted with dichloromethane (6x). The combined extracts were dried with sodium sulphate and concentrated to give the amide (3.7g) mp 111-113°C; νₘₐₓ (nujol) 3350, 3175, 1695, 1665 and 1635 cm⁻¹; m/e 213 (CI⁻, [M-1]⁻); δ (360 MHz, CDCl₃) 1.45 (9H, s, (CH₃)₃), 2.07-2.16 (2H,m,4CH₂), 2.88-3.00 amd 3.28-3.38 (each 1H, each m, 5CH₂), 3.44-3.68 (3H, m, 2CH₂ and 3CH) and 5.82-6.04 (2H, bd, NH₂).

### d) 1-t-Butyloxycarbonyl-3-thiocarboxamidopyrrolidine

1-t-Butyloxycarbonyl-3-carboxamidopyrrolidine (214 mg, 1 mmol) was heated under reflux in benzene (10 ml) with Lawesson's reagent (202 mg, 0.5 mmol) for 2 hours. The mixture was cooled then chromatographed on silica gel eluting with methanol/dichloromethane (1:20) to give the thioamide (103 mg), as a solid, mp 131-133°C; νₘₐₓ (nujol) 3300, 3180, 1670, 1650, 1165, and 1130 cm⁻¹; m/e 229 (CI⁻, [M-1]⁻); δ(360MH₂, CDCl₃) 1.44 (9H, s, (CH₃)₃), 2.14-2.22 (2H, m, 4CH₂), 3.27-3.37 (2H, m, 5CH₂), 3.54-3.70 (3H, m, 2CH₂ and 3CH) and 8.06-8.20 (2H, bs, NH₂).

### e) 1-t-Butyloxycarbonyl-3-(N,N-dimethylacetamidinothiocarbonyl) pyrrolidine

1-t-Butyloxycarbonyl-3-thiocarboxamidopyrrolidine (230 mg, 1mmol) in dichloromethane (10 ml) was treated with dimethylacetamide dimethylacetal (287 mg, 2.4 mmol) for 16 hours. The mixture was chromatographed on silica eluting with methanol/dichloromethane (1:20) to give the title compound as an oil (320 mg); m/e 300 (CI⁺, [M+1]⁺); δ(360MHz, CDCl₃) 1.45 (9H,s,C(CH₃)₃), 2.13-2.27(2H, m, 4CH₂), 2.42 and 2.43 (3H, 2 x s, N=CCH₃ E and Z isomers), 3.10 and 3.12 (3H, 2 x s) and 3.20 and 3.21 (3H, 2 x s, (N(CH₃)₂),, 3.25-3.36 (1H, m) and 3.42-3.66 (4H, m) (2CH₂, 3CH and 5CH₂).

### f) 3-[5(3-Methyl-1,2,4-thiadiazol)-yl]pyrrolidine Hydrochloride

The thioacylamidine prepared as in (e) (2.8 g, 9.4 mmol) in ethanol (50 ml) was treated with pyridine (1.5 g, 18.8 mmol) and hydroxylamine-O-sulphonic acid (1.3 g, 11.2 mmol) in methanol (10 ml) for 2 hours. The solvents were removed in vacuo and the residue taken up in water and dichloromethane. The dichloromethane was separated, dried with sodium sulphate and concentrated in vacuo. The residue was dissolved in ethanol (50 ml) and 2N HCl (20 ml) and heated under reflux for 20 minutes. The ethanol was removed in vacuo and water (10 ml) was added and extracted with dichloromethane. The aqueous solution was adjusted to pH10 with sodium carbonate and extracted four times with dichloromethane. The combined extracts were dried and concentrated under reduced pressure and the residue, in diethyl ether, treated with ethereal hydrogen chloride. The resulting gum was triturated with diethyl ether to give the title compound as a white solid (670 mg), mp 135-137°C; (Found: C, 38.2; H, 5.5; N,18.9. C₇H₁₁N₃S. HCl requires C, 38.2; H, 5.7; N, 19.1%); m/e 170 (CI⁺,[M+1]⁺); δ(360 MHz, D₂O) 2.25-3.39 (1H,m) and 2.63-2.70 (1H,m, (4CH₂), 2.62 (3H, s, CH₃), 3.47-3.64 (3H, m, one of 2CH₂, and 5CH₂), 3.85 (1H, dd, J=7Hz and 9.5 Hz, one of 2CH₂) and 4.24 (1H, quin, J=7Hz).

### EXAMPLE 2

### 1-Methyl-3-[5-(3-methyl-1,2,4-thiadiazol)-yl] pyrrolidine Hydrogen Oxalate

A solution of 3-[5-(3-methyl-1,2,4-thiadiazol)-yl]-pyrrolidine in formic acid (3 ml) containing formaldehyde (3 ml of a 40% solution in water) was heated under reflux for 15 minutes. The mixture was evaporated to dryness under reduced pressure and the residue partitioned between aqueous potassium carbonate solution and dichloromethane. The dichloromethane portion was separated, dried and concentrated in vacuo. The residue was purified by chromatography on silica gel eluting with methanol/dichloromethane (1:10) and the product thus obtained treated with oxalic acid. The Precipitated salt was triturated with diethyl ether leaving the title compound as a white solid (125 mg), mp 105-107°C; Found: C, 43.2; H, 5.2; N, 15.0. C₈H₁₃N₃S (COOH)₂.0.25H₂O requires C,43.2; H. 5.6; N, 15.1%); m/e 184 (CI⁺, [M+1]⁺); δ(360 MHz, D₂O) shows two isomers in ratio 1:1, 2.28-2.39, 2.44-2.50, 2.65-2.72 and 2.79-2.84 (each 0.5H, each m, 4CH₂) 2.61 and 2.62 (each 1.5H, each s. CCH₃), 3.03 and 3.05 (each 1.5H, each s, NCH₃), 3.29-3.46 (1.5H, m) with 3.65 (0.5H. dd, J=9Hz and 12Hz), 3.83-3,98(1.5H, m) and 4.18(0.5H, dd, J=7.5Hz and 12Hz) (2CH₂ and 5CH₂), 4.28(0.5H, quin, J=8.5Hz, 0.5(3CH)) and 4.44 (0.5H. quin, J=7Hz, 0.5(3CH)).

### EXAMPLE 3

### 3-[5-(3-Methyl-1,2,4-thiadiazol)-yl]quinuclidine Hydrogen Oxalate

### a) 3-[5-(3-Methyl-1,2,4-thiadiazol)-yl]-3-methoxycarbonyl-quinuclidine Hydrochloride

3-Methoxycarbonylquinuclidine (7.2g, 42 mmol), prepared by the method of C.A. Grob and E. Renk (Helv. Chim. Acta., (1954), 37, 1689), was dissolved in tetrahydrofuran (400 ml) at -78°C under an atmosphere of nitrogen. Lithium diisopropylamide. THF (40 ml of a 1.5M solution in cyclohexane, 60 mmol) was added slowly and the reaction was stirred at -78°C for 1 hour. 5-Chloro-3-methylthiadiazole (7.0 g, 52 mmol), prepared by the method of J. Goerdeler et al, Chem. Ber., (1957), 90, 182, was added and the mixture was allowed to warm slowly to room temperature over 2 hours. The solvent was removed under reduced pressure and dilute hydrochloric acid was added to the residue. The aqueous solution was extracted twice with diethyl ether then made basic with Potassium carbonate. The aqueous solution was again extracted three times and these extracts were dried with sodium sulphate and then treated with dry hydrogen chloride in diethyl ether. The precipitated salt was recrystallised from methanol-ethyl acetate to give the title compound (1.52 g), mp 142°C, (Found: C, 47.55; H, 5.96; N, 13.76. C₁₂H₁₈N₃O₂S. HCl requires C, 47.28; H, 6.28; N. 13.78%). νₘₐₓ (dichloromethane) 2260 (N-H). 1750 cm⁻¹ (C=0); m/e 267 (M⁺ of free base); δ(360 MHz, D₂O) 1,74-1.85 (1H, m) and 1.91-2.14 (3H, m) (5CH₂ and 8CH₂), 2.65 (3H,s, CCH₃), 2.90-2.92 (1H,m,4CH), 3.28-3.46 (4H,m, 6CH₂ and 7CH₂), 3.92 (3H, s, OCH₃), 4.27 (1H,d, J=14Hz, one of 2CH₂) and 4.43 (1H, dd, J=14Hz and 2.5 Hz, one of 2CH₂).

### b) 3-[5-(3-Methyl-1,2,4-thiadiazol)-yl] quinuclidine Hydrogen Oxalate

3-[5-(3-Methyl-1,2,4-thiadiazol)-yl]-3-methoxycarbonyl-quinuclidine hydrochoride (300 mg, 0.97 mmol) in tetrahydrofuran (7 ml) was treated with 1N aqueous sodium hydroxide (2.2 ml) for 2 hours. The tetrahydrofuran was evaporated off under reduced pressure and the aqueous solution adjusted to pH 2 with concentrated hydrochloric acid. After 15 minutes the solution was made basic with potassium carbonate and extracted with dichloromethane which was then dried with sodium sulphate and concentrated in vacuo. The residue dissolved in diethyl ether was treated with oxalic acid in diethyl ether and the resulting salt recrystallised from methanol/diethyl ether to give the title compound (260 mg), mp 159-160°C. (Found: C, 45.51; H, 5.31; N, 12.27; C₁₀H₁₅N₃S. 1.5(COOH)₂ requires C, 45.34; H, 5.27; N, 12.20%); m/e 210 (CI⁺, [M+1]⁺ of free base); δ(360 MHz, D₂O) 1.88-1.94 and 2.07-2.16 (each 2H, each m, 5CH₂ and 8CH₂), 2.50-2.55 (1H, m, 4CH), 2.63 (3H, s, CH₃), 3.30-3.52 (4H, m, 6CH₂ and 7CH₂), 3.78(1H, dd, J=7Hz and 14Hz, one of 2CH₂), 3.86 (1H, t, J=14Hz, one of 2CH₂) and 4.06-4.13(1H, m, 3CH).

### EXAMPLE 4

### 3-[5-(3-Methyl-1,2,4-thiadiazol)-yl]-1-azabicyclo[2,2,1]-heptane Hydrogen Oxalate

### a) 1-Benzyl-3-ethoxycarbonylpyrrolidine

A solution of 1-benzyl-3-hydroxymethylpyrrolidine (60 g, 0.314 mol; J. Org. Chem., (1961). 26, 1519) in conc. sulphuric acid (7.3 ml) and water (350 ml) was treated at 0°C with a solution of chromium trioxide (26.2 g) in conc. sulphuric acid (18 ml) and water (410 ml). The mixture was stirred at 0°C for 5 min. 100°C for 2 min and then cooled back to 0°C. A further charge of the chromium trioxide solution was then added and the mixture heated at 100°C for 0.5h. After cooling again to 10°C, excess sodium metabisulphite was added to destroy any remaining oxidant and the pH adjusted to 10 with 6N-sodium hydroxide solution. After filtration, the mixture was acidified to pH 2 with 6N-hydrochloric acid and the solution evaporated. The rigorously dried residue was treated at 20°C for 16h with anhydrous ethanol saturated with hydrogen chloride. The gum after evaporation of the solvent was partitioned between dichloromethane and water made basic with excess potassium carbonate and the required ethyl ester isolated from the organic layer (25g); δ (60 MHz, CDCl₃) 1.20 (3H, t, J = 6.5 Hz, CH₃); 1.9-2.2 (5H, m, 2 X CH₂ and CH); 3.60 (2H, s, CH₂ Ph); 4.10 (2H, q, J = 6.5Hz, CH₂ CH₃) and 7.23 (5H, broad s, C₆H₅).

### b) 1-Ethoxycarbonylmethyl-3-ethoxycarbonylpyrrolidine

The foregoing 1-benzylpyrrolidine (18g) in ethanol (400 ml) was subjected to hydrogenolysis over Pd(OH)₂ (5g) at 50 psi in a Paar shaker for 72h. After filtration, the solvent was evaporated and the resulting oil purified by chromatography on alumina in methanol-dichloromethane (1:19) to give 3-ethoxycarbonyl-pyrrolidine (8g) as a colourless oil. This amine (7.75g, 54mmol) in ether (70 ml) was treated at 0°C with a solution of ethyl bromoacetate (4.53g, 27 mmol in ether (40 ml) in the presence of solid potassium carbonate (5g). After 0.5h at 0°C and 1h at reflux, the precipitated solid was removed by filtration and the residue isolated from the filtrate purified by chromatography on alumina in dichloromethane to give the diester (5.56g), δ (60MHz, CDCl₃) 1.25 (6H, t, J = 7Hz, 2 X CH₃); 2.1-3.2 (7H, m, 3 X CH₂ and CHCO); 3.3 (2H, s, CH₂CO) and 4.15 and 4.20 (each 2H, each q, each J = 7Hz, 2 X OCH₂).

### c) 1-Azabicyclo[2,2,1]heptan-3-one

A mixture of ethanol (4.5 ml) and toluene (6 ml) was added dropwise to a rapidly stirred suspension of potassium (2.66 g, 68.2 mmol) in toluene (15 ml) at 120°C under nitrogen. After 1h at this temperature, a solution of the foregoing diester (6.24g, 27.3 mmol) in toluene (25 ml) was then added and the mixture heated at 140°C for 3h. Concentrated hydrochloric acid (90 ml) was added, the two solvent phases separated and the aqueous phase heated under reflux for 18h. The reaction mixtur e was evaporated to half volume, neutralised with solid potassium csrbonate and exracted with dichloromethane. The material isolated from the organic extracts was chromatographed on silica in methanol-dichloromethane (1:9) to give the required azabicycle (250 mg), δ (CDCl₃, 360 MHz) 1.75-1.80 (1H, m, H of CH₂); 2.06-2.12 (1H, m, H of CH₂); 2.70-2.81 (4H, m, 2 X NCH₂) and 3.00-3.12 (3H, m, COCH₂ and CH).

### d) 3-(1,3-Dithian-2-ylidene)-1-azabicyclo[2,2,1]heptane

A solution of n-butyl lithium in hexane (1.4 ml of a 1.6M solution; 2.3 mmol) was added to a solution of 2-trimethylsilyl-1,3-dithiane (457 mg, 2.37 mmol) in tetrahydrofuran (5ml) stirred under nitrogen at -35°C. After 1.5h, the foregoing ketone (220 mg, 1.98 mmol) in tetrahydrofuran (5 ml) was added and the mixture allowed to warm to 20° over 1h. Water (20 ml) was added and the solution extracted with dichloromethane. Chromatography of the material isolated from the organic extracts on alumina in methanol-dichloromethane (1:49) gave the dithioacetal ketene (370 mg), δ (CDCl₃, 360 MHz) 1.35-1.48 (1H, m, CH); 1.76-1.90 (2H, m, CH₂); 2.10-2.17 (2H, m, CH₂); 2.43 (1H, dd, J = 3Hz and 9Hz, bridge CH); 2.46-2.58 (1H, m, CH); 2.62 (1H, m, CHN); 2.70-2.94 (5H, m, CH and 2 X CH₂S); 3.02 (1H, dd, J = 3Hz and 18Hz. CH-C=C) and 3.41 (1H, dd, J = 3Hz and 18Hz, CH-C=C).

### e) 3-Methoxycarbonyl-1-azabicyclo-[2,2,1]-heptane

The foregoing ketene dithioacetal (3.4g) in dry methanol (100 ml) saturated with anhydrous hydrogen chloride was stirred at 55°C for 12 hours. After evaporation of the solvent, the residue was dissolved in water which was washed (6 X) with diethyl ether. The aqueous solution was adjusted to pH 10 with potassium carbonate and extracted (3 X) with dichloromethane. The combined extracts were dried and concentrated in vacuo to give the title compound (1.56g) as an oil; δ (360 MHz, CDCl₃) 1.12-1.19 (1H, m, one of 5CH₂), 1.56-1.65 (1H, m, one of 5CH₂), 2.21-2.25 (1H, m) with 2.32-2.35 (1H, m), 2.40-2.50 (1H, m), 2.62-2.66 (1H, m), 2.73-2.87 (3H, m) and 2.96-3.01 (1H, m) (2CH₂, 3CH, 4CH, 6CH₂ and 7 CH₂), and 3.67 (3H, s, OCH₃).

### f) 3-[5-(3-Methyl-1,2,4-thiadiazol)-yl]-1-azabicyclo-[2,2,1]-heptane Hydrogen Oxalate

To a solution of 3-methoxycarbonyl-1-azabicyclo-[2,2,1]- heptane (1.55g, 10mmol) in tetrahydrofuran (40 ml) at -78°C under an atmosphere of nitrogen was added lithium diispropylamide. THF complex (8.6 ml of a 1.5M solution in cyclohexane, 12.9 mmol). The solution was stirred at -78°C for 1 hour then 3-methyl-5-chloro-1,2,4-thiadiazole was added after which the temperature was maintained at -78°C for 1 hour then allowed to warm slowly to room temperature. The solvent was removed in vacuo, then aqueous potassium carbonate was added and extracted (4 X) with dichloromethane. The combined extracts were dried and concentrated. and the residue dissolved in methanol (50 ml). 2N sodium hydroxide solution (50 ml) was added and the mixture stirred for 0.75 hours, then the methanol was evaporated under reduced pressure. The aqueous solution was washed (3 x) with dichloromethane then adjusted to pH2 with concentrated hydrochloric acid. After 10 minutes the solution was made basic with potassium carbonate and extracted (3 X) with dichloromethane. The combined extracts were dried and concentrated and the residue chromatographed on alumina. Elution with a gradient going from neat ethyl acetate to 5% methanol/ethyl acetate gave first, the minor isomer of the title compound free base, converted to the oxalic acid salt in ether and recrystallised from methanol/diethyl ether (15 mg), mp 163-4°C. δ(360 MHz, D₂O) 1.98-2.06 (1H, m, one of 5CH₂), 2.22-2.32 (1H, m, one of 5CH₂), 2.61 (3H, s, CH₃), 3.25-3.30 (2H, m) with 3.34-3.42 (1H, m), 3.51-3.58 (2H, m), 3.82 (2H, m), and 3.98 (1H, m), (2CH₂, 3CH, 4CH, 6CH₂ and 7CH₂); m/e.

Further elution gave the second product which was treated with oxalic acid and recrystallised from methanol/diethyl ether to give the major isomer of the title compound (90 mg), mp 143-4°C. (Found: C, 46.21; H, 5.35; N, 14.49. C₉H₁₃N₃S.(COOH)₂ requires C, 46.31; H, 5.30; N, 14.73%). m/e 195 (M⁺ of free base); δ(360 MHz, D₂O) 1.64-1.74 (1H, m, one of 5CH₂, 2.00-2.10 (1H, m, one of 5CH₂), 2.64 (3H, s, CH₃), 3.32-3.46 (3H, m) with 3.50-3.58 (2H, m), 3.66 (1H, ddd, J=2.2, 5.7Hz, and 12.2 Hz), 3.97 (1H, dt, J = 3.0Hz and 11.5Hz) and 4.35-4.42 (1H, m) (2CH₂, 3CH, 4CH, 6CH₂ and 7CH₂).

### EXAMPLE 5

### 3-[5-(3-Phenyl-1,2,4-thiadiazol)-yl]quinuclidine Hydrochloride

A solution of 3-methoxycarbonyl quinuclidine (940mg, 5.56mmol) in tetrahydrofuran (75ml) was treated with a 1.5M solution of lithium diisopropylamide-tetrahydrofuran complex in cyclohexane (5ml, 7.5mmol) at -78°C under an atmosphere of nitrogen for 1 hour. 5-Chloro-3-phenyl-1,2,4-thiadiazole (1.5g, 7.7mmol prepared by the method of Chem. Ber., (1957), 90, 182) was added and the reaction was allowed to warm slowly to room temperature. The solvent was evaporated under reduced pressure and the residue stirred in methanol (30 ml) and 2N NaOH (20ml) for 1 hour. The methanol was removed in vacuo and the aqueous solution extracted with ethyl acetate (3 x 20 ml) then adjusted to pH 2 with concentrated hydrochloric acid. After three hours the solution was made basic and extracted with dichloromethane (3 x 40 ml). The combined dichloromethane solutions were dried with sodium sulphate and concentrated under reduced pressure to give the title compound free base (210mg) which was treated with ethereal hydrogen chloride. The resulting salt was recrystallised from dichloromethane-diethyl ether to give the title compound (187mg), mp 213°C (decomp.); (Found: C, 56.76; H, 5.84; N, 13.18. C₁₅H₁₇N₃S. HCl. 0.5H₂O requires C, 56.86; H, 6.04; N, 13.26%); m/e 271 (M⁺ of free base); δ (360MHz, D₂O) 1.88-2.06 and 2.13-2.32 (each 2H, each m, 5CH₂ and 8CH₂), 2.56-2.62 (1H, m, 4CH), 3.36-3.60 (4H, m, 6CH₂ and 7CH₂), 3.89 (1H, ddd, J = 2.5, 10.5 and 13.0Hz, one of 2CH₂), 4.01 (1H, dd, J = 6.2 and 13.0Hz, one of 2CH₂), 4.12-4.22 (1H, m, 3CH), 7.6 - 7.65 and 8.20 - 8.26 (3H and 2H respectively, each m, Ph).

The free bases of Example 6 were prepared by the method of Example 5 using the appropriate 5-chloro-1,2,4-thiadiazole.

### EXAMPLE 6

### 3-[5-(3-Cyclopropyl-1,2,4-thiadiazol)-yl]quinuclidine Hydrogen Oxalate

3-Methoxycarbonyl quinuclidine (2.0g, 11.8mmol) and 5-chloro-3-cyclopropyl-1,2,4-thiadiazole (2.5g, 15.4mmol, prepared by the general method described in Chem. Ber., (1957), 90, 182) gave the title compound free base which was treated with oxalic acid in ether. Crystallisation from methanol-diethyl ether afforded the title compound (540mg), mp 175-6°C; m/e 235 (M⁺ of free base); δ (360MHz, D₂O) 1.02-1.14 (4H, m, 2 x cyclopropyl CH₂), 1.84-1.92 and 2.05-2.14 (each 2H, each m, 5CH₂ and 8CH₂), 2.29-2.38 (1H, m, cyclopropyl CH), 2.46-2.52 (1H, m, 4CH), 3.29-3.49 (4H, m, 6CH₂ and 7CH₂), 3.81 (2H, d, J = 8.7Hz, 2CH₂) and 4.04 (1H, dt, J = 2 and 8.7Hz, 3CH).

### EXAMPLE 7

### exo- and endo-3-[5-(3-Dimethylamino-1,2,4-thiadiazol)-yl]-1-azabicyclo[2.2.1]heptane

### a) 1-Carbomethoxymethylene-4-carbomethoxy pyrrolidin-2-one

To a solution of glycine methyl ester hydrochloride (476g, 3.79mol) in methanol (900ml) was added sodium methoxide (205g, 3.79mol) and dimethyl itaconate (500g, 3.16mol) and the mixture was heated under reflux for 16 hours. The reaction was filtered and the solvent removed under reduced pressure. 5N hydrochloric acid (500ml) was added to the residue which was then extracted with dichloromethane (3 x 500ml). The combined extracts were dried with sodium sulphate and evaporated in vacuo to give a residue which was distilled to give the title compound (337g), bp. 132-135°C at 1mmHg; m/e CI⁺ 216 [(M + 1)⁺ of free base] δ (360MHz, CDCl₃) 2.68 (1H, dd, J = 9.6 and 17.2 Hz, one of 3CH₂), 2.75 (1H, dd, J = 7.5 and 17.2Hz, one of 3CH₂), 3.30-3.40 (1H, m, 4CH), 3.69-3.78 (2H, m, 5CH₂), 3.71 (3H, s, OCH₃), 3.74 (3H, s, OCH₃), 3.99 (1H, d, J = 17.6Hz, one of NCH₂CO₂) and 4.16 (1H, d, J = 17.6Hz, one of NCH₂CO₂).

### b) 1-Carbomethoxymethylene-3-carbomethoxy pyrrolidine

The foregoing amide (86g, 0.4mol) in THF (500ml) was added slowly to a 1M solution of BH₃. THF (800ml, 0.8mol) under nitrogen with cooling from an ice bath. When addition was complete the reaction was heated under reflux for 1 hour then allowed to cool. A saturated aqueous potassium carbonate solution was added and the mixture refluxed for 1 hour, then cooled. The solution was decanted from the precipitated solid and concentrated in vacuo to give a residue which was treated with 5N hydrochloric acid and washed with dichloromethane. The aqueous solution was basified with aqueous potassium carbonate and extracted three times with dichloromethane. The combined extracts were dried (Na₂SO₄), concentrated, and purified by chromatography on silica gel, eluting with ethyl acetate, to give the title compound (30g) as a yellow oil, δ (360MHz, CDCl₃) 2.11-2.18 (2H, m, 4CH₂), 2.56-2.63 with 2.71-2.78 and 2.88-2.94 (each 1H, each m, 3CH and 5CH₂), 3.06-3.15 (2H, m, 2CH₂), 3.33 (1H, d, J = 16.9Hz, one of NCH₂CO₂), 3.39 (1H, d, J = 16.9Hz, one of NCH₂CO₂), 3.69 (3H, s, OCH₃) and 3.73 (3H, s, OCH₃).

### c) 1-Azabicyclo[2.2.1]heptan-3-one

The foregoing diester (28g, 0.14mol) in dry toluene (300ml) was added over 3 hours to a refluxing solution of potassium tert-butoxide (43g) in toluene (1.31t) with vigorous stirring. After complete addition, heating was continued for 2 hours then, after cooling, concentrated hydrochloric acid (500ml) was added. The toluene was decanted off and the acid solution heated to reflux for 15 hours then cooled and concentrated in vacuo. Potassium carbonate solution was added to the residue which was then extracted several times with dichloromethane. The combined extracts were dried and concentrated in vacuo. Diethyl ether was added to the residue and the solution filtered and evaporated to give the title compound (7.2g) also described in Example 4c.

### d) 3-Methoxycarbonyl-1-azabicyclo-[2.2.1]heptane

This was prepared from the foregoing ketone by the method of Example 4d and 4c.

### e) endo-3-[5-(3-Dimethylamino-1,2,4-thiadiazol)-yl]-1-azabicyclo[2.2.1]heptane Hydrogen Oxalate

The foregoing ester (1.8g, 11.6mmol) in tetrahydrofuran (30ml) was treated with lithium diisopropylamide-tetrahydrofuran complex (10ml of a 1.5M solution in cyclohexane, 15mmol) for 1 hour at -78°C under an atmosphere of nitrogen. 5-Chloro-3-dimethylamino-1,2,4-thiadiazole (2.5g, 15.1mmol) was added as a solution in tetrahydrofuran (20ml) and the reaction was allowed to warm slowly to room temperature. The solvent was removed under reduced pressure and the residue stirred in methanol (30ml) and 2N NaOH (25ml) for 1 hour. The methanol was evaporated under reduced pressure and the aqueous solution washed 3 x with ethyl acetate then adjusted to pH2 with concentrated hydrochloric acid and left to stand for 24 hours. The solution was treated with aqueous potassium carbonate until basic and extracted (3 x) with dichloromethane. The combined extracts were dried (Na₂SO₄) and concentrated in vacuo to give a residue which was treated with oxalic acid in methanol. Crystallisation from methanol-propan-2-ol gave endo-3-[5-(3-dimethylamino-1,2,4-thiadiazol)-yl]-1-azabicyclo[2.2.1]heptane (317mg), mp 134-136°C; (Found: C, 45.76; H, 5.70; N, 17.70. C₁₀H₁₆N₄S. (COOH)₂ requires C, 45.85; H, 5.70; N, 17.82%); m/e 224 (M⁺ of free base); δ (360MHz, D₂O) 1.70-1.79 and 1.97-2.08 (each 1H, each m, 5CH₂), 3.14 (6H, s, NMe₂), 3.33-3.41 and 3.46-3.55 (3H and 2H respectively, each m, 4CH, 6CH₂ and 7CH₂), 3.67 (1H, ddd, J = 2.3, 5.6 and 12.1Hz, one of 2CH₂), 3.88 (1H, dt, J = 12.1 and 2.9Hz, one of 2CH₂) and 4.20-4.27 (1H, m, 3CH).

### f) exo-3-[5-(3-Dimethylamino-1,2,4-thiadiazol)-yl ]-1-azabicyclo[2.2.1]heptane Dihydrochloride

The mother liquor from the crystallisation above was treated with sodium methoxide in methanol until strongly basic and left to stand for 1 hour. The solvent was removed and the residue chromatographed on grade III neutral alumina eluting with 0.2% methanol in dichloromethane. Fractions containing the pure faster eluting isomer were combined and evaporated to give exo-3-[5-(3-dimethylamino-1,2,4-thiadiazol)-yl]-1-azabicyclo[2.2.1]heptane (70mg) which was crystallised as the dihydrochloride salt, mp 155-156°C (methanol-diethyl ether); (Found: C, 39.19; H, 6.06; N, 17.86. C₁₀H₁₆N₄S. 2HCl. 0.6H₂O requires C, 38.99; H, 6.28; N, 18.19%); m/e 224 (M⁺ of free base); δ (360MHz, D₂O) 1.94-2.01 and 2.19-2.29 (each 1H, each m, 5CH₂), 3.13 (6H, s, NMe₂), 3.20 (1H, d, J = 4.0Hz, 4CH), 3.26 (1H, d, J = 9.4Hz, one of 7CH₂), 3.33-3.40 (1H, m, one of 6CH₂), 3.43-3.55 (1H, m, one of 6CH₂), 3.59 (1H, d, J = 9.4Hz, one of 7CH₂) and 3.74-3.87 (3H, m, 3CH and 2CH₂).

### EXAMPLE 8

### exo- and endo-3-[5-(3-Cyclopropyl-1,2,4-thiadiazol)-yl]-1-azabicyclo[2.2.1]heptane Hydrogen Oxalate

These compounds were prepared using 3-methoxycarbonyl-1-azabicyclo[2.2.1]heptane (1.5g, 9.7mmol) and 5-chloro-3-cyclopropyl-1,2,4-thiadiazole (2.0g, 12.6mmol) giving:
a) endo-3-[5-(3-Cyclopropyl-1,2,4-thiadiazol)-yl]-1-azabicyclo[2.2.1]heptane hydrogen oxalate (270mg), mp 133-134°C, (Found: C, 49.64; H, 5.47; N, 13.38. C₁₁H₁₅N₃S.(COOH)₂.0.25H₂O requires C, 49.43; H, 5.58; N, 13.30%); m/e 221 (M⁺ of free base); δ (360MHz, D₂O) 1.07-1.16 (4H, m, 2 x CH₂ of cyclopropyl); 1.62-1.72 and 1.98-2.10 (each 1H, each m, 5CH₂), 2.31-2.38 (1H, m, CH of cyclopropyl), 3.36-3.42 and 3.46-3.55 (3H and 2H respectively, each m, 4CH, 6CH₂ and 7CH₂), 3.67 (1H, ddd, J = 2.3, 5.6 and 12.2Hz, one of 2CH₂), 3.93 (1H, td, J = 2.9 and 12.2Hz, one of 2CH₂) and 4.37 (1H, m, 3CH).
b) exo-3-[5-(3-Cyclopropyl-1,2,4-thiadiazol-yl]-1-azabicyclo[2.2.1]heptane hydrogen oxalate (105mg), mp 159-160°C; (Found: C, 49.91; H, 5.49; N, 13.44. C₁₁H₁₅N₃S.(COOH)₂ requires C, 50.15; H, 5.50; N, 13.50%); m/e 221 (M⁺ of free base), δ (360MHz, D₂O) 1.04-1.14 (4H, m, 2 x cyclopropyl CH₂), 1.95-2.10 and 2.20-2.36 (1H and 2H respectively, each m, 5CH₂ and cyclopropyl CH), 3.22 (1H, d, J = 4.4Hz, 4CH), 3.27 (1H, d, J = 9.5Hz, one of 7CH₂), 3.33-3.41 (1H, m, one of 6CH₂), 3.50-3.57 (1H, m, one of 6CH₂), 3.54 (1H, d, J = 9.5Hz, one of 7CH₂), 3.78-3.82 (2H, m, 2CH₂) and 3.91-3.95 (1H, m, 3CH).

### EXAMPLE 9

### 3-[5-(3-Benzyl-1,2,4-thiadiazol)-yl]quinuclidine Hydrogen Oxalate

Reaction of 3-methoxycarbonylquinuclidine (1.69g, 10mmol) with 3-benzyl-5-chloro-1,2,4-thiadiazole (3.15g, 15mmol, prepared according to Chem. Ber., (1956), 89, 1534) by the method of Example 5 gave the title compound free base which was treated with oxalic acid and crystallised from methanol-diethyl ether to give the title compound (1.1g), mp 88-90°C; (Found: C, 56.77, H, 5.75; N, 10.83. C₁₆H₁₉N₃S. (COOH)₂.0.45H₂O requires C, 56.36; H, 5.75; N, 10.96%); m/e 285 (M⁺ of free base); δ (360MHz, D₂O) 1.76-1.98 and 2.08-2.26 (each 2H, each m, 5CH₂ and 8CH₂), 2.46-2.52 (1H, m, 4CH), 3.29-3.50 (4H, m, 6CH₂ and 7CH₂), 3.74-3.88 (2H, m, 2CH₂), 4.03-4.10 (1H, m, 3CH), 4.34 (2H, s, CH₂Ph) and 7.23-7.43 (5H, m, Ph).

### EXAMPLE 10

### 3-[5-(3-tert-Butyl-1,2,4-thiadiazol)-yl]quinuclidine Hydrogen Oxalate

The title compound free base was prepared from 3-methoxycarbonyl quinuclidine (1.5g, 8.9mmol) and 3-tert-butyl-5-chloro-1,2,4-thiadiazole (2.04g, 11.5mmol) by the method of Example 5. Treatment of the free base with oxalic acid and crystallisation from methanol-diethyl ether gave the title compound as a hygroscopic solid (120mg); (Found: M⁺ = 251.1445. C₁₃H₂₁N₃S (free base M⁺) requires M⁺ 251.1456); δ (360MHz, D₂O) 1.41 (9H, s, 3 x CH₃), 1.85-1.95 and 2.08-2.27 (each 2H, each m, 5CH₂ and 8CH₂), 2.50-2.56 (1H, m, 4CH), 3.28-3.54 (4H, m, 6CH₂ and 7CH₂), 3.80-3.90 (2H, m, 2CH₂) and 4.02-4.12 (1H, m, 3CH).

### EXAMPLE 11

### exo-3-[5-(3-Methyl-1,2,4-thiadiazol)-yl]-endo-5-hydroxy-1-azabicyclo[2.2.1]heptane

### a) trans-3,4-Dimethoxycarbonylpyrrolidine

This was prepared from glycine and dimethylfumarate by the procedure reported by Joucla et al, J. Chem. Soc. Chem. Commun., (1985), 1566.

### b) 1-Methoxycarbonylmethyl-trans-3,4-dimethoxycarbonyl pyrrolidine

A solution of trans-3,4-dimethoxycarbonylpyrrolidine (4.1g, 22mmol) in xylene (30ml) was added to a rapidly stirred suspension of potassium carbonate (7g) in xylene (150ml), at 120°C. After 0.25 hour, a solution of methylbromoacetate (3.45g, 22.5mmol) in xylene (30ml) was added dropwise and the mixture stirred rapidly at 140°C for 2 hours. The solution was decanted from the inorganic residue which was taken up into water (100ml) and extracted with dichloromethane (3 x 150ml). The combined organics were dried (Na₂SO₄) and the solvent removed under vacuum to give the title triester as a yellow liquid (6g); δ (360MHz, CDCl₃) 2.96-3.11 (4H, m, 2CH₂ and 5CH₂), 3.31 (1H, d, J = 16.5Hz, one of NCH₂), 3.38 (1H, d, J = 16.5Hz, one of NCH₂), 3.46-3.52 (2H, m, 3CH and 4CH), 3.74 (9H, s, 3 x CH₃).

### c) 3-Methoxycarbonyl-5,5-dimethoxy-1-azabicyclo[2.2.1]heptane

A solution of 1-methoxycarbonylmethyl-trans-3,4-dimethoxycarbonylpyrrolidine (5g, 19.31mmol) in toluene (75ml) was added dropwise over a 1 hour period to a rapidly stirred solution of potassium-t-butoxide (9g, 80mmol) in toluene (250ml) at 130°C. The mixture was refluxed for 4 hours, cooled to room temperature and concentrated hydrochloric acid (75ml) added dropwise and stirred for 0.25 hours. The organic phase was extracted with further portions of hydrochloric acid (3 x 50ml) and the combined aqueous heated at 110°C for 16 hours. The solvent was then removed in vacuo, the residue dried and taken up into methanol (saturated with hydrogen chloride, 150ml). The mixture was stirred at room temperature for 24 hours and the solvent removed under vacuum. The residue was dissolved in water (50ml), basified to pH > 10 with potassium carbonate and extracted with dichloromethane (5 x 150ml). The combined extracts were dried (Na₂SO₄) and the residue remaining after removal of the solvents was chromatographed through silica-gel, using dichloromethane/methanol (93:7) as eluant, to give the title ester as a yellow liquid (0.5g). An analytical sample was prepared as the hydrogen oxalate salt, mp 134.5-136.5°C (propan-2-ol); (Found: C, 47.04; H. 6.20; N, 4.50. C₁₀H₁₇NO₄.(CO₂H)₂ requires C, 47.21; H, 6.27; N, 4.59%); δ (360MHz, CDCl₃) 2.44 (1H, dd, J = 9.8 and 3.2Hz), 2.63 (1H, dd, J = 12.7 and 3.2Hz), 2.77 (1H, d, J = 12.7Hz), 2.80-3.10 (5H, m), 3.11 (3H, s, OCH₃), 3.24 (3H, s, OCH₃), 3.71 (3H, s, CO₂CH₃).

### d) 3-[5-(3-Methyl-1,2,4-thiadiazol)-yl]-3-methoxycarbonyl-5,5-dimethoxy-1-azabicyclo[2.2.1]heptane

Lithium diisopropylamide (4.7ml of a 1.5M solution in tetrahydrofuran, 7.05mmol) was added dropwise to a solution of 3-methoxycarbonyl-5,5-dimethoxy-1-azabicyclo[2.2.1]heptane (1g, 4.7mmol) in tetrahydrofuran (40ml), at -78°C, and stirred for 2 hours. A solution of 5-chloro-3-methyl-1,2,4-thiadiazole (1g, 7.4mmol) in tetrahydrofuran (5ml) was added to the reaction mixture at -78°C, stirred for 1 hour and then warmed to room temperature and stirred for 16 hours. Water (25ml) and dichloromethane (70ml) were added and the mixture extracted with dichloromethane (4 x 150ml). The combined extracts were dried (Na₂SO₄), evaporated, and the crude product chromatographed through silica-gel using dichloromethane/methanol (95:5) as eluant to give the title compound (0.4g) as a pale yellow oil, m/e 314 (CI⁺, [M+1]⁺); δ (360MHz, CDCl₃) 2.64 (3H, s, CH₃), 2.74-2.85 and 2.97-3.02 (3H and 1H respectively, each m), 3.16 (3H, s, OCH₃), 3.27 (4H, s, 4CH and OCH₃), 3.70 (3H, s, CO₂CH₃), 3.68 (1H, dd, J = 12.7 and 1.5Hz), 3.80 (1H, dd, J = 12.7 and 3Hz).

### e) exo-3-[5-(3-Methyl-1,2,4-thiadiazol)-yl]-5,5-dimethoxy-1-azabicyclo[2.2.1]heptane

Sodium hydroxide (6ml of a 5N solution) was added to a solution of the preceding ester (0.4g, 1.3mmol) in methanol (2ml) and heated at 70°C for 1.5 hours. The solution was adjusted to pH1 with concentrated hydrochloric acid and stirred at room temperature for 16 hours. The methanol was removed under vacuum, dichloromethane (75ml) added, and the aqueous basified with potassium carbonate. The residue remaining (0.2g) after extraction into dichloromethane (4 x 75ml), drying (Na₂SO₄) and removal of solvent under vacuum, was taken up into methanol (2ml) and sodium methoxide (50mg, 1mmol) added. The solution was stirred at room temperature for 1 hour before removing the solvent under vacuum and chromatography of the residue through alumina using dichloromethane/methanol (97:3) as eluant to give exo-3-[5-(3-methyl-1,2,4-thiadiazol)-yl]-5,5-dimethoxy-1-azabicyclo[2.2.1]heptane (0.2g); m/e 256 (CI⁺, [M+1]⁺); δ (360MHz, CDCl₃) 2.45 (1H, dd, J = 12.7 and 3.2Hz), 2.64 (3H, s, CH₃), 2.78 (1H, dd, J = 10.2 and 3.2Hz), 2.90 (1H, s, 4CH), 2.95 (1H, d, J = 12.7Hz), 3.01 (1H, d, J = 10.9Hz), 3.10 (1H, dd, J = 14 and 5Hz), 3.19 (1H, m) 3.22 (3H, s, OCH₃), 3.27 (3H, s, OCH₃) and 3.70 (1H, m, 3CH).

### f) exo-3-[5-(3-Methyl-1,2,4-thiadiazol)-yl]-1-azabicyclo[2.2.1]heptan-5-one

A solution of exo-3-[5-(3-methyl-1,2,4-thiadiazol)-yl]-5,5-dimethoxy-1-azabicyclo[2.2.1] heptane (0.2g, 0.8mmol) in perchloric acid (3ml of 70% solution in water) was heated at 65°C for 2 hours. Water (20ml) and dichloromethane (40ml) were added to the reaction mixture and the aqueous basified with sodium carbonate. Extraction into dichloromethane (5 x 60ml), drying (Na₂SO₄) and removal of solvent under vacuum gave the title ketone as a crystalline solid (0.14g) mp 79-83°C; m/e 210 (CI⁺, [M+1]⁺); δ (360MHz, CDCl₃) 2.66 (3H, s, CH₃), 2.93 (1H, dd, J = 17.9 and 4.2Hz), 3.07 (1H, s, 4CH), 3.12-3.16 (2H, m), 3.29-3.44 (3H, m) and 3.70-3.74 (1H, m, 3CH).

### g) exo-3-[5-(3-Methyl-1,2,4-thiadiazol)-yl]endo-5-hydroxy-1-azabicyclo[2.2.1]heptane

Sodium borohydride (50mg, 1.3mmol) was added to a stirred solution of the preceding ketone (0.14g, 0.7mmol) in ethanol (10ml), at 10°C. After 0.5 hours at 10°C the solution was warmed to room temperature and stirred for a further 0.5 hours. Excess reagent was destroyed by addition of 2N hydrochloric acid and the solvents then removed under vacuum. The residue was taken up into water (15ml), basified with potassium carbonate and extracted into dichloromethane (5 x 50ml). The combined extracts were dried (Na₂SO₄) and the solvent removed under vacuum to give exo-3-[5-(3-methyl-1,2,4-thiadiazol)-yl]-endo-5-hydroxy-1-azabicyclo[2.2.1]heptane (0.1g) as a crystalline solid, mp 127-131°C (ethyl acetate); (Found: C, 51.14; H. 6.22; N, 19.88. C₉H₁₃N₃SO requires C, 51.15; H, 6.20; N, 19.89%); m/e 212 (CI⁺, [M+1]⁺), δ (360MHz, CDCl₃) 1.8-2.1 (1H, broad s, OH), 2.18 (1H, dt, J = 3.6 and 12.6 Hz, endo 6CH), 2.64 (3H, s, CH₃), 2.66 (1H, d, J = 11.5Hz, one of 7CH₂), 2.84 (1H, dd, J = 3.6 and 11.5 Hz, one of 7CH₂), 2.86 (1H, d, J = 4.8 Hz, 4CH), 3.11-3.20 (2H, m, exo 2CH and exo 6CH), 3.27 (1H, ddd, J = 2.5, 8.0 and 12.0 Hz, endo 2CH), 4.08 (1H, dd, J = 6.2 and 8.0 Hz, 3CH) and 4.53 (1H, quintet, J = 3.6 Hz, 5CH).

### EXAMPLE 12

### 3-[5-(3-iso-Propyl-1,2,4-thiadiazol)-yl]quinuclidine Hydrogen Oxalate

The title compound free base was prepared from 3-methoxycarbonyl quinuclidine (2.0g, 11.8mmol) and 5-chloro-3-iso-propyl-1,2,4-thiadiazole (2.5g, 15.4mmol) by the method of Example 5. Treatment with oxalic acid and crystallisation from dichloromethanediethyl ether gave the title compound (380mg), mp 115-117°C; (Found: C, 51.29; H, 6.37; N. 12.69. C₁₂H₁₉N₃S.(COOH)₂ requires C, 51.36; H, 6.46; N, 12.83%); m/e 237 (M⁺ of free base); δ (360MHz, D₂O) 1.33 and 1.35 (each 3H, each s, 2 x CH₃), 1.88-1.93 and 2.10-2.22 (each 2H, each m, 5CH₂ and 8CH₂), 2.51-2.56 (1H, m, 4CH), 3.30-3.50 (5H, m, 6CH₂, 7CH₂ and CH(CH₃)₂), 3.81 (1H, ddd, J = 1.9, 7 and 13 Hz, one of 2CH₂), 3.87 (1H, ddd, J = 2.4, 10 and 13 Hz, one of 2CH₂) and 4.08 (1H, ddd, J = 2.4, 7 and 10 Hz, 3CH).

### EXAMPLE 13

### exo- and endo-3-[5-(3-Ethyl-1,2,4-thiadiazol)-yl]-1-azabicyclo[2.2.1]heptane Hydrogen Oxalate

Reaction of 3-methoxycarbonyl-1-azabicyclo[2.2.1] heptane (1.37g, 8.8mmol) with 5-chloro-3-ethyl-1,2,4-thiadiazole (1.7g, 11.5mmol) gave:
a) endo-3-[5-(3-Ethyl-1,2,4-thiadiazol)-yl]-1-azabicyclo[2.2.1]heptane hydrogen oxalate (370mg), mp 142-143°C; (Found: C, 48.14; H, 5.69; N. 14.03. C₁₀H₁₅N₃S.(COOH)₂ requires C, 48.15; H, 5.72; N, 14.04%); m/e 209 (M⁺ of free base); δ (360MHz, D₂O) 1.32 (3H, t, J = 7.6Hz, CH₃); 1.62-1.72 and 1.98-2.10 (each 1H, each m, 5CH₂), 3.00 (2H. q, J = 7.6Hz, CH₂CH₃), 3.32-3.60 (5H, m, 4CH, 6CH₂ and 7CH₂), 3.67 (1H, ddd, J = 2.2, 5.7 and 12.0Hz, one of 2CH₂), 3.98 (1H, dt, J = 2.9 and 12.0Hz, one of 2CH₂) and 4.34-4.42 (1H, m, 3CH).
b) exo-3-[5-(3-Ethyl-1,2,4-thiadiazol)-yl]-1-azabicyclo[2.2.1]heptane hydrogen oxalate (370mg), mp 133-135°C; (Found: C, 48.00; H, 5.64; N, 13.98. C₁₀H₁₅N₃S.(COOH)₂ requires C, 48.15; H, 5.72; N, 14.04%); m/e 209 (M⁺ of free base); δ (360MHz, D₂O) 1.31 (3H, t, J = 7.6Hz, CH₃), 1.98-2.10 and 2.22-2.34 (each 1H, each m, 5CH₂), 2.97 (2H, q, J = 7.6Hz, CH₂CH₃), 3.26 (1H, d, J = 5Hz, 4CH), 3.29 (1H, d, J = 10Hz, one of 7CH₂), 3.32-3.43 (1H, m, one of 6CH₂), 3.52 (1H, d, J = 10Hz, one of 7CH₂), 3.50-3.58 (1H, m, one of 6CH₂), 3.82 (2H, d, J = 7.2Hz, 2CH₂) and 3.98 (1H, t, J = 7.2Hz, 3CH).

### EXAMPLE 14

### 1-Methyl-3-[5-(3-methyl-1,2,4-thiadiazol)-yl]-1,2,5,6-tetrahydropyridine Hydrochloride

### a) 3-[5-(3-methyl-1,2,4-thiadiazol)-yl]pyridine.

This compound was prepared from thionicotinamide and dimethylacetamide dimethylacetal and obtained as an oil after chromatography on silica gel; δ (360MHz, CDCl₃) 2.76 (3H, s,CH₃), 7.45 (1H, ddd, J = 1.2, 7.0 and 11.4Hz, 5CH), 8.24 (1H, ddd, J = 2.3, 3.2 and 11.4Hz, 4CH), 8.75 (1H, dd, J = 2.3 and 7.0Hz, 6CH) and 9.17 (1H, dd, J = 1.2 and 3.2Hz, 2CH).

### b) 1-Methyl-3-[5-(3-methyl-1,2,4-thiadiazol)-yl]-1,2,5,6-tetrahydropyridine Hydrochloride

The title compound was prepared from the foregoing pyridyl-thiadiazole by the method of Example 20 and obtained as a white crystalline solid, mp 182° (decomp.); (Found: C, 45.43; H, 6.04; N, 17.63. C₉H₁₃N₃S.HCl.0.33H₂O requires C, 45.78; H, 6.22; N, 17.68%); m/e 195 (M⁺ of free base); δ (250MHz, D₂O) 2.62 (3H, s, thiadiazole CH₃), 2.74-2.85 (2H, m, 5CH₂), 3.09 (3H, s, NCH₃), 3.30-3.46 (1H, m, one of 6CH₂), 3.64-3.76 (1H, m, one of 6CH₂), 4.09 (1H, dd, J = 2.4 and 16.0Hz, one of 2CH₂), 4.42 (1H, d, J = 16.0Hz, one of 2CH₂) and 7.03-7.10 (1H, m, 4CH).

### EXAMPLE 15

### 3-[5-(3-Methyl-1,2,4-thiadiazol)-yl]-1,2,5,6-tetrahydropyridine Hydrochloride

1-Methyl-3-[5-(3-methyl-1,2,4-thiadiazol)-yl]-1,2,5,6-tetrahydropyridine was treated with vinyl chloroformate and then methanolic hydrogen chloride to give the title compound, mp 177°C (decomp.). (Found: C, 41.05; H, 5.33; N, 17.86. C₈H₁₁N₃S. 1.5HCl requires C, 40.72; H, 5.34; N, 17.81%); m/e 181 (M⁺ of free base); δ (360MHz, D₂O) 2.63 (3H, s, CH₃), 2.68-2.76 (2H, m, 5CH₂), 3.47 (2H, t, J = 6.2Hz, 6CH₂), 4.17-4.22 (2H, m, 2CH₂) and 7.03-7.10 (1H, m, 4CH).

### EXAMPLE 16

### 3-[5-(3-(1-Hydroxy-1-phenylmethyl)-1,2,4-thiadiazol)-yl]quinuclidine Hydrogen Oxalate

### a) α-(Tetrahydropyranyloxy)-phenylacetamidine Hydrochloride

### To a solution of sodium (230mgs, 10mmol) in dry ethanol was added α-(tetrahydropyranyloxy)benzylcyanide (21.7g, 100mmol, J.R. Anderson. R.L. Edwards and A.J.S. Whalley, JCS Perkin I, 215, (1982)). After stirring for 16 hours the reaction was cooled to -50°C and 50ml of dry ammonia (50ml) was condensed into the solution. Dry ammonium chloride (5.3g, 100mmol) was added and the reaction allowed to warm to room temperature overnight. After filtration and evaporation of the solvents the residue was taken up into water (200ml), washed with dichloromethane (2 x 200ml) and evaporated to give a white solid (22.9g) mp 53-55°C: m/e 235 (M+H)⁺; δ (360MHz; D₂O) 1.50-1.86 (6H, m, 3 x CH₂); 3.49-3.53, 3.60-3.69 and 3.91-3.96 (0.5H, 1H and 0.5H respectively, CH₂O); 4.64 and 5.00 (each 0.5H, each t, J = 7Hz, CHO-); 5.61 and 5.64 (each 0.5H, each s, PhCH); 7.47-7.61 (5H, m, C₆H₅).

### b) 3-(1-Phenyl-1-tetrahydropyranyloxymethyl)-5-chloro-1,2,4-thiadiazole

The foregoing amidine (22.8g, 85mmol) was dissolved in cold 4.2N sodium hydroxide solution (120ml, 0.5mol) and a solution of perchloromethyl mercaptan (19.5g, 110mmol) in dichloromethane (120ml) was added to the vigorously stirred reaction mixture over 1 hour. After a further hour the organic layer was separated and the aqueous solution was re-extracted three times with dichloromethane to give an oil which was purified by silica gel chromatography eluting with hexane-diethyl ether to give the title compound (10.0g); m/e 209 (M-C₅H₉O₂)⁺; (Found: C, 54.49; H, 4.96; N, 8.92; C₁₄H₁₅N₂O₂SCl requires C, 54.10; H, 4.86; N, 9.01%); δ (360MHz, CDCl₃) 1.51-1.93 (6H, m, 3 x CH₂); 3.46-3.54, 3.73-3.80 and 3.88-3.94 (1H, 0.5H and 0.5H respectively, each m, CH₂O); 4.69 and 4.84 (each 0.5H, t, J = 3Hz, CHO-); 6.04 and 6.09 (each 0.5H, each s, PhCH); 7.25-7.55 (5H, m, Ph).

### c) 3-[5-(3-(1-Hydroxy-1-phenylmethyl)-1,2,4-thiadiazol)-yl]quinuclidine Hydrogen oxalate.

Reaction of 3-methoxycarbonyl quinuclidine (4.0g. 24mmol) with 3-(1-phenyl-1-tetrahyropyranyloxymethyl)-5-chloro-1,2,4-thiadiazole (7.75g, 24mmol) by the method of Example 5 gave the title compound free base (1.1g, 15%) which was treated with oxalic acid to give the hydrogen oxalate salt (1.4g); mp = 61-62°C; m/e 301 (M⁺ of free base); δ (360MHz, D₂O) 1.70-1.90 and 2.06-2.25 (each 2H, each m, 5CH₂ and 8CH₂), 1.45-1.52 (1H, m, 4CH), 3.24-3.47 (4H, m, 6CH₂ and 7CH₂), 3.73-3.87 (2H, m, 2CH₂), 4.06-4.13 (1H, m, 3CH), 6.14 (1H, s, CHOH) and 7.36-7.50 (5H, m, Ph).

### EXAMPLE 17

### 3-[5-(3-Benzoyl-1,2,4-thiadiazol)-yl]-quinuclidine Hydrogen oxalate

3-[5-(3-(1-Hydroxy-1-phenylmethyl)-1,2,4-thiadiazol)-yl]quinuclidine (1.0g, 3.3mmol) in dichloromethane (50ml) was stirred with activated manganese dioxide (5g). After 0.5hour, the reaction was filtered and the manganese dioxide repeatedly washed with dichloromethane. The combined extracts were evaporated to yield the title compound free base (1.0g) which was treated with oxalic acid. Crystallisation from diethyl ether gave the title compound, mp 95-97°C (decomp. ); m/e 299 (M⁺ of free base); (Found: C, 53.94; H, 5.15; N, 10.20; C₁₆H₁₇N₃OS.(COOH)₂.0.6H₂O requires C, 54.02; H, 5.09; N, 10.49%); δ (360MHz, D₂O) 1.92-1.98 and 2.16-2.34 (each 2H, each m, 5CH₂ and 8CH₂), 2.58-2.62 (1H, m, 4CH), 3.36-3.58 (4H, m, 6CH₂ and 7CH₂), 3.84-4.04 (2H, m, 2CH₂), 4.22-4.30 (1H, m, 3CH), 7.62 (2H, t, J = 8Hz, 3H and 5H of Ph), 7.79 (1H, t, J = 8Hz, 4H of Ph) and 8.13 (2H, d, J = 8Hz, 2H and 6H of Ph).

### EXAMPLE 18

### 3-[5-(3-(1,1-Diphenyl-1-hydroxymethyl)-1,2,4-thiadiazol)-yl]quinuclidine Hemi-Hydrogen Oxalate

3-[5-(3-Benzoyl)-1,2,4-thiadiazol)-yl]-quinuclidine (0.92g, 3.1mmol) in dry tetrahydrofuran (50ml) under an atmosphere of dry nitrogen was treated with phenyl magnesium bromide (3ml of a 3M solution in diethyl ether) at room temperature for 2 hours. Saturated ammonium chloride solution was added and the mixture partitioned and extracted twice with dichloromethane. The combined organic solutions were dried and concentrated to give a residue which was purified by chromatography to yield the title compound free base an an oil (633mg). Treatment with oxalic acid in diethyl ether gave the title compound, mp 185-186°C; (Found: C, 63.22; H, 5.71; N, 9.55. C₂₂H₂₃N₃OS. (COOH). 0.9H₂O requires C, 62.96; H, 5.93; N, 9.58%); m/e 377 (M⁺ of free base); δ (360MHz, D₂O) 1.72-1.96 and 2.04-2.26 (each 2H, each m, 5CH₂ and 8CH₂), 2.46-2.50 (1H, m, 4CH), 3.26-3.40 (4H, m, 6CH₂ and 7CH₂), 3.73-3.83 (2H, m, 2CH₂), 4.10-4.15 (1H, m, 3CH) and 7.31-7.42 (10H, m, 2 x Ph).

### EXAMPLE 19

### 3-[5-(3-(1,1-Diphenyl-1-fluoromethyl)-1,2,4-thiadiazol)-yl]quinuclidine Hydrogen Oxalate

3-[5-(3-(1,1-Diphenyl-1-hydroxymethyl)-1,2,4-thiadiazol-yl]quinuclidine (377mg, 1mmol) in dichloromethane (5ml) was treated with diethylamino sulphurtrifluoride (1.7g, 6.3mmol) at -78°C. The reaction was allowed to warm slowly to room temperature then aqueous potassium carbonate was added and extracted with dichloromethane. The extracts were dried and concentrated in vacuo to give an oil which was treated with oxalic in diethylether yielding the title compound, mp 114-115°C; (Found: C, 59.34; H, 5.26; N, 8.65. C₂₂H₂₂N₃FS.(COOH)₂. H₂O requires C, 59.13; H, 5.38; N, 8.62%); m/e 379 (M⁺ of free base); δ (360MHz, D₂O) 1.64-1.90 and 2.02-2.25 (each 2H, each m, 5CH₂ and 8CH₂), 2.42-2.52 (1H, m, 4CH), 3.18-3.44 (4H, m, 6CH₂ and 7CH₂), 3.67-3.90 (2H, m, 2CH₂), 4.07-4.18 (1H, m, 3CH) and 7.24-7.50 (10H, m, 2 x Ph).

### EXAMPLE 20

### 6-[5-(3-Methyl-1,2,4-thiadiazol)-yl]-1-azabicyclo[3.2.1.]octane

### a) 1-Ethoxycarbonylmethyl-3-ethoxycarbonylpiperidine

Ethyl bromoacetate (21.2g, 0.127mol) was added dropwise to a solution of 3-methoxycarbonylpiperidine (40g, 0.254mol) in diethyl ether (250ml) at 0°C. The reaction was heated under reflux for 1 hour and the resulting precipitate filtered off and washed with diethyl ether. The ethereal solution was concentrated under reduced pressure to give the title compound (27.6g), δ (60MHz, CDCl₃) 1.25 and 1.27 (each 3H, each t, J = 7Hz, 2 x OCH₂CH₃), 1.40-3.70 (9H, m, 2CH₂, 3CH, 4CH₂, 5CH₂ and 6CH₂), 3.20 (2H, s, NCH₂CO₂), 4.10 and 4.15 (each 2H, each q, J = 7Hz, 2 x NCH₂CO₂).

### b) 1-Azabicyclo[3.2.1]octan-6-one

The foregoing diester (30.0g. 0.123mol) in toluene (300ml) was added dropwise to a solution of potassium tert-butoxide (41.4g, 0.37mmol) in toluene (11) at reflux under an atmosphere of nitrogen with vigorous stirring. After complete addition (2.5 hours) the reaction was allowed to cool and the solvent decanted from the resulting solid. The solid was heated under reflux in concentrated hydrochloric acid (600ml) for 16 hours then reduced under high vacuum. The residue was added to aqueous potassium carbonate which was extracted (3 x) with dichloromethane. The combined extracts were dried (Na₂SO₄) and concentrated to give the title compound (8.8g) as a crystalline solid, mp 83-87°C; m/e 125 (M⁺); δ (360MHz, CDCl₃) 1.3-2.4 (5H, m, 3CH₂, 4CH₂ and 5CH), 2.6-3.6 (6H, m, 2CH₂, 7CH₂ and 8CH₂).

### c) 6-(1,3-Dithian-2-ylidene)-1-azabicyclo[3.2.1]octane

n-Butyl lithium (6.05ml of a 1.6M solution in hexane, 9.7mmol) was added dropwise to a solution of 2-trimethylsilyl-1,3-dithiane (1.86g, 9.7mmol) in tetrahydrofuran (30ml) at -30°C and the reaction mixture stirred for 2 hours. A solution of 1-azabicyclo[3.2.1]octan-6-one (1.1g, 8.8mmol) in tetrahydrofuran (10ml) was added dropwise and the reaction mixture allowed to warm to room temperature. Water (20ml) was added and extracted (3 x) with dichloromethane. The combined extracts were dried (MgSO₄) and concentrated and the residue purified by chromatography on alumina eluting with dichloromethane/methanol (97:3) to give the title compound as a colourless oil (2g); m/e 227 (M⁺); δ (360MHz, CDCl₃) 1.23-1.38 and 1.56-1.86 (1H and 3H respectively, each m, 3CH₂ and 4CH₂), 2.08-2.24 (2H, m, SCH₂CH₂), 2.76-3.00 (9H, m, 2 x SCH₂, 2CH₂, 5CH and 8CH₂), 3.42 (1H, d, J = 17Hz, one of 7CH₂) and 3.56 (1H, d, J = 17Hz, one of 7CH₂).

### d) 6-Methoxycarbonyl-1-azabicyclo[3.2.1]octane

The preceding compound (2.0g, 8.8mmol) was stirred in methanolic hydrogen chloride (75ml) at 55°C for 5 hours then the solvent was removed in vacuo. The residue was treated with potassium carbonate solution and extracted (4 x) with dichloromethane. The combined extracts were dried (Na₂SO₄) and concentrated to give the title compound as an oil (0.5g), characterised as the hydrochloride salt, mp 151-154°C; (Found: C, 51.92; H, 7.65; N, 6.83, C₉H₁₆NO₂Cl. 0.125H₂O requires: C, 51.98, H, 7.82; N, 6.73%); δ (360MHz, D₂O) 1.30-1.41 and 1.54-1.72 (1H and 3H respectively, each m, 3CH₂ and 4CH₂), 2.41-2.48 (1H, m, 5CH), 2.78-2.87 (5H, m, 2CH₂, 6CH and 8CH₂), 3.05-3.20 (2H, m, 7CH₂) and 3.68 (3H, s, CH₃).

### e) 6-[5-(3-Methyl-1,2,4-thiadiazol)-yl]-1-azabicyclo[3.2.1]octane

The foregoing ester (6.0g, 35.5mmol in tetrahydrofuran (250ml) was treated with lithium diisopropylamide-tetrahydrofuran complex (35.5ml of a 1.5M solution in cyclohexane, 53.3mmol) at -78°C under an atmosphere of nitrogen. After 1 hour 5-chloro-3-methyl-1,2,4-thiadiazole (7.17g, 53.3mmol) was added and the reaction allowed to warm alowly to room temperature over 3 hours. The solvent was removed under reduced pressure and the residue in aqueous potassium carbonate extracted with dichloromethane. The combined extracts were dried and concentrated and the residue stirred in methanol (80ml) and 2N NaOH (80ml) for 2 hours. The methanol was evaporated off and the aqueous solution extracted with ethyl acetate (3 x). The remaining aqueous solution was adjusted to pH2 for 2 hours then made basic with potassium carbonate and extracted (3 x) with dichloromethane. The combined extracts, dried and concentrated, gave an oil which was treated with sodium methoxide (250mg) in methanol (5ml) for 1 hour. The solvent was removed in vacuo and the residue purified by chromatography on alumina (eluting with methanol/dichloromethane (2:98) to give the title compound (80mg), mp 62-63°C; (Found: C, 57.5; H, 7.3; N, 19.8. C₁₀H₁₅N₃S requires: C, 57.4; H, 7.2; N. 20.1%); m/e 209 (M⁺); δ (360MHz, CDCl₃) 1.64-1.84 (4H, m, 3CH₂ and 4CH₂), 2.46-2.50 (1H, m, 5CH), 2.64 (3H, s, CH₃), 2.87-3.04 (4H, m, 2CH₂ and 8CH₂), 3.15 (1H, dd, J = 5 and 13Hz, one of 7CH₂), 3.52 (1H, ddd, J = 2, 8 and 13Hz, one of 7CH₂), 3.68 (1H, dd, J = 5Hz and 8Hz, 6CH).

### EXAMPLE 21

### 3-[5-(3-Dimethylamino-1,2,4-thiadiazol)-yl] 1,2,5,6-tetrahydropyridine Hydrochloride

Treatment of 3-[5-(3-Dimethylamino-1,2,4-thiadiazol)-yl]-1-methyl-1,2,5,6-tetrahydropyridine with vinyl chloroformate then methanolic hydrogen chloride gave the title compound, mp 203-204°C: (Found: C, 42.34; H, 5.92; N, 21.40. C₉H₁₄N₄S. 1.3HCl requires: C, 41.95; H, 5.98; N, 21.74%); (Found: M⁺ = 210.0930. C₉H₁₄N₄S requires: M⁺ = 210.0939); δ (360MHz, D₂O) 2.64-2.72 (2H, m, 5CH₂), 3.15 (6H, s, 2 x CH₃), 3.46 (2H, t, J = 6.2Hz, 6CH₂), 4.16-4.20 (2H, m, 2CH₂) and 6.94-7.00 (1H, m, 4CH).

### EXAMPLE 22

### exo- and endo-3-[5-(3-iso-Propyl-1,2,4-thiadiazol)-yl]-1-azabicyclo[2.2.1]heptane Hydrogen Oxalate

Reaction of 3-methoxycarbonyl-1-azabicyclo[2.2.1] heptane (1.25g, 8.0mmol) with 5-chloro-3-iso-propyl-1,2,4-thiadiazole (1.70g, 10.5mmol) gave:
a) endo-3-[5-(3-iso-Propyl-1,2,4-thiadiazol)-yl]-1-azabicyclo[2.2.1]heptane hydrogen oxalate (531 mg), mp 127°C; (Found: C, 49.87; H, 6.04, N, 13.36. C₁₁H₁₇N₃S. (COOH)₂ requires: C, 49.83; H, 6.11, N, 13.41%); m/e 223 (M⁺ of free base); δ (360MHz, D₂O) 1.34 (6H, d, J = 7.0Hz, 2 x CH₃), 1.62-1.71 and 1.99-2.10 (each 1H, each m, 5CH₂), 3.34 (1H, septet, J = 7.0Hz, CH(CH₃)₂), 3.35-3.45 (3H, m, 4CH, one of 6CH₂ and one of 7CH₂), 3.50 (1H, ddd, J = 2.9, 5.1 and 12.0Hz, one of 6CH₂), 3.54-3.59 (1H, m, one of 7CH₂), 3.69 (1H, ddd, J = 2.3, 5.7 and 12.0Hz, one of 2CH₂), 3.98 (1H, dt, J = 2.9 and 12.0Hz, one of 2CH₂), 4.34-4.41 (1H, m, 3CH).
b) exo-3-[5-(3-iso-Propyl-1,2,4-thiadiazol)-yl]-1-azabicyclo[2.2.1]heptane hydrogen oxalate (421mg), mp 131°C; (Found: C, 49.77; H, 6.07; N, 13.36. C₁₁H₁₇N₃S. (COOH)₂ requires: C, 49.83; H, 6.11; N, 13.41%); m/e 223 (M⁺ of free base); δ (360MHz, D₂O) 1.32 (6H, d, J = 7.0HZ, 2 x CH₃), 1.98-2.08 and 2.22-2.32 (each 1H, each m, 5CH₂), 3.24-3.43 (4H, m, CH(CH₃)₂, 4CH, one of 6CH₂ and one of 7CH₂), 3.48-3.58 (2H, m, one of 6CH₂ and one of 7CH₂), 3.78-3.85 (2H, m, 2CH₂) and 3.93-3.99 (1H, m, 3CH).

### EXAMPLE 23

### (1R*,6R*) and (1R*,6S*) 6-[5-(3-Cyclopropyl-1,2,4-thiadiazol)-yl]-2-azabicyclo[2.2.2]octane Hydrogen Oxalate

### a) Methyl 2-t-butyloxycarbonyl-2-azabicyclo [2.2.2]octane-6-carboxylate

Di-t-butyldicarbonate (21.8g, 0.10mol) in dry dichloromethane (50ml) was added dropwise to a stirred, cooled (0°C) solution of methyl 2-azabicyclo[2.2.2]octane-6-carboxylate (18.2g, 0.09mol, mixture of endo and exo isomers, prepared as described in Example 21a, EP 0239309) in dry dichloromethane (100ml). The resulting solution was stirred at room temperature for 4 hours, water (100ml) was added and the mixture was stirred for 15 minutes. The organic layer was separated and washed with 0.5M hydrochloric acid (100ml), water (100ml), saturated sodium hydrogen carbonate solution (100ml), water (100ml) then dried (sodium sulphate) and evaporated to dryness. The residue was purified by column chromatography on silica by elution with ethyl acetate/petroleum ether (60-80) [1:40] to give Isomer A as a colourless oil which crystallised on standing (12.0g), mp 44-45°C; Rf = 0.35 in ethyl acetate/petroleum ether (60-80) [1:1] on silica; (Found: C, 62.59; H, 8.55; N, 5.10. C₁₄H₂₃NO₄ requires: C, 62.43; H, 8.61; N, 5.20%); νₘₐₓ (film) 1740 and 1695cm⁻¹ (C=O); δ (360MHz, CDCl₃) 1.47 (9H, s C(CH₃)₃), 1.55-2.20 (7H, m, 4CH, 5CH₂, 7CH₂ and 8CH₂), 2.86-3.00 (1H, m, 6CH), 3.30 (2H, broad s, 3CH₂), 3.69 and 3.72 (total 3H, each broad s, CO₂CH₃, rotamers), 4.21 and 4.38 (total 1H, each broad s, 1CH, rotamers).

Mixed fractions were collected (1:1 mixture, 4.80g) followed by Isomer B as a colourless oil (6.80g), Rf = 0.32 in ethyl acetate/petroleum ether (60-80) [1:1] on silica; δ (360MHz, CDCl₃) 1.42 and 1.43 (total 9H, each s, C(CH₃)₃, rotamers), 1.52-2.20 (7H, m, 4CH, 5CH₂, 7CH₂ and 8CH₂), 2.63-2.73 (1H, m, 6CH), 3.19-3.25 (1H, m, 3CH), 3.36-3.42 (1H, m, 3CH), 3.66 and 3.69 (total 3H, each s, CO₂CH₃, rotamers), 4.27-4.30 and 4.36-4.38 (total 1H, each m, 1CH, rotamers), m/e 269 (M⁺).

### b) (1R*,6R*) and (1R*,6S*) 2-t-Butyloxycarbonyl-6-[5-(3-cyclopropyl-1,2,4-thiadiazol)-yl]-2-azabicyclo[2.2.2]octane

A freshly prepared solution of lithium diisopropylamide (prepared from n-butyllithium (8.8ml of a 1.6M solution) and diisopropylamine (1.96ml, 14mmol) in dry tetrahydrofuran (10ml at -78°C) was added dropwise to a cooled (-78°C), stirred solution of the preceding ester (2.50g. 9.3mmol, Isomer A) in dry tetrahydrofuran (30ml) under a nitrogen atmosphere. After 2 hours at -78°C a solution of 5-chloro-3-cyclopropyl-1,2,4-thiadiazole (2.25g, 14mmol) in dry tetrahydrofuran (5ml) was added dropwise. After 2 hours at -78°C the reaction mixture was allowed to warm to room temperature over 16 hours and the solvent was removed in vacuo. The residue was treated with methanol (20ml) and 2M sodium hydroxide solution (20ml). After 2 hours the solution was washed with ethyl acetate (20ml) then the aqueous was adjusted to pH1 with concentrated hydrochloric acid and left standing at room temperature for 24 hours. This solution was extracted with diethyl ether (2 x 20ml) and the combined organics were dried (sodium sulphate) then evaporated to dryness to give an orange oil (1.70g) which was purified by column chromatography on silica by elution with ethyl acetate/petroleum ether (60-80) [1:10] to give Isomer A (1R*,6R*) as a pale yellow oil which crystallised on standing (250mg), mp 90°C; Rf = 0.62 in ethyl acetate/petroleum ether (60-80) [1:1] on silica; (Found: C, 60.82; H, 7.46; N, 12.38. C₁₇H₂₅N₃SO₂ requires: C, 60.86; H, 7.51; N, 12.53%); νₘₐₓ (film) 1695cm⁻¹ (C=O); δ (360MHz, CDCl₃) 1.00-1.16 (4H, m, 2 x cyclopropyl (CH₂), 1.49 (9H, s, C(CH₃)₃), 1.58-1.78 (4H, m), 2.00-2.12 (2H, m) and 2.18-2.36 (2H, m, cyclopropyl CH, 4CH, 5CH₂, 7CH₂ and 8CH₂). 3.39 and 3.40 (total 2H, each broad s, 3CH₂, rotamers), 3.58-3.70 (1H, m, 6CH); 4.19 and 4.36 (total 1H, each broad s, 1CH, rotamers).

Isomer B (1R*,6S*) was isolated as a pale yellow oil (660mg), Rf = 0.56 in ethyl acetate/petroleum ether (60-80) [1:1] on silica; (Found: C, 60.84; H, 7.42; N, 12.80. C₁₇H₂₅N₃SO₂ requires: C, 60.86; H, 7.51; N, 12.53%); νₘₐₓ (film) 1690cm⁻¹ (C=O). δ (360MHz, CDCl₃) 1.00-1.18 (4H, m, 2 x cyclopropyl CH₂), 1.28 and 1.44 (total 9H, each s, C(CH₃)₃, rotamers), 1.56-2.36 (8H, m, cyclopropyl CH, 4CH, 5CH₂, 7CH₂ and 8CH₂), 3.32-3.58 (3H, m, 3CH₂ and 6CH), 4.11-4.15 (m) and 4.24 (total 1H, broad s, 1CH, rotamers).

### c) (1R*,6R*) and (1R*,6S*) 6-[5-(3-Cyclopropyl-1,2,4-thiadiazol)-yl]-2-azabicyclo[2.2.2]octane Hydrogen Oxalate

To a cooled (4°C) solution of (1R*,6R*) 2-t-butyloxycarbonyl-6-[5-(3-cyclopropyl-1,2,4-thiadiazol)-yl]-2-azabicyclo[2.2.2]octane (235mg), 0.7mmol) in dichloromethane (5ml) was added trifluoroacetic acid (1ml, 14mmol). After stirring at room temperature for 4 hours water (20ml) was added and the mixture stirred for 10 minutes. The aqueous layer was separated, basified with potassium carbonate and extracted with ethyl acetate (4 x 20ml). The combined organics were dried (Na₂SO₄) and evaporated to dryness to afford (1R*,6R*) 6-[5-(3-cyclopropyl-1,2,4-thiadiazol)-yl]-2-azabicyclo[2.2.2]octane free base as a pale yellow oil (140mg). The hydrogen oxalate salt had mp 165-168°C (aqueous propan-2-ol); m/e 235 (M⁺ of free base); δ (360MHz, D₂O) 1.04-1.16 (4H, m, 2 x cyclopropyl CH₂), 1.72-2.08 (5H, m, 5CH, 7CH₂ and 8CH₂), 2.16-2.22 (1H, m, 4CH). 2.30-2.36 (1H, m, cyclopropyl CH), 2.44-2.54 (1H, m, 5CH), 3.28-3.40 (2H, m, 3CH₂), 3.84 (1H, broad s, 1CH), 3.90-3.98 (1H, m, 6CH).

(1R*,6S*) 6-[5-(3-cyclopropyl-1,2,4-thiadiazol)-yl]-2-azabicyclo[2.2.2]octane free base (370mg) was obtained from (1R*,6S*) 2-t-butyloxycarbonyl-6-[5-(3-cyclopropyl-1,2,4-thiadiazol)-yl]-2-azabicyclo [2.2.2]octane (640mg, 1.9mmol) and trifluoroacetic acid (2.9ml, 38mmol) as described above. The hydrogen oxalate salt had mp 130-132°C (aqueous propan-2-ol); m/e 235 (M⁺ of free base); δ (360MHz, D₂O) 1.07-1.17 (4H, m, 2 x cyclopropyl CH₂), 1.80-2.20 (6H, m, 4CH, 5CH, 7CH₂ and 8CH₂), 2.30-2.48 (2H, m, cyclopropyl CH and 5CH), 3.29 (1H, d, J = 12Hz, 3CH), 3.39 (1H, d, J = 12Hz, 3CH), 3.82-3.90 (2H, m, 1CH and 6CH).

### EXAMPLE 24

### exo- and endo-3-[5-(3-n-Propyl-1,2,4-thiadiazol)-yl]-1-azabicyclo[2.2.1]heptane Hydrogen Oxalate

Reaction of 3-methoxycarbonyl-1-azabicyclo[2.2.1] heptane with 5-chloro-3-n-propyl-1,2,4-thiadiazole gave:
a) endo-3-[5-(3-n-propyl-1,2,4-thiadiazol)-yl]-1-azabicyclo[2.2.1]heptane hydrogen oxalate, mp 145-147°C; m/e 223 (M⁺ of free base); δ (360MHz, D₂O) 0.91 (3H, t, J = 7.4Hz, CH₃), 1.60-1.70 and 1.98-2.10 (each 1H, each m, 5CH₂), 1.79 (2H, sextet, J = 7.4Hz, CH₂CH₃), 2.96 (2H, t, J = 7.4Hz, CH₂CH₂CH₃), 3.32-3.42 and 3.46-3.58 (3H and 2H respectively, each m, 4CH, 6CH₂ and 7CH₂), 3.67 (1H. ddd, J = 2.3, 5.7 and 12.2Hz, one of 2CH₂), 3.99 (1H, dt, J = 3.0 and 12.2Hz, one of 2CH₂) and 4.36-4.44 (1H, m, 3CH).
b) exo-3-[5-(3-n-propyl-1,2,4-thiadiazol)-yl]-1-azabicyclo[2.2.1]heptane hydrogen oxalate, mp 129-130°C; m/e 223 (M⁺ of free base); δ (250MHz, D₂O) 0.90 (3H, t, J = 7.4Hz, CH₃), 1.78 (2H, sextet, J = 7.4Hz, CH₂CH₃), 1.95-2.08 and 2.19-2.34 (each 1H, each m, 5CH₂), 2.94 (2H, t. J = 7.4Hz, CH₂CH₂CH₂), 3.24-3.45 and 3.47-3.61 (3H and 2H respectively, each m, 4CH, 6CH₂ and 7CH₂), 3.83 (2H, d, J = 7.6Hz, 2CH₂) and 3.98 (1H, t, J = 7.6Hz, 3CH).

### EXAMPLE 25

### exo- and endo-3-[5-(3-Methoxy-1,2,4-thiadiazol)-yl]-1-azabicyclo[2.2.1]heptane Hydrogen Oxalate

Reaction of 3-methoxycarbonyl-1-azabicyclo[2.2.1]heptane with 5-chloro-3-methoxy-1,2,4-thiadiazole gave:
a) endo-3-[5-(3-Methoxy-1,2,4-thiadiazol)-yl]-1-azabicyclo[2.2.1]heptane hydrogen oxalate, mp 113-114°C; m/e 211 (M⁺ of free base); δ (360MHz, D₂O) 1.70-1.80 and 1.98-2.10 (each 1H, each m, 5CH₂), 3.30-3.42 and 3.46-3.56 (3H and 2H respectively, each m, 4CH, 6CH₂ and 7CH₂), 3.62 (1H, ddd, J = 2.3, 5.5 and 12.1Hz, one of 2CH₂), 3.91 (1H, dt, J = 2.9 and 12.1Hz, one of 2CH₂), 4.09 (3H, s, CH₃) and 4.28-4.36 (1H, m, 3CH).
b) exo-3-[5-(3-Methoxy-1,2,4-thiadiazol)-yl]-1-azabicyclo[2.2.1]heptane hydrogen oxalate. (Found: M⁺ = 211.0762. C₉H₁₃N₃OS (free base) requires M⁺ = 211.0779); δ (250MHz, D₂O) 1.93-2.06 and 2.18-2.35 (each 1H, each m, 5CH₂), 3.20-3.45 and 3.48-3.62 (3H and 2H respectively, each m, 4CH, 6CH₂ and 7CH₂), 3.79 (2H, d, J = 7Hz, 2CH₂), 3.90 (1H, t, J = 7Hz, 3CH) and 4.07 (3H, s, CH₃).

### EXAMPLE 26

### exo- and endo-3-[5-(3-Methylthio-1,2,4-thiadiazol)-yl]-1-azabicyclo[2.2.1]heptane Hydrogen Oxalate

Reaction of 3-methoxycarbonyl-1-azabicyclo[2.2.1] heptane with 5-chloro-3-methylthio-1,2,4-thiadiazole gave:
a) endo-3-[5-(3-Methylthio-1,2,4-thiadiazol)-yl]-1-azabicyclo[2,2.1]heptane hydrogen oxalate, mp 122-124°C; (Found: C, 38.65; H, 4.22; N, 10.63. C₉H₁₃N₃S.2(COOH)₂ requires C, 38.32; H, 4.20; N, 10.31%); m/e 227 (M⁺ of free base), δ (360MHz, D₂O) 1.66-1.78 and 1.98-2.10 (each 1H, each m, 5CH₂), 2.69 (3H, s, CH₃). 3.32-3.42 and 3.46-3.78 (3H and 2H respectively, each m, 4CH, 6CH₂ and 7CH₂), 3.68 (1H, ddd, J = 2.4, 5.6 and 12.1Hz, one of 2CH₂), 3.93 (1H, td, J = 3.0 and 12.1Hz, one of 2CH₂) and 4.36-4.42 (1H, m, 3CH).
b) exo-3-[5-(3-Methylthio-1,2,4-thiadiazol)-yl]-1-azabicyclo[2.2.1]heptane hydrogen oxalate, m/e 227 (M⁺ of free base); δ (250MHz, D₂O) 1.94-2.07 and 2.12-2.32 (each 1H, each m, 5CH₂), 2.67 (3H, s, CH₃), 3.20-3.44 and 3.46-3.60 (3H and 2H respectively, each m, 4CH, 6CH₂ and 7CH₂), 3.75-3.85 (2H, m, 2CH₂) and 3.97 (1H, dd, J = 5 and 7.5Hz, 3CH).

### EXAMPLE 27

### 3-[5-(3-n-Propyl-1,2,4-thiadiazol)-yl]quinuclidine Hydrogen Oxalate

The title compound free base was prepared from 3-methoxycarbonylquinuclidine and 5-chloro-3-n-propyl-1,2,4-thiadiazole by the method described in Example 5. The hydrogen oxalate salt was obtained as a gum, (Found: M⁺ = 237.1294. C₁₂H₁₉N₃S (free base) requires M⁺ = 237.12996); δ (250MHz, D₂O) 0.91 (3H, t, J = 7.4Hz, CH₃), 1.80 (2H, sextet, J = 7.4Hz, CH₂CH₃), 1.85-1.96 and 2.06-2.30 (each 2H, each m, 5CH₂ and 8CH₂), 2.50-2.58 (1H, m, 4CH), 2.96 (2H, t, J = 7.4Hz, CH₂CH₂CH₃), 3.28-3.50 (4H, m, 6CH₂ and 7CH₂), 3.74-3.94 (2H, m, 2CH₂) and 4.04-4.14 (1H, m, 3CH).

### EXAMPLE 28

### (1R*,6R*) and (1R*,6S*) 6-[5-(3-iso-Propyl-1,2,4-thiadiazol)-yl]-2-azabicyclo[2.2.2]octane Hydrogen Oxalate

The title compounds were prepared by the method of Example 35 using 5-chloro-3-iso-propyl-1,2,4-thiadiazole to give:
a) (1R*,6R*) 6-[5-(3-iso-Propyl-1,2,4-thiadiazol)-yl]-2-azabicyclo[2.2.2]octane hydrogen oxalate, mp 60-62°C (propan-2-ol/diethyl ether); m/e 237 (M⁺ of free base); δ (250MHz, D₂O) 1.33 (6H, d, J = 7Hz, (CH₃)₂), 1.66-2.12 (5H, m, 5CH, 7CH₂ and 8CH₂), 2.14-2.24 (1H, m, 4CH), 2.42-2.58 (1H, m, 5CH), 3.33 (1H, septet, isopropyl CH) overlapped with 3.36 (2H, s, 3CH₂), 3.87 (1H, broad s, 1CH) and 3.90-4.04 (1H, m, 6CH).
b) (1R*,6S*) 6-[5-(3-iso-Propyl-1,2,4-thiadiazol)-yl]-2-azabicyclo[2.2.2]octane hydrogen oxalate, (obtained as a 5:1 mixture of isomers), mp 45-48°C (propan-2-ol/diethyl ether); m/e 237 (M⁺ of free base); δ (250MHz, D₂O) 1.34 (6H, d, J = 7Hz, (CH₃)₂), 1.65-2.24 (6H, m, 4CH, 5CH, 7CH₂ and 8CH₂), 2.42-2.54 (1H, m, 5CH), 3.32 (1H, d, J = 11Hz, 3CH) overlapped with 3.33 (1H, septet, J = 7Hz, isopropyl CH), 3.37 (1H, d, J = 11Hz, 3CH) and 3.84-4.02 (2H, m, 1CH and 6CH).

### EXAMPLE 29

### exo-3-[5-(3-Isopropy1-1,2,4-thiadiazol)-yl]-endo-5-hydroxy-1-azabicyclo[2.2.1]heptane Hydrogen Oxalate

The title compound, prepared using 5-chloro-3-iso-propyl-1,2,4-thiadiazole, had mp 175-180°C; δ (360MHz, D₂O) 1.34 (6H, d, J = 7Hz, (CH₃)₂), 2.97 (1H, dt, J = 3.5 and 12Hz, 6CH), 3.29-3.38 (2H, m, isopropyl CH and 4CH), 3.44 (1H, d, J = 10Hz, 7CH), 3.62 (1H, dd, J = 3 and 10Hz, 7CH), 3.78-3.94 (3H, m, 2CH₂ and 6CH), 4.57 (1H, dd, J = 7Hz, 3CH) and 4.81-4.86 (1H, m, 5CH).

### EXAMPLE 30

### exo- and endo-3-[5-(3-Benzyl-1,2,4-thiadiazol)-yl]-1-azabicyclo[2.2.1]heptane

The title compounds were prepared by reaction of 3-methoxycarbonyl-1-azabicyclo[2.2.1]heptane (1.5g, 9.7mol) with 3-benzyl-5-chloro-1.2.4-thiadiazole (2.6g, 12.6mol). Chromatography on silica eluting with methanoldichloromethane (1:20) gave:
a) endo-3-[5-(3-Benzyl-1,2,4-thiadiazol)-yl]-1-azabicyclo[2.2.1]heptane (330mg), mp 40-41°C; m/e 271 (M⁺ of free base); δ (360MHz, CDCl₃) 1.22-1.31 and 1.40-1.51 (each 1H, each m, 5CH₂), 2.57-2.67. 2.70-2.75 and 2.84-2.95 (2H, 1H and 3H respectively, each m, one of 2CH₂, 4CH, 6CH₂ and 7CH₂), 3.34 (1H, dt, J = 3Hz and 12Hz, one of 2CH₂), 3.63-3.70 (1H, m, 3CH), 4.30 (2H, s, CH₂Ph) and 7.19-7.35 (5H, m, Ph).
b) exo-3-[5-(3-Benzyl-1,2,4-thiadiazol)-yl]-1-azabicyclo[2.2.1]heptane (310mg), mp 35-36°C; m/e 271 (M⁺ of free base); δ (360MHz, CDCl₃) 1.29-1.38 and 1.66-1.76 (each 1H, each m, 5CH₂), 2.43 (1H, d, J = 7.9Hz, one of 7CH₂), 2.50-2.59 (1H, m, one of 6CH₂), 2.73-2.81 (2H, m, 4CH and one of 7CH₂), 2.90 (1H, dt, J = 4.4 and 11.0Hz, one of 6CH₂), 3.00-3.09 (3H, m, 2CH₂ and 3CH), 4.28 (2H, m, CH₂Ph) and 7.20-7.36 (5H, m, Ph).

### EXAMPLE 31

### Tablet Preparation

Tablets containing 1.0, 2.0, 25.0, 26.0, 50.0 and 100.0 mg, respectlvely, of the following compounds are prepared as illustrated below:
endo-3-[5-(3-Methyl-1,2,4-thiadiazol)-yl]-1-azabicyclo[2.2.1]heptane hydrogen oxalate.
3-[5-(3-Benzyl-1,2,4-thiadiazol)-yl]quinuclidine hydrogen oxalate.
3-[5-(3-Dimethylamino-1,2,4-thiadiazol)-yl]-1,2,5,6-tetrahydropyridine hydrochloride.

| TABLE FOR DOSES CONTAINING FROM 1-25 MG OF THE ACTIVE COMPOUND | | | |
|---|---|---|---|
| | Amount-mg | | |
| Active Compound | 1.0 | 2.0 | 25.0 |
| Microcrystalline cellulose | 49.25 | 48.75 | 37.25 |
| Modified food corn starch | 49.25 | 48.75 | 37.25 |
| Magnesium stearate | 0.50 | 0.50 | 0.50 |

| TABLE FOR DOSES CONTAINING FROM 26-100 MG OF THE ACTIVE COMPOUND | | | |
|---|---|---|---|
| | Amount-mg | | |
| Active Compound | 26.0 | 50.0 | 100.0 |
| Microcrystalline Cellulose | 52.0 | 100.0 | 200.0 |
| Modified food corn starch | 2.21 | 4.25 | 8.5 |
| Magnesium stearate | 0.39 | 0.75 | 1.5 |

All of the active compound, lactose, and a portion of the corn starch are mixed and granulated to a 10% corn starch paste. The resulting granulation is sieved, dried and blended with the remainder of the corn starch and the magnesium stearate. The resulting granulation is then compressed into tablets containing 1.0 mg, 2.0 mg, 25.0 mg, 26.00 mg, 50.0 mg and 100.0 mg of active ingredient per tablet.

## Claims (Claims for the following Contracting State(s): AT, BE, CH, DE, FR, GB, IT, LI, LU, NL, SE)

1. A thiadiazole, or a salt or prodrug, which thiadiazole is substituted on one of the ring carbon atoms thereof with a non-aromatic azacyclic or azabicyclic ring system containing from 4 to 10 ring atoms & one nitrogen atom as the sole heteroatom; and substituted on the other ring atom with a substituent of low lipophilicity, having a rekker f value of not greater than 1.5, or a substituted or unsubstituted hydrocarbon substituent selected from the group consisting of C₁₋₁₅ alkyl, C₂₋₁₅ alkenyl, C₂₋₁₅ alkynyl, phenyl, naphthyl, benzyl, or phenyl (C₁₋₃) alkyl
PROVIDED THAT,
when any azabicyclic ring system is 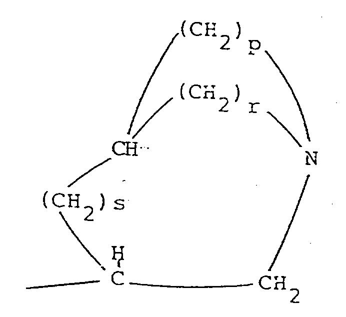
and (i) (p,r,s) is (2,2,0) or (2,2,1), then either the substituent of low lipophilicity is other than H or methyl, or the compound is in the endo configuration; or
(ii) (p,r,s) is (2,1,0), (2,1,1) or (3,1,0), then, except in the cases of 3-[5-(3-methyl-1,2,4-thiadiazol)yl]-1-azabicyclo[2.2.1]heptane; 3-[5-(3-ethyl-1,2,4-thiadiazol)yl]-1-azabicyclo[2.2.1]heptane; 6-[5-(3-methyl-1,2,4-thiadiazol)yl]-1-azabicyclo[3.2.1]octane; either the substituent of low lipophilicity is other than H or C₁₋₂ alkyl, or the compound is in the endo configuration.

2. A compound according to Claim 1 wherein the non-aromatic ring is azacyclic.

3. A compound according to Claim 2, represented by structural formula IA, IB or IC: 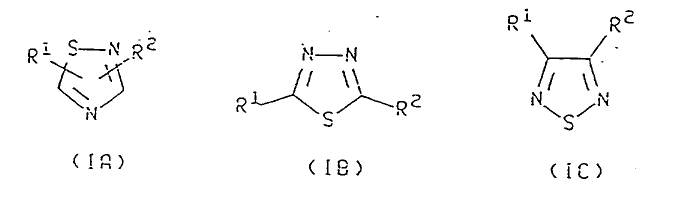 or a salt or prodrug as defined in Claim 1 hereof; wherein
R¹ represents a non-aromatic azacyclic ring system; and R² represents hydrogen, halogen, -CF₃, -OR⁷, -NR⁷R⁸, -NHOR⁷, -NHNH₂, -CN, -CO₂R⁷, -CONR⁷R⁸, or a hydrocarbon substituent; wherein R⁷ and R⁸ independently represent hydrogen or C₁₋₂ alkyl.

4. A compound according to Claim 2, represented by structural formula IA, IB or IC: 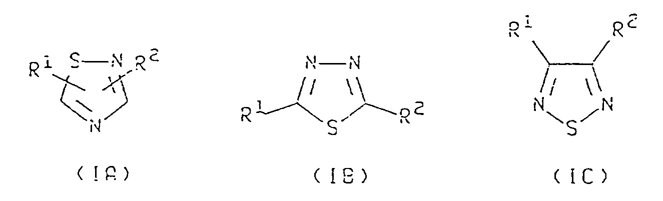 or a salt or prodrug thereof; wherein
R¹ represents a non-aromatic azacyclic ring system; and R² represents hydrogen, halogen, -CF₃, -OR⁷, -NR⁷R⁸, -NHOR⁷, -NHNH₂, -CN, -CO₂R⁷, -CON⁷R⁸, C₂₋ ₅alkenyl, C₂₋₅alkynyl, C₁₋₂alkyl or C₁₋₂alkyl substituted with -OR⁷, -NR⁷R⁸, -SR⁷, -CO₂R⁷, -CONR⁷R⁸ or halogen; wherein R⁷ and R⁸ independently represent hydrogen or C₁₋₂ alkyl.

5. A compound according to claim 4, selected from:
3-[5-(3-methyl-1,2,4-thiadiazol)-yl]pyrrolidine;
1-methyl-3-[5-(3-methyl-1,2,4-thiadiazol)-yl]pyrrolidine;
1-methyl-3-[5-(3-amino-1,2,4-thiadiazol)-yl]pyrrolidine;
1-methyl-3-[5-(3-amino-1,2,4-thiadiazol)-yl]-1,2,5,6-tetrahydropyridine;
3-[5-(3-amino-1,2,4-thiadiazol)-yl]-1,2,5,6-tetrahydropyridine;
3-[5-(3-ethyl-1,2,4-thiadiazol)-yl]-1,2,5,6-tetrahydropyridine;
3-[5-(3-methoxy-1,2,4-thiadiazol)-yl]-1,2,5,6-tetrahydropyridine;
3-[5-(3-ethoxy-1,2,4-thiadiazol)-yl]-1,2,5,6-tetrahydropyridine;
3-[5-(3-chloro-1,2,4-thiadiazol)-yl]-1,2,5,6-tetrahydropyridine;
3-[5-(3-methylamino-1,2,4-thiadiazol)-yl]-1,2,5,6-tetrahydropyridine; and
3-[5-(3-ethylamino-1,2,4-thiadiazol)-yl]-1,2,5,6-tetrahydropyridine;
1-methyl-3-[5-(3-methyl-1,2,4-thiadiazol)-yl]-1,2,5,6-tetrahydropyridine;
3-[5-(3-methyl-1,2,4-thiadiazol)-yl]-1,2,5,6-tetrahydropyridine;
3-[5-(3-dimethylamino-1,2,4-thiadiazol)-yl]-1,2,5,6-tetrahydropyridine;
and salts and prodrugs thereof.

6. A compound according to claim 4, selected from:
3-[5-(3-dimethylamino-1,2,4-thiadiazol)-yl]-1,2,5,6-tetrahydropyridine;
3-[5-(3-n-propyl-1,2,4-thiadiazol)-yl]-1,2,5,6-tetrahydropyridine;
3-[5-(3-cyclopropyl-1,2,4-thiadiazol)-yl]-1,2,5,6-tetrahydropyridine;
3-[5-(3-benzyl-1,2,4-thiadiazol)-yl]-1,2,5,6-tetrahydropyridine;
1-methyl-3-[5-(3-n-propyl-1,2,4-thiadiazol)-yl]-1,2,5,6-tetrahydropyridine;
1-methyl-3-[5-(3-cyclopropyl-1,2,4-thiadiazol)-yl]-1,2,5,6-tetrahydropyridine;
1-methyl-3-[5-(3-benzyl-1,2,4-thiadiazol)-yl]-1,2,5,6-tetrahydropyridine;
1-methyl-3-[5-(3-isopropyl-1,2,4-thiadiazol)-yl]-1,2,5,6-tetrahydropyridine; and
3-[5-(3-isopropyl-1,2,4-thiadiazol)-yl]-1,2,5,6-tetrahydropyridine;
and salts and prodrugs thereof.

7. A compound according to claim 1 wherein the non-aromatic ring is azabicyclic.

8. A compound according to claim 7, represented by structural formula IA, IB or IC: 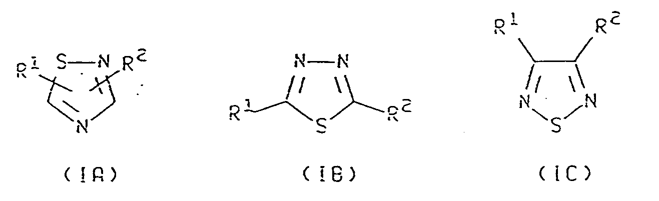 or a salt or prodrug as defined in claim 1 thereof; wherein
R¹ represents a non-aromatic azabicyclic ring system; and R² represents hydrogen, halogen, -CF₃, -OR⁷, -NR⁷R⁸, -NHOR⁷, -NHNH₂, -CN, -CO²R⁷, -CONRR⁷R⁸, or a hydrocarbon substituent; wherein R⁷ and R⁸ independently represent hydrogen or C₁₋₂ alkyl.

9. A compound according to claim 7 or claim 8 wherein the azabicyclic system is selected from: 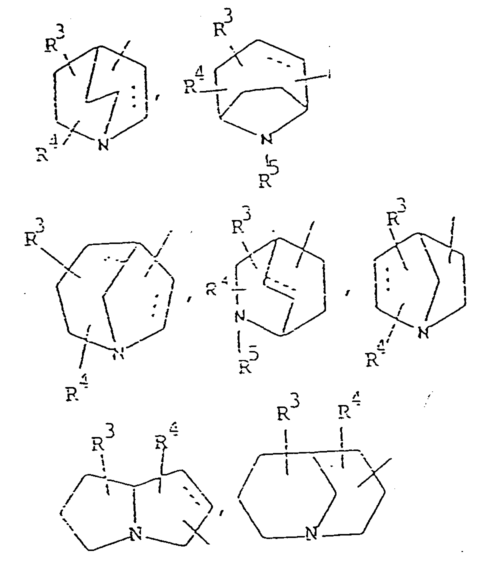 wherein the broken line represents an optional chemical bond;
the substituents R³ and R⁴ independently represent hydrogen, C₁₋₄ alkyl, halo, C₁₋₄ alkoxy, hydroxy or carboxy; or R³ and R⁴ together with the carbon atom to which they are attached represent carbonyl;
the group R⁵ represents hydrogen or C₁₋₄ alkyl; and the nitrogen atom in the azabicyclic ring system carries a lone pair of electrons.

10. A compound according to claim 7 or claim 8, wherein the azabicyclic system is selected from: 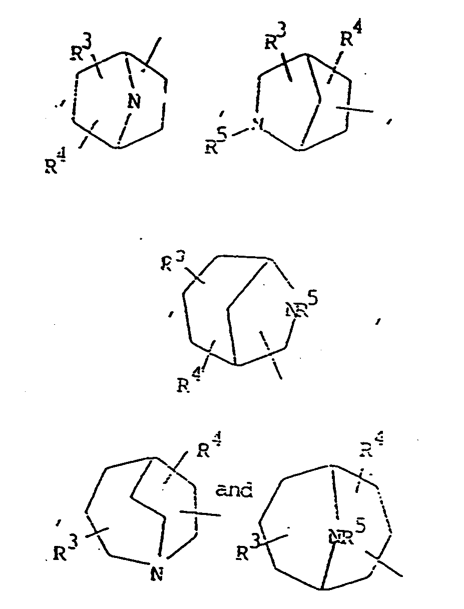 wherein the broken line represents an optional chemical bond;
the substituents R³ and R⁴ independently represent hydrogen, C₁₋₄ alkyl, halo, C₁₋₄ alkoxy, hydroxy or carboxy; or R³ and R⁴ together with the carbon atom to which they are attached represent carbonyl;
the group R⁵ represents hydrogen or C₁₋₄ alkyl; and the nitrogen atom in the azabicyclic ring system carries a lone pair of electrons.

11. A compound according to any of claims 7 to 10, represented by structural formula IA, IB or IC: 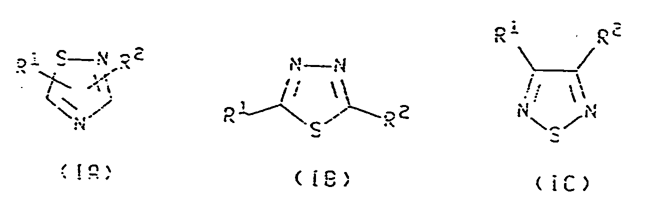 or a salt or prodrug thereof; wherein
R¹ represents a non-aromatic azabicyclic ring system; and R² represents hydrogen, halogen, -CF₃, -OR⁷, -NR⁷R⁸, -NHOR⁷, -NHNH₂, -CN, -CO₂R⁷, -CON⁷R⁸, C₂₋₅alkenyl, C₂₋₅alkynyl, C₁₋₂alkyl or C₁₋₂alkyl substituted with -OR⁷, -NR⁷R⁸, -SR⁷, -CO₂R⁷, -CONR⁷R⁸ or halogen; wherein R⁷ and R⁸ independently represent hydrogen or C₁₋₂ alkyl.

12. A compound according to claim 7, selected from:
3-[5-(3-methyl-1,2,4-thiadiazol)-yl]-1-azabicyclo-[2.2.1]heptane;
3-[5-(3-amino-1,2,4-thiadiazol)-yl]quinuclidine;
6-[5-(3-methyl-1,2,4-thiadiazol)-yl]-1-azabicyclo-[3.2.1]octane;
6-[5-(3-amino-1,2,4-thiadiazol)-yl]-1-azabicyclo-[3.2.1]octane;
3-[5-(3-amino-1,2,4-thiadiazol)-yl]-1-azabicyclo-[2.2.1]heptane;
5-[5-(3-methyl-1,2,4-thiadiazol)-yl]quinuclidin-3-ol;
5-[5-(3-amino-1,2,4-thiadiazol)-yl]quinuclidin-3-ol;
5-methyl-3-[5-(3-methyl-1,2,4-thiadiazol)-yl]-quinuclidine;
5-methyl-3-[5-(3-amino-1,2,4-thiadiazol)-yl]-quinuclidine; and
and salts and prodrugs thereof.

13. A compound according to claim 7, selected from:
3-[5-(3-phenyl-1,2,4-thiadiazol)-yl]quinuclidine;
3-[5-(3-cyclopropyl-1,2,4-thiadiazol)-yl]quinuclidine;
exo- and endo-3-[5-(3-Cyclopropyl-1,2,4-thiadiazol)-yl]1-azabicyclo[2.2.1]heptane;
3-[5-(3-benzyl-1,2,4-thiadiazol)-yl]quinuclidine;
3-[5-(3-tert-butyl-1,2,4-thiadiazol)-yl]quinuclidine;
3-[5-(3-iso-propyl-1,2,4-thiadiazol)-yl]quinuclidine;
3-[5-(3-(1-hydroxy-1-phenylmethyl)-1,2,4-thiadiazol)-yl]quinuclidine;
3-[5-(3-benzoyl-1,2,4-thiadiazol)-yl]quinuclidine;
3-[5-(3-(1,1-diphenyl-1-hydroxymethyl)-1,2,4-thiadiazol)-yl]quinuclidine;
3-[5-(3-(1,1-diphenyl-1-fluoromethyl)-1,2,4-thiadiazol)-yl]quinuclidine;
exo- and endo-3-[5-(3-iso-propyl-1,2,4-thiadiazol)-yl]1-azabicyclo[2.2.1]heptane;
(1R*,6R*) and (1R*,6S*) 6-[5-(3-cyclopropyl-1,2,4-thiadiazol)-yl]-2-azabicyclo[2.2.2]octane;
exo- and endo-3-[5-(3-n-propyl-1,2,4-thiadiazol)-yl]1-azabicyclo[2.2.1]heptane;
3-[5-(3-n-propyl-1,2,4-thiadiazol)-yl]quinuclidine;
(1R*,6R*) and (1R*,6S*) 6-[5-(3-iso-propyl-1,2,4-thiadiazol)-yl]-2-azabicyclo[2.2.2]octane;
exo-3-[5-(3-isopropyl-1,2,4-thiadiazol)-yl]-endo-5-hydroxy-1-azabicyclo[2.2.1]heptane; and
exo- and endo-3-[5-(3-benzyl-1,2,4-thiadiazol)-yl]-1-azabicyclo[2.2.1]heptane;
and salts and prodrugs thereof.

14. A compound according to claim 7, selected from:
exo- and endo-3-[5-(3-ethyl-1,2,4-thiadiazol)-yl]1-azabicyclo[2.2.1]heptane;
and salts and prodrugs thereof.

15. A pharmaceutical composition comprising a compound according to any of claims 1 to 14 in association with a pharmaceutically acceptable carrier.

16. A pharmaceutical composition according to claim 16 further comprising a peripheral cholinergic antagonist.

17. The use of a compound as claimed in any one of claims 1 to 14 for the preparation of a medicament for the treatment of neurological and mental disorders or for the treatment of severe painful conditions.

18. A process for the preparation of a compound as claimed in any one of claims 1 to 14, which process comprises:
(A) cyclising a compound of formula III: 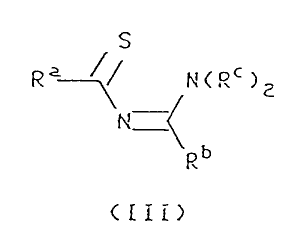 wherein one of R^{a} and R^{b} is a non-aromatic azacyclic or azabicyclic ring system, and the other is a substituent of low lipophilicity or a hydrocarbon substituent as defined in claim 1; and R^{c} is hydrogen or an alkyl group;
(B) cycloaddition of a nitrile sulphide R^{a}-C≡N⁺-S-with a nitrile of formula R^{b}CN where R^{a} and R^{b} are as defined above;
(C) reacting a thiadiazole of formula IV: 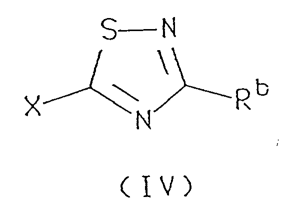 with a reagent which provides an anion ⁻R^{a}, where R^{a} and R^{b} are as defined above and X represents halogen;
(D) reacting a diamine of the type 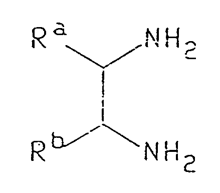 where R^{a} and R^{b} are as defined above, with a sulphur chloride; or
(E) dehydration of a thiosemicarbazide of formula R^{x}CSNHNHCONR^{P}R^{q}, where R^{x} is a non-aromatic azacyclic or azabicyclic ring system and R^{P} and R^{q} independently are hydrogen or an alkyl group.

19. 3-[5-(3-Benzoyl-1,2,4-thiadiazol)-yl]-quinuclidine and salts and prodrugs thereof.

## Claims (Claims for the following Contracting State(s): ES, GR)

1. A process for the preparation of a thiadiazole, or a salt or prodrug, which thiadiazole is substituted on one of the ring carbon atoms thereof with a non-aromatic azacyclic or azabicyclic ring system containing from 4 to 10 ring atoms & one nitrogen atom as the sole heteroatom; and substituted on the other ring atom with a substituent of low lipophilicity, having a rekker f value of not greater than 1.5, or a substituted or unsubstituted hydrocarbon substituent selected from the group consisting of C₁₋₁₅ alkyl, C₂₋₁₅ alkenyl, C₂₋₁₅ alkynyl, phenyl, naphthyl, benzyl, or phenyl (C₁₋₃) alkyl
PROVIDED THAT,
when any azabicyclic ring system is 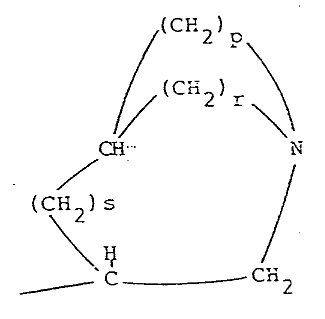
and (i) (p,r,s) is (2,2,0) or (2,2,1), then either the substituent of low lipophilicity is other than H or methyl, or the compound is in the endo configuration; or
(ii) (p,r,s) is (2,1,0), (2,1,1) or (3,1,0), then, except in the cases of 3-[5-(3-methyl-1,2,4-thiadiazol)yl]-1-azabicyclo[2.2.1]heptane; 3-[5-(3-ethyl-1,2,4-thiadiazol)yl]-1-azabicyclo[2.2.1]heptane; 6-[5-(3-methyl-1,2,4-thiadiazol)yl]-1-azabicyclo[3.2.1]octane; either the substituent of low lipophilicity is other than H or C₁₋₂ alkyl, or the compound is in the endo configuration; which process comprises:
(A) cyclising a compound of formula III: 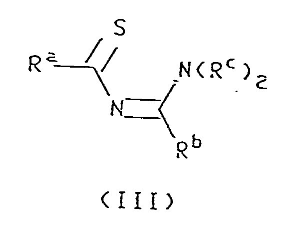 wherein one of R^{a} and R^{b} is a non-aromatic azacyclic or azabicyclic ring system, and the other is a suhstituent of low lipophilicity or a hydrocarbon substituent as defined above; and R^{c} is hydrogen or an alkyl group; or
(B) cycloaddition of a nitrile sulphide R^{a}-C=N⁺-S⁻ with a nitrile of formula R^{b}CN where R^{a} and R^{b} are as defined above; or
(C) reacting a thiadiazole of formula IV: 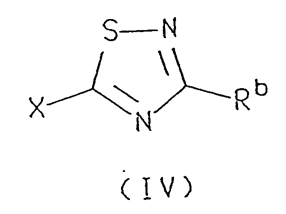 with a reagent which provides an anion ⁻R^{a}, where R^{a} and R^{b} are as defined above and X represents halogen; or
(D) reacting a diamine of the type 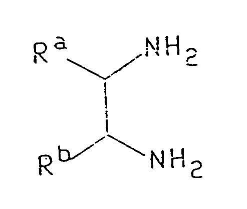 where R^{a} and R^{b} are as defined above, with a sulphur chloride; or
(E) dehydration of a thiosemicarbazide of formula R^{x}CSNHNHCONR^{p}R^{q}, where R^{x} is an azacyclic or azabicyclic ring system as defined above and R^{p} and R^{q} are hydrogen or an alkyl group.

2. A process according to Claim 1 wherein the non-aromatic ring is azacyclic.

3. A process according to Claim 2, wherein the thiadiazole is represented by structural formula IA, IB or IC: 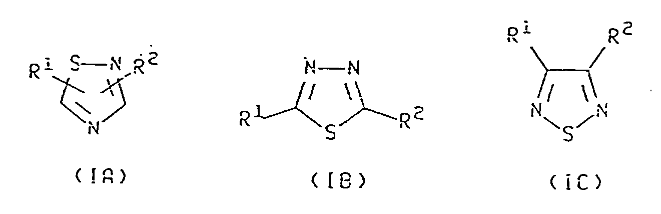 or a salt or prodrug as defined in Claim 1 hereof; wherein
R¹ represents a non-aromatic azacyclic ring system; and R² represents hydrogen, halogen, -CF₃, -OR⁷, -NR⁷R⁸, -NHOR⁷, -NHNH₂, -CN, -CO₂R⁷, -CONR⁷R⁸, or a hydrocarbon substituent; wherein R⁷ and R⁸ independently represent hydrogen or C₁₋₂ alkyl.

4. A process according to Claim 2, wherein the thiadiazole is represented by structural formula IA, IB or IC: 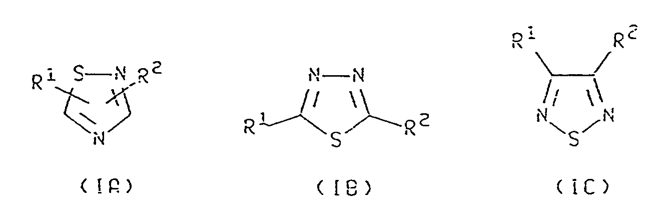 or a salt or prodrug thereof; wherein
R¹ represents a non-aromatic azacyclic ring system; and R² represents hydrogen, halogen, -CF₃, -OR⁷, -NR⁷R⁸, -NHOR⁷, -NHNH₂, -CN, -CO₂R⁷, -CON⁷R⁸, C₂₋ ₅alkenyl, C₂₋₅alkynyl, C₁₋₂alkyl or C₁₋₂alkyl substituted with -OR⁷, -NR⁷R⁸, -SR⁷, -CO₂R⁷, -CONR⁷R⁸ or halogen; wherein R⁷ and R⁸ independently represent hydrogen or C₁₋₂ alkyl.

5. A process according to claim 4, wherein the thiadiazole is selected from:
3-[5-(3-methyl-1,2,4-thiadiazol)-yl]pyrrolidine;
1-methyl-3-[5-(3-methyl-1,2,4-thiadiazol)-yl]pyrrolidine;
1-methyl-3-[5-(3-amino-1,2,4-thiadiazol)-yl]pyrrolidine;
1-methyl-3-[5-(3-amino-1,2,4-thiadiazol)-yl]-1,2,5,6-tetrahydropyridine;
3-[5-(3-amino-1,2,4-thiadiazol)-yl]-1,2,5,6-tetrahydropyridine;
3-[5-(3-ethyl-1,2,4-thiadiazol)-yl]-1,2,5,6-tetrahydropyridine;
3-[5-(3-methoxy-1,2,4-thiadiazol)-yl]-1,2,5,6-tetrahydropyridine;
3-[5-(3-ethoxy-1,2,4-thiadiazol)-yl]-1,2,5,6-tetrahydropyridine;
3-[5-(3-chloro-1,2,4-thiadiazol)-yl]-1,2,5,6-tetrahydropyridine;
3-[5-(3-methylamino-1,2,4-thiadiazol)-yl]-1,2,5,6-tetrahydropyridine; and
3-[5-(3-ethylamino-1,2,4-thiadiazol)-yl]-1,2,5,6-tetrahydropyridine;
1-methyl-3-[5-(3-methyl-1,2,4-thiadiazol)-yl]-1,2,5,6-tetrahydropyridine;
3-[5-(3-methyl-1,2,4-thiadiazol)-yl]-1,2,5,6-tetrahydropyridine;
3-[5-(3-dimethylamino-1,2,4-thiadiazol)-yl]-1,2,5,6-tetrahydropyridine;
and salts and prodrugs thereof.

6. A process according to claim 4, wherein the thiadiazole is selected from:
3-[5-(3-dimethylamino-1,2,4-thiadiazol)-yl]-1,2,5,6-tetrahydropyridine;
3-[5-(3-n-propyl-1,2,4-thiadiazol)-yl]-1,2,5,6-tetrahydropyridine;
3-[5-(3-cyclopropyl-1,2,4-thiadiazol)-yl]-1,2,5,6-tetrahydropyridine;
3-[5-(3-benzyl-1,2,4-thiadiazol)-yl]-1,2,5,6-tetrahydropyridine;
1-methyl-3-[5-(3-n-propyl-1,2,4-thiadiazol)-yl]-1,2,5,6-tetrahydropyridine;
1-methyl-3-[5-(3-cyclopropyl-1,2,4-thiadiazol)-yl]-1,2,5,6-tetrahydropyridine;
1-methyl-3-[5-(3-benzyl-1,2,4-thiadiazol)-yl]-1,2,5,6-tetrahydropyridine;
1-methyl-3-[5-(3-isopropyl-1,2,4-thiadiazol)-yl]-1,2,5,6-tetrahydropyridine; and
3-[5-(3-isopropyl-1,2,4-thiadiazol)-yl]-1,2,5,6-tetrahydropyridine;
and salts and prodrugs thereof.

7. A process according to claim 1 wherein the non-aromatic ring is azabicyclic.

8. A process according to claim 7, wherein the thiadiazole is represented by structural formula IA, IB or IC: 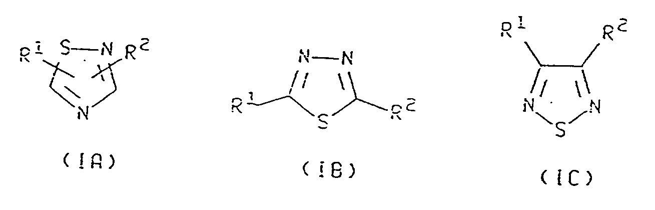 or a salt or prodrug as defined in claim 1 thereof; wherein
R¹ represents a non-aromatic azabicyclic ring system; and R² represents hydrogen, halogen, -CF₃, -OR⁷, -NR⁷R⁸, -NHOR⁷, -NHNH₂, -CN, -CO²R⁷, -CONRR⁷R⁸, or a hydrocarbon substituent; wherein R⁷ and R⁸ independently represent hydrogen or C₁₋₂ alkyl.

9. A process according to claim 7 or claim 8 wherein the azabicyclic system is selected from: 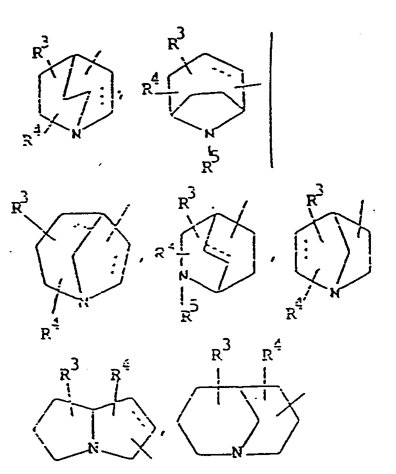 wherein the broken line represents an optional chemical bond;
the substituents R³ and R⁴ independently represent hydrogen, C₁₋₄ alkyl, halo, C₁₋₄ alkoxy, hydroxy or carboxy; or R³ and R⁴ together with the carbon atom to which they are attached represent carbonyl;
the group R⁵ represents hydrogen or C₁₋₄ alkyl; and the nitrogen atom in the azabicyclic ring system carries a lone pair of electrons.

10. A process according to claim 7 or claim 8, wherein the azabicyclic system is selected from: 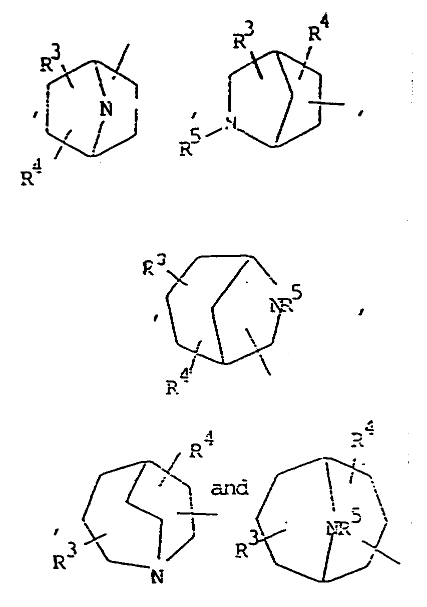 wherein the broken line represents an optional chemical bond;
the substituents R³ and R⁴ independently represent hydrogen, C₁₋₄ alkyl, halo, C₁₋₄ alkoxy, hydroxy or carboxy; or R³ and R⁴ together with the carbon atom to which they are attached represent carbonyl;
the group R⁵ represents hydrogen or C₁₋₄ alkyl; and the nitrogen atom in the azabicyclic ring system carries a lone pair of electrons.

11. A process according to any of claims 7 to 10, wherein the thiadiazole is represented by structural formula IA, IB or IC: 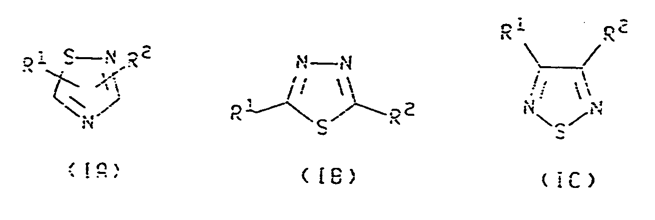 or a salt or prodrug thereof; wherein
R¹ represents a non-aromatic azabicyclic ring system; and R² represents hydrogen, halogen, -CF₃, -OR⁷, -NR⁷R⁸, -NHOR⁷, -NHNH₂, -CN, -CO₂R⁷, -CON⁷R⁸, C₂₋₅alkenyl, C₂₋₅alkynyl, C₁₋₂alkyl or C₁₋₂alkyl substituted with -OR⁷, -NR⁷R⁸, -SR⁷, -CO₂R⁷, -CONR⁷R⁸ or halogen; wherein R⁷ and R⁸ independently represent hydrogen or C₁₋₂ alkyl.

12. A process according to claim 7, wherein the thiadiazole is selected from:
3-[5-(3-methyl-1,2,4-thiadiazol)-yl]-1-azabicyclo-[2.2.1]heptane;
3-[5-(3-amino-1,2,4-thiadiazol)-yl]quinuclidine;
6-[5-(3-methyl-1,2,4-thiadiazol)-yl]-1-azabicyclo-[3.2.1]octane;
6-[5-(3-amino-1,2,4-thiadiazol)-yl]-1-azabicyclo-[3.2.1]octane;
3-[5-(3-amino-1,2,4-thiadiazol)-yl]-1-azabicyclo-[2.2.1]heptane;
5-[5-(3-methyl-1,2,4-thiadiazol)-yl]quinuclidin-3-ol;
5-[5-(3-amino-1,2,4-thiadiazol)-yl]quinuclidin-3-ol;
5-methyl-3-[5-(3-methyl-1,2,4-thiadiazol)-yl]-quinuclidine;
5-methyl-3-[5-(3-amino-1,2,4-thiadiazol)-yl]-quinuclidine; and
and salts and prodrugs thereof.

13. A process according to claim 7, wherein the thiadiazole is selected from:
3-[5-(3-phenyl-1,2,4-thiadiazol)-yl]quinuclidine;
3-[5-(3-cyclopropyl-1,2,4-thiadiazol)-yl]quinuclidine;
exo- and endo-3-[5-(3-Cyclopropyl-1,2,4-thiadiazol)-yl]1-azabicyclo[2.2.1]heptane;
3-[5-(3-benzyl-1,2,4-thiadiazol)-yl]quinuclidine;
3-[5-(3-tert-butyl-1,2,4-thiadiazol)-yl]quinuclidine;
3-[5-(3-iso-propyl-1,2,4-thiadiazol)-yl]quinuclidine;
3-[5-(3-(1-hydroxy-1-phenylmethyl)-1,2,4-thiadiazol)-yl]quinuclidine;
3-[5-(3-benzoyl-1,2,4-thiadiazol)-yl]quinuclidine;
3-[5-(3-(1,1-diphenyl-1-hydroxymethyl)-1,2,4-thiadiazol)-yl]quinuclidine;
3-[5-(3-(1,1-diphenyl-1-fluoromethyl)-1,2,4-thiadiazol)-yl]quinuclidine;
exo- and endo-3-[5-(3-iso-propyl-1,2,4-thiadiazol)-yl]1-azabicyclo[2.2.1]heptane;
(1R*,6R*) and (1R*,6S*) 6-[5-(3-cyclopropyl-1,2,4-thiadiazol)-yl]-2-azabicyclo[2.2.2]octane;
exo- and endo-3-[5-(3-n-propyl-1,2,4-thiadiazol)-yl]1-azabicyclo[2.2.1]heptane;
3-[5-(3-n-propyl-1,2,4-thiadiazol)-yl]quinuclidine;
(1R*,6R*) and (1R*,6S*) 6-[5-(3-iso-propyl-1,2,4-thiadiazol)-yl]-2-azabicyclo[2.2.2]octane;
exo-3-[5-(3-isopropyl-1,2,4-thiadiazol)-yl]-endo-5-hydroxy-1-azabicyclo[2.2.1]heptane; and
exo- and endo-3-[5-(3-benzyl-1,2,4-thiadiazol)-yl]-1-azabicyclo[2.2.1]heptane;
and salts and prodrugs thereof.

14. A process according to claim 7, wherein the thiadiazole is selected from:
exo- and endo-3-[5-(3-ethyl-1,2,4-thiadiazol)-yl]1-azabicyclo[2.2.1]heptane;
and salts and prodrugs thereof.

15. A process for the preparation of a pharmaceutical composition comprising bringing a compound prepared according to any of claims 1 to 14 into association with a pharmaceutically acceptable carrier.

16. A process according to claim 15 further comprising incorporating a peripheral cholinergic antagonist.

17. The use of a compound as claimed in any one of claims 1 to 14 for the preparation of a medicament for the treatment of neurological and mental disorders or for the treatment of severe painful conditions.

18. A process according to claim 1 for the preparation of 3-[5-(3-Benzoyl-1,2,4-thiadiazol)-yl]-quinuclidine.

## Patentansprüche (Patentansprüche für folgende(n) Vertragsstaat(en): AT, BE, CH, DE, FR, GB, IT, LI, LU, NL, SE)

1. Thiadiazol oder ein Salz oder eine Arzneimittelvorstufe, wobei das Thiadiazol an einem seiner Ringkohlenstoffatome substituiert ist mit einem nichtaromatischen azacyclischen oder azabicyclischen Ringsystem, das 4 bis 10 Ringatome plus ein Stickstoffatom als einziges Heteroatom enthält, und an dem anderen Ringatom mit einem Substituenten niedriger Lipophilie, der einen Rekker-f-Wert von nicht mehr als 1,5 aufweist, oder einem substituierten oder unsubstituierten Kohlenwasserstoff-Substituenten, ausgewählt aus der Gruppe, bestehend aus C₁-C₁₅-Alkyl, C₂-C₁₅-Alkenyl, C₂-C₁₅-Alkinyl, Phenyl, Naphthyl, Benzyl oder Phenyl-(C₁₋₃)-alkyl, mit der Maßgabe, daß, wenn ein azabicyclisches Ringsystem die Formel 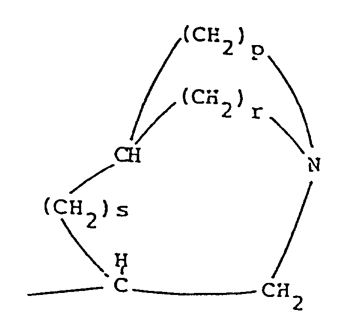
besitzt und (i) (p,r,s) (2,2,0) oder (2,2,1) darstellt, dann entweder der Substituent niedriger Lipophilie etwas anderes als H oder Methyl bedeutet oder die Verbindung in der endo-Konfiguration vorliegt; oder wenn
(ii) (p,r,s) (2,1,0), (2,1,1) oder (3,1,0) bedeutet, dann entweder der Substituent geringer Lipophilie etwas anders als H oder C₁₋₂-Alkyl bedeutet oder die Verbindung in der endo-Konfiguration vorliegt, außer in den Fällen von 3-[5-(3-Methyl-1,2,4-thiadiazol)-yl]-1-azabicyclo[2.2.1]heptan, 3-[5-(3-Ethyl-1,2,4-thiadiazol)-yl]-1-azabicyclo[2.2.1]heptan, 6-[5-(3-Methyl-1,2,4-thiadiazol)-yl]-1-azabicyclo[3.2.1]octan.

2. Verbindung nach Anspruch 1, worin der nichtaromatische Ring azacyclisch ist.

3. Verbindung nach Anspruch 2, dargestellt durch die Strukturformel IA, IB oder IC 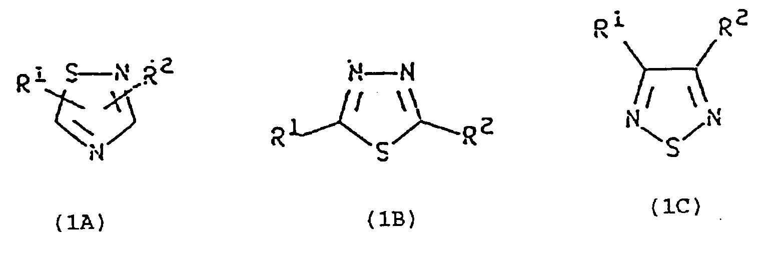 oder ein Salz oder eine Arzneimittelvorstufe davon nach Anspruch 1, worin R¹ für ein nichtaromatisches azacyclisches Ringsystem steht; und R² für Wasserstoff, Halogen, -CF₃, -OR⁷, -NR⁷R⁸, -NHOR⁷, -NHNH₂, -CN, -CO₂R⁷, -CONR⁷R⁸ oder einen Kohlenwasserstoff-Substituenten steht, worin R⁷ und R⁸ unabhängig für Wasserstoff oder C₁₋₂-Alkyl stehen.

4. Verbindung nach Anspruch 2, dargestellt durch die Strukturformel IA, IB oder IC 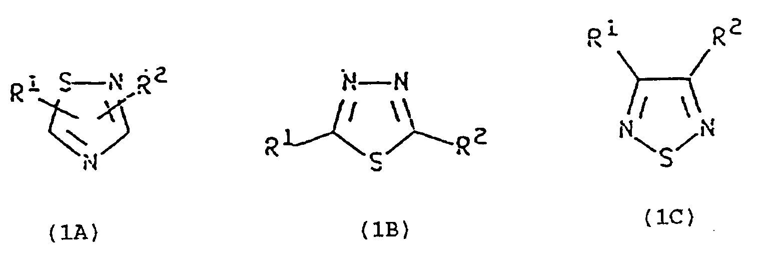 oder ein Salz oder eine Arzneimittelvorstufe davon, worin
R¹ für ein nichtaromatisches azacyclisches Ringsystem steht; und R² für Wasserstoff, Halogen, -CF₃, -OR⁷, -NR⁷R⁸, -NHOR⁷, -NHNH₂, -CN, -CO₂R⁷, -CONR⁷R⁸, C₂₋₅-Alkenyl, C₂₋₅-Alkinyl, C₁₋₂-Alkyl oder C₁₋₂-Alkyl, substituiert mit -OR⁷, -NR⁷R⁸, -SR⁷, -CO₂R⁷, -CONR⁷R⁸ oder Halogen, steht, worin R⁷ und R⁸ unabhängig für Wasserstoff oder C₁₋₂-Alkyl stehen.

5. Verbindung nach Anspruch 4, ausgewählt aus
3-[5-(3-Methyl-1,2,4-thiadiazol)-yl]-pyrrolidin,
1-Methyl-3-[5-(3-methyl-1,2,4-thiadiazol)-yl]-pyrrolidin,
1-Methyl-3-[5-(3-amino-1,2,4-thiadiazol)-yl]-pyrrolidin,
1-Methyl-3-[5-(3-amino-1,2,4-thiadiazol)-yl]-1,2,5,6-tetrahydropyridin,
3-[5-(3-Amino-1,2,4-thiadiazol)-yl]-1,2,5,6-tetrahydropyridin,
3-[5-(3-Ethyl-1,2,4-thiadiazol)-yl]-1,2,5,6-tetrahydropyridin,
3-[5-(3-Methoxy-1,2,4-thiadiazol)-yl]-1,2,5,6-tetrahydropyridin,
3-[5-(3-Ethoxy-1,2,4-thiadiazol)-yl]-1,2,5,6-tetrahydropyridin,
3-[5-(3-Chlor-1,2,4-thiadiazol)-yl]-1,2,5,6-tetrahydropyridin,
3-[5-(3-Methylamino-1,2,4-thiadiazol)-yl]-1,2,5,6-tetrahydropyridin,
3-[5-(3-Ethylamino-1,2,4-thiadiazol)-yl]-1,2,5,6-tetrahydropyridin,
1-Methyl-3-[5-(3-methyl-1,2,4-thiadiazol)-yl]-1,2,5,6-tetrahydropyridin;
3-[5-(3-Methyl-1,2,4-thiadiazol)-yl]-1,2,5,6-tetrahydropyridin,
3-[5-(3-Dimethylamino-1,2,4-thiadiazol)-yl]-1,2,5,6-tetrahydropyridin,
und die Salze und Arzneimittelvorstufen davon.

6. Verbindung nach Anspruch 4, ausgewählt aus
3-[5-(3-Dimethylamino-1,2,4-thiadiazol)-yl]-1,2,5,6-tetrahydropyridin,
3-[5-(3-n-Propyl-1,2,4-thiadiazol)-yl]-1,2,5,6-tetrahydropyridin,
3-[5-(3-Cyclopropyl-1,2,4-thiadiazol)-yl]-1,2,5,6-tetrahydropyridin,
3-[5-(3-Benzyl-1,2,4-thiadiazol)-yl]-1,2,5,6-teträhydropyridin,
1-Methyl-3-[5-(3-n-propyl-1,2,4-thiadiazol)-yl]-1,2,5,6-tetrahydropyridin,
1-Methyl-3-[5-(3-cyclopropyl-1,2,4-thiadiazol)-yl]-1,2,5,6-tetrahydropyridin,
1-Methyl-3-[5-(3-benzyl-1,2,4-thiadiazol)-yl]-1,2,5,6-tetrahydropyridin,
1-Methyl-3-[5-(3-Isopropyl-1,2,4-thiadiazol)-yl]-1,2,5,6-tetrahydropyridin, und
3-[5-(3-Isopropyl-1,2,4-thiadiazol)-yl]-1,2,5,6-tetrahydropyridin,
und die Salze und Arzneimittelvorstufen davon.

7. Verbindung nach Anspruch 1, worin der nichtaromatische Ring azabicyclisch ist.

8. Verbindung nach Anspruch 7, dargestellt durch die Strukturformel IA, IB oder IC 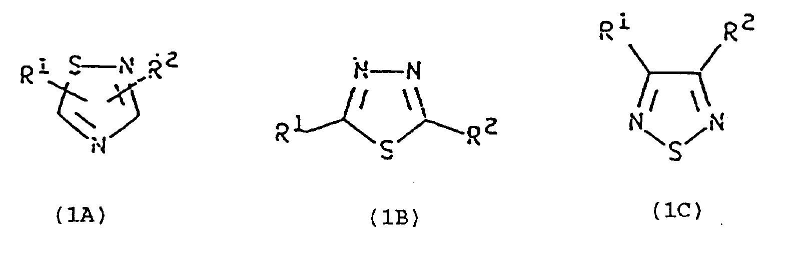 oder ein Salz oder eine Arzneimittelvorstufe nach Anspruch 1 davon, worin R¹ für ein nichtaromatisches azabicyclisches Ringsystem steht und R² für Wasserstoff, Halogen, -CF₃, -OR⁷, -NR⁷R⁸, -NHOR⁷, -NHNH₂, -CN, -CO₂R⁷, -CONR⁷R⁸, oder einen Kohlenwasserstoff-Substituenten steht, worin R⁷ und R⁸ unabhängig Wasserstoff oder C₁₋₂-Alkyl bedeuten.

9. Verbindung nach Anspruch 7 oder Anspruch 8, worin das azabicyclische Ringsystem ausgewählt ist aus 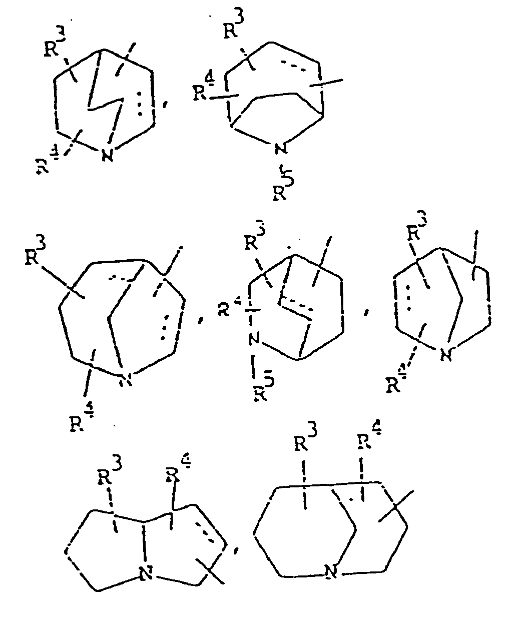 worin die gestrichelte Linie eine frei wählbare chemische Bindung darstellt, die Substituenten R³ und R⁴ unabhängig für Wasserstoff, C₁₋₄-Alkyl, Halogen, C₁₋₄-Alkoxy, Hydroxy oder Carboxy stehen, oder R³ und R⁴ zusammen mit dem Kohlenstoffatom, an das sie angeknüpft sind, Carbonyl darstellen, die Gruppe R⁵ für Wasserstoff oder C₁₋₄-Alkyl steht, und das Stickstoffatom in dem azabicyclischen Ringsystem ein einsames Elektronenpaar aufweist.

10. Verbindung nach Anspruch 7 oder Anspruch 8, worin das azabicyclische Ringsystem ausgewählt ist aus 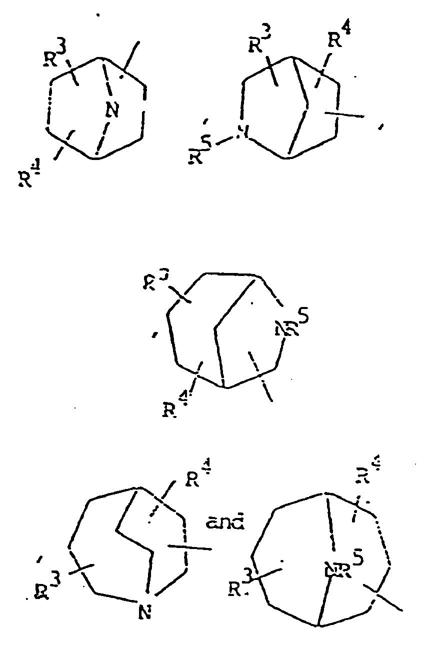 worin die gestrichelte Linie eine frei wählbare Doppelbindung darstellt, die Substituenten R³ und R⁴ unabhängig für Wasserstoff, C₁₋₄-Alkyl, Halogen, C₁₋₄-Alkoxy, Hydroxy oder Carboxy stehen oder R³ und R⁴ zusammen mit dem Kohlenstoffatom, an das sie angeknüpft sind, Carbonyl darstellen, die Gruppe R⁵ für Wasserstoff oder C₁₋₄-Alkyl steht und das Stickstoffatom in dem azabicyclischen Ringsystem ein einsames Elektronenpaar aufweist.

11. Verbindung nach einem der Ansprüche 7 bis 10, dargestellt durch die Strukturformel IA, IB oder IC 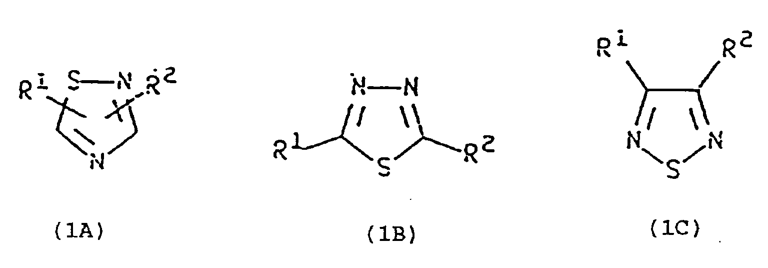 oder ein Salz oder eine Arzneimittelvorstufe davon, worin
R¹ für ein nichtaromatisches azabicyclisches Ringsystem steht; und R² für Wasserstoff, Halogen, -CF₃, -OR⁷, -NR⁷R⁸, -NHOR⁷, -NHNH₂, -CN, -CO₂R⁷, -CONR⁷R⁸, C₂₋₅-Alkenyl, C₂₋₅-Alkinyl, C₁₋₂-Alkyl oder C₁₋₂-Alkyl, substituiert mit -OR⁷, -NR⁷R⁸, -SR⁷, -CO₂R⁷, -CONR⁷R⁸ oder Halogen, steht, worin R⁷ und R⁸ unabhängig für Wasserstoff oder C₁₋₂-Alkyl stehen.

12. Verbindung nach Anspruch 7, ausgewählt aus
3-[5-(3-Methyl-1,2,4-thiadiazol)-yl]-1-azabicyclo-[2.2.1]heptan,
3-[5-(3-Amino-1,2,4-thiadiazol)-yl]-quinuclidin;
6-[5-(3-Methyl-1,2,4-thiadiazol)-yl]-1-azabicyclo[3.2.1]octan,
6-[5-(3-Amino-1,2,4-thiadiazol)-yl]-1-azabicyclo[3.2.1]-octan,
3-[5-(3-Amino-1,2,4-thiadiazol)-yl]-1-azabicyclo[2.2.1]-heptan,
5-[5-(3-Methyl-1,2,4-thiadiazol)-yl]-quinuclidin-3-ol,
5-[5-(3-Amino-1,2,4-thiadiazol)-yl)-quinuclidin-3-ol,
5-Methyl-3-[5-(3-methyl-1,2,4-thiadiazol)-yl]-quinuclidin,
5-Methyl-3-[5-(3-Amino-1,2,4-thiadiazol)-yl]-quinuclidin, und
die Salze und Arzneimittelvorstufen davon.

13. Verbindung nach Anspruch 7, ausgewählt aus
3-[5-(3-Phenyl-1,2,4-thiadiazol)-yl]-quinuclidin;
3-[5-(3-Cyclopropyl-1,2,4-thiadiazol)-yl]-quinuclidin;
exo- und endo-3-[5-(3-Cyclopropyl-1,2,4-thiadiazol)-yl]-1-azabicyclo[2.2.1]-heptan,
3-[5-(3-Benzyl-1,2,4-thiadiazol)-yl]-quinuclidin,
3-[5-(3-tert-Butyl-1,2,4-thiadiazol)-yl]-quinuclidin,
3-[5-(3-Isopropyl-1,2,4-thiadiazol)-yl]-quinuclidin,
3-[5-(3-(1-Hydroxy-1-phenylmethyl)-1,2,4-thiadiazol)-yl]-quinuclidin,
3-[5-(3-Benzoyl-1,2,4-thiadiazol)-yl]-quinuclidin,
3-[5-(3-(1,1-Diphenyl-1-hydroxymethyl)-1,2,4-thiadiazol)-yl]-quinuclidin,
3-[5-(3-(1,1-Diphenyl-1-fluormethyl)-1,2,4-thiadiazol)-yl]-quinuclidin,
exo- und endo-3-[5-(3-Isopropyl-1,2,4-thiadiazol)-yl]-1-azabicyclo[2.2.1]heptan,
(1R*,6R*)- und (1R*,6S*)-6-[5-(3-Cyclopropyl-1,2,4-thiadiazol)-yl]-2-azabicyclo[2.2.2]octan,
exo- und endo-3-[5-(3-n-Propyl-1,2,4-thiadiazol)-yl]-1-azabicyclo[2.2.1]heptan,
3-[5-(3-n-Propyl-1,2,4-thiadiazol)-yl]-quinuclidin;
(1R*,6R*)- und (1R*,6S*)-6-[5-(3-Isopropyl-1,2,4-thiadiazol)-yl]-2-azabicyclo[2.2.2]octan,
exo-3-[5-(3-Isopropyl-1,2,4-thiadiazol)-yl]-endo-5-hydroxy-1-azabicyclo-[2.2.1]heptan und
exo- und endo-3-[5-(3-Benzyl-1,2,4-thiadiazol)-yl]-1-azabicyclo[2.2.1]heptan, und die Salze und Arzneimittelvorstufen davon.

14. Verbindung nach Anspruch 7, ausgewählt aus
exo- und endo-3-[5-(3-Ethyl-1,2,4-thiadiazol)-yl]-1-azabicyclo[2.2.1]heptan, und die Salze und Arzneimittelvorstufen davon.

15. Pharmazeutisches Präparat, umfassend eine Verbindung nach einem der Ansprüche 1 bis 14 in Verbindung mit einem pharmazeutisch verträglichen Trägerstoff.

16. Pharmazeutisches Präparat nach Anspruch 15, das außerdem einen peripheren cholinergen Antagonisten umfaßt.

17. Verwendung einer Verbindung nach einem der Ansprüche 1 bis 14 zur Herstellung eines Medikaments zur Behandlung neurologischer und mentaler Störungen oder zur Behandlung schwerer schmerzhafter Zustände.

18. Verfahren zur Herstellung einer Verbindung nach einem der Ansprüche 1 bis 14, wobei das Verfahren umfaßt:
(A) Cyclisierung einer Verbindung der Formel III 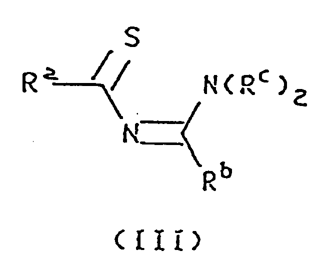 worin eines von R^{a} und R^{b} ein nichtaromatisches azacyclisches oder azabicyclisches Ringsystem und das andere einen Substituenten niedriger Lipophilie oder einen Kohlenwasserstoff-Substituenten nach Anspruch 1 darstellt, und R^{c} für Wasserstoff oder eine Alkylgruppe steht;
(B) Cycloaddition eines Nitrilsulphids R^{a}-C≡N⁺-S- mit einem Nitril der Formel R^{b}CN, worin R^{a} und R^{b} die oben gegebenen Definitionen besitzen;
(C) Umsetzung eines Thiadiazols der Formel IV 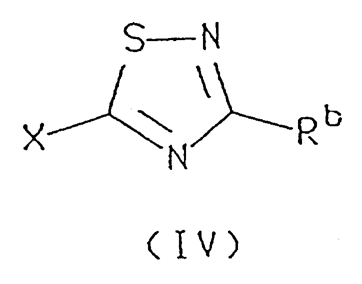 mit einem Reagens, welches ein Anion ⁻R^{a} bereitstellt, worin R^{a} und R^{b} die oben gegebenen Definitionen besitzen und X für Halogen steht;
(D) Umsetzung eines Diamins des Typs 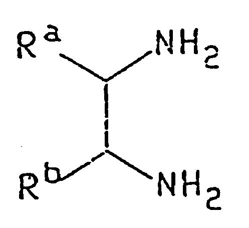 worin R^{a} und R^{b} die oben gegebenen Definitionen besitzen, mit einem Schwefelchlorid; oder
(E) Dehydrierung eines Thiosemicarbazids der Formel R^{x}CSNHNHCONR^{P}R^{q}, worin R^{x} für ein nichtaromatisches azacyclisches oder azabicyclisches Ringsystem und R^{P} und R^{q} unabhängig für Wasserstoff oder eine Alkylgruppe stehen.

19. 3-[5-(3-Benzoyl-1,2,4-thiadiazol)-yl]-quinuclidin und die Salze und Arzneimittelvorstufen davon.

## Patentansprüche (Patentansprüche für folgende(n) Vertragsstaat(en): ES, GR)

1. Verfahren zur Herstellung eines Thiadiazols oder eines Salzes oder einer Arzneimittelvorstufe, wobei das Thiadiazol an einem seiner Ringkohlenstoffatome substituiert ist mit einem nichtaromatischen azacyclischen oder azabicyclischen Ringsystem, das 4 bis 10 Ringatome plus ein Stickstoffatom als einziges Heteroatom enthält, und am anderen Ringatom mit einem Substituenten niedriger Lipophilie, der einen Rekker-f-Wert von nicht mehr als 1,5 aufweist, oder einem substituierten oder unsubstituierten Kohlenwasserstoff-Substituenten, ausgewählt aus der Gruppe, bestehend aus C₁-C₁₅-Alkyl, C₂-C₁₅-Alkenyl, C₂-C₁₅-Alkinyl, Phenyl, Naphthyl, Benzyl oder Phenyl-(C₁₋₃)-alkyl, mit der Maßgabe, daß, wenn ein azabicyclisches Ringsystem die Formel 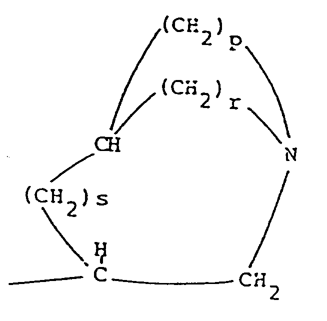
besitzt und (i) (p,r,s) (2,2,0) oder (2,2,1) darstellt, dann entweder der Substituent niedriger Lipophilie etwas anderes als H oder Methyl bedeutet oder die Verbindung in der endo-Konfiguration vorliegt; oder wenn
(ii) (p,r,s) (2,1,0), (2,1,1) oder (3,1,0) bedeutet, dann entweder der Substituent geringer Lipophilie etwas anders als H oder C₁₋₂-Alkyl bedeutet oder die Verbindung in der endo-Konfiguration vorliegt, wobei das Verfahren umfaßt:
(A) Cyclisierung einer Verbindung der Formel III: 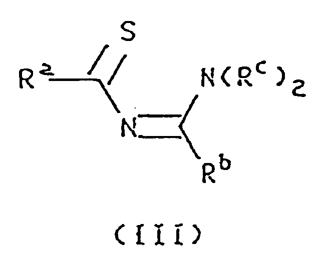 worin eines von R^{a} und R^{b} ein nichtaromatisches azacyclisches oder azabicyclisches Ringsystem darstellt und das andere einen Substituenten niedriger Lipophilie oder einen Kohlenwasserstoff-Substituenten nach Anspruch 1 darstellt und R^{c} für Wasserstoff oder eine Alkylgruppe steht;
(B) Cycloaddition eines Nitrilsulphids R^{a}-C≡N⁺-S- mit einem Nitril der Formel R^{b}CN, worin R^{a} und R^{b} die oben gegebenen Definitionen besitzen;
(C) Umsetzung eines Thiadiazols der Formel IV 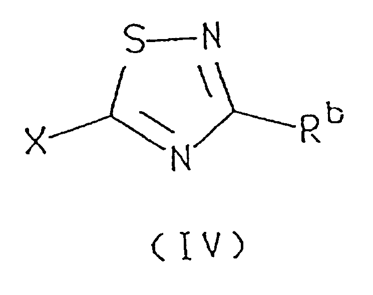 mit einem Reagens, welches ein Anion ⁻R^{a} bereitstellt, worin R^{a} und R^{b} die oben gegebenen Definitionen besitzen und X für Halogen steht;
(D) Umsetzung eines Diamins des Typs 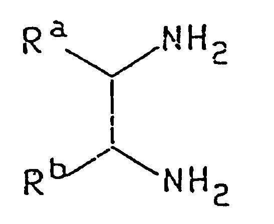 worin R^{a} und R^{b} die oben gegebenen Definitionen besitzen, mit einem Schwefelchlorid; oder
(E) Dehydrierung eines Thiosemicarbazids der Formel R^{x}CSNHNHCONR^{P}R^{q}, worin R^{x} für ein nichtaromatisches azacyclisches oder azabicyclisches Ringsystem steht, und R^{P} und R^{q} unabhängig für Wasserstoff oder eine Alkylgruppe stehen.

2. Verfahren nach Anspruch 1, worin der nichtaromatische Ring azacyclisch ist.

3. Verfahren nach Anspruch 2, dargestellt durch die Strukturformel IA, IB oder IC 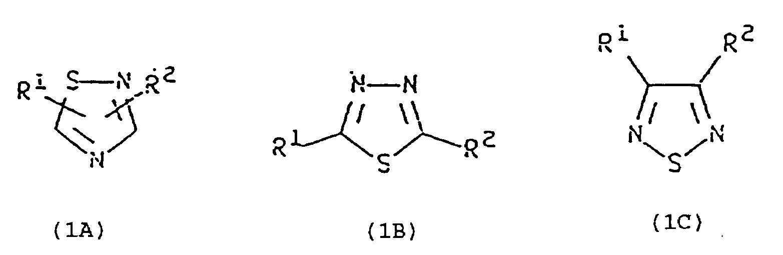 oder ein Salz oder eine Arzneimittelvorstufe davon nach Anspruch 1, worin
R¹ für ein nichtaromatisches azacyclisches Ringsystem steht und R² für Wasserstoff, Halogen, -CF₃, -OR⁷, -NR⁷R⁸, -NHOR⁷, -NHNH₂, -CN, -CO₂R⁷, -CONR⁷R⁸ oder einen Kohlenwasserstoff-Substituenten worin R⁷ und R⁸ unabhängig für Wasserstoff oder C₁₋₂-Alkyl stehen.

4. Verfahren nach Anspruch 2, bei dem das Thiadiazol durch die Strukturformel IA, IB oder IC dargestellt wird 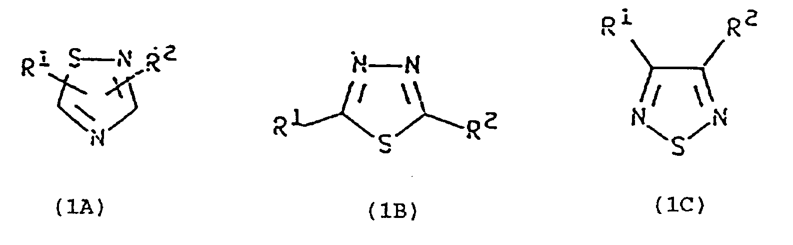 oder ein Salz oder eine Arzneimittelvorstufe davon, worin
R¹ für ein nichtaromatisches azacyclisches Ringsystem steht und R² für Wasserstoff, Halogen, -CF₃, -OR⁷, -NR⁷R⁸, -NHOR⁷, -NHNH₂, -CN, -CO₂R⁷, -CONR⁷R⁸, C₂₋₅-Alkenyl, C₂₋₅-Alkinyl, C₁₋₂-Alkyl oder C₁₋₂-Alkyl, substituiert mit -OR⁷, -NR⁷R⁸, -SR⁷, -CO₂R⁷, -CONR⁷R⁸ oder Halogen steht, worin R⁷ und R⁸ unabhängig für Wasserstoff oder C₁₋₂-Alkyl stehen.

5. Verfahren nach Anspruch 4, bei dem das Thiadiazol ausgewählt wird aus
3-[5-(3-Methyl-1,2,4-thiadiazol)-yl]-pyrrolidin,
1-Methyl-3-[5-(3-methyl-1,2,4-thiadiazol)-yl]-pyrrolidin,
1-Methyl-3-[5-(3-amino-1,2,4-thiadiazol)-yl]pyrrolidin,
1-Methyl-3-[5-(3-amino-1,2,4-thiadiazol)-yl]-1,2,5,6-tetrahydropyridin,
3-[5-(3-Amino-1,2,4-thiadiazol)-yl]-1,2,5,6-tetrahydropyridin,
3-[5-(3-Ethyl-1,2,4-thiadiazol)-yl]-1,2,5,6-tetrahydropyridin,
3-[5-(3-Methoxy-1,2,4-thiadiazol)-yl]-1,2,5,6-tetrahydropyridin,
3-[5-(3-Ethoxy-1,2,4-thiadiazol)-yl]-1,2,5,6-tetrahydropyridin,
3-[5-(3-Chlor-1,2,4-thiadiazol)-yl]-1,2,5,6-tetrahydropyridin,
3-[5-(3-Methylamino-1,2,4-thiadiazol)-yl]-1,2,5,6-tetrahydropyridin,
3-[5-(3-Ethylamino-1,2,4-thiadiazol)-yl]-1,2,5,6-tetrahydropyridin,
1-Methyl-3-[5-(3-methyl-1,2,4-thiadiazol)-yl]-1,2,5,6-tetrahydropyridin;
3-[5-(3-Methyl-1,2,4-thiadiazol)-yl]-1,2,5,6-tetrahydropyridin,
3-[5-(3-Dimethylamino-1,2,4-thiadiazol)-yl]-1,2,5,6-tetrahydropyridin, und aus
den Salzen und Arzneimittelvorstufen davon.

6. Verfahren nach Anspruch 4, bei dem das Thiadiazol ausgewählt wird aus
3-[5-(3-Dimethylamino-1,2,4-thiadiazol)-yl]-1,2,5,6-tetrahydropyridin,
3-[5-(3-n-Propyl-1,2,4-thiadiazol)-yl]-1,2,5,6-tetrahydropyridin,
3-[5-(3-Cyclopropyl-1,2,4-thiadiazol)-yl]-1,2,5,6-tetrahydropyridin,
3-[5-(3-Benzyl-1,2,4-thiadiazol)-yl]-1,2,5,6-tetrahydropyridin,
1-Methyl-3-[5-(3-n-propyl-1,2,4-thiadiazol)-yl]-1,2,5,6-tetrahydropyridin,
1-Methyl-3-[5-(3-cyclopropyl-1,2,4-thiadiazol)-yl]-1,2,5,6-tetrahydropyridin,
1-Methyl-3-[5-(3-benzyl-1,2,4-thiadiazol)-yl]-1,2,5,6-tetrahydropyridin,
1-Methyl-3-[5-(3-Isopropyl-1,2,4-thiadiazol)-yl]-1,2,5,6-tetrahydropyridin, und
3-[5-(3-Isopropyl-1,2,4-thiadiazol)-yl]-1,2,5,6-tetrahydropyridin, und aus den
Salzen und Arzneimittelvorstufen davon.

7. Verfahren nach Anspruch 1, bei dem der nichtaromatische Ring azabicyclisch ist.

8. Verfahren nach Anspruch 7, bei dem das Thiadiazol dargestellt wird durch die Strukturformel IA, IB oder IC 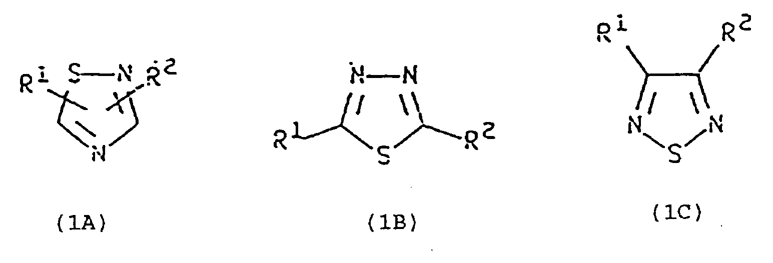 oder durch ein Salz oder eine Arzneimittelvorstufe nach Anspruch 1 davon worin R¹ für ein nichtaromatisches azabicyclisches Ringsystem steht und R² für Wasserstoff, Halogen, -CF₃, -OR⁷, -NR⁷R⁸, -NHOR⁷, -NHNH₂, -CN, -CO₂R⁷, -CONR⁷R⁸ oder einen Kohlenwasserstoff-Substituenten worin R⁷ und R⁸ unabhängig Wasserstoff oder C₁₋₂-Alkyl bedeuten.

9. Verfahren nach Anspruch 7 oder Anspruch 8, bei dem das azabicyclische Ringsystem ausgewählt wird aus 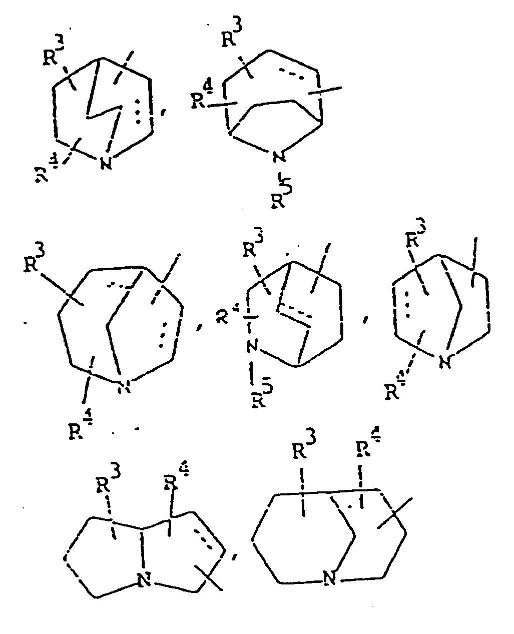 worin die gestrichelte Linie eine frei wählbare chemische Bindung darstellt, die Substituenten R³ und R⁴ unabhängig für Wasserstoff, C₁₋₄-Alkyl, Halogen, C₁₋₄-Alkoxy, Hydroxy oder Carboxy stehen oder R³ und R⁴ zusammen mit dem Kohlenstoffatom, an das sie angeknüpft sind, Carbonyl darstellen, die Gruppe R⁵ für Wasserstoff oder C₁₋₄-Alkyl steht, und das Stickstoffatom in dem azabicyclischen Ringsystem ein einsames Elektronenpaar aufweist.

10. Verfahren nach Anspruch 7 oder Anspruch 8, bei dem das azabicyclische Ringsystem ausgewählt wird aus 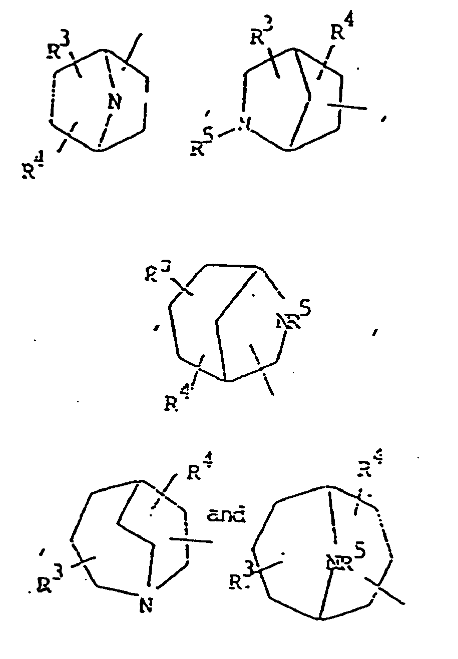 worin die gestrichelte Linie eine frei wählbare Doppelbindung darstellt, die Substituenten R³ und R⁴ unabhängig für Wasserstoff, C₁₋₄-Alkyl, Halogen, C₁₋₄-Alkoxy, Hydroxy oder Carboxy stehen oder R³ und R⁴ zusammen mit dem Kohlenstoffatom, an das sie angeknüpft sind, Carbonyl darstellen, die Gruppe R⁵ für Wasserstoff oder C₁₋₄-Alkyl steht und das Stickstoffatom in dem azabicyclischen Ringsystem ein einsames Elektronenpaar aufweist.

11. Verfahren nach einem der Ansprüche 7 bis 10, bei dem das Thiadiazol dargestellt wird durch die Strukturformel IA, IB oder IC 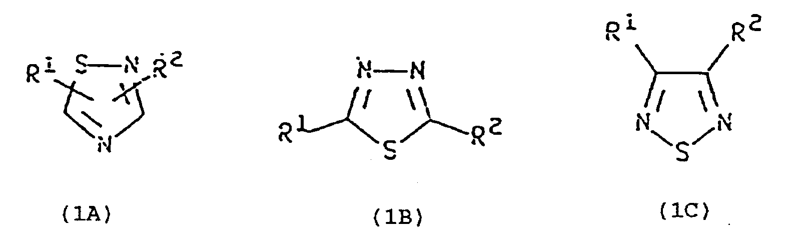 oder durch ein Salz oder eine Arzneimittelvorstufe davon, worin
R¹ für ein nichtaromatisches azabicyclisches Ringsystem steht; und R² für Wasserstoff, Halogen, -CF₃, -OR⁷, -NR⁷R⁸, -NHOR⁷, -NHNH₂, -CN, -CO₂R⁷, -CONR⁷R⁸, C₂₋₅-Alkenyl, C₂₋₅-Alkinyl, C₁₋₂-Alkyl oder C₁₋₂-Alkyl, substituiert mit -OR⁷, -NR⁷R⁸, -SR⁷, -CO₂R⁷, -CONR⁷R⁸ oder Halogen, steht, worin R⁷ und R⁸ unabhängig für Wasserstoff oder C₁₋₂-Alkyl stehen.

12. Verfahren nach Anspruch 7, bei dem das Thiadiazol ausgewählt wird aus
3-[5-(3-Methyl-1,2,4-thiadiazol)-yl]-1-azabicyclo-[2.2.1]heptan,
3-[5-(3-Amino-1,2,4-thiadiazol)-yl]-quinuclidin;
6-[5-(3-Methyl-1,2,4-thiadiazol)-yl]-1-azabicyclo-[3.2.1]octan,
6-[5-(3-Amino-1,2,4-thiadiazol)-yl]-1-azabicyclo[3.2.1]-octan,
3-[5-(3-Amino-1,2,4-thiadiazol)-yl]-1-azabicyclo[2.2.1]-heptan,
5-[5-(3-Methyl-1,2,4-thiadiazol)-yl]-quinuclidin-3-ol,
5-[5-(3-Amino-1,2,4-thiadiazol)-yl]-quinuclidin-3-ol,
5-Methyl-3-[5-(3-methyl-1,2,4-thiadiazol)-yl]-quinuclidin,
5-Methyl-3-[5-(3-Amino-1,2,4-thiadiazol)-yl]-quinuclidin und aus den Salzen und
Arzneimittelvorstufen davon.

13. Verfahren nach Anspruch 7, bei dem das Thiadiazol ausgewählt wird aus
3-[5-(3-Phenyl-1,2,4-thiadiazol)-yl]-quinuclidin;
3-[5-(3-Cyclopropyl-1,2,4-thiadiazol)-yl]-quinuclidin;
exo- und endo-3-[5-(3-Cyclopropyl-1,2,4-thiadiazol)-yl]-1-azabicyclo[2.2.1]-heptan,
3-[5-(3-Benzyl-1,2,4-thiadiazol)-yl]-quinuclidin,
3-[5-(3-tert-Butyl-1,2,4-thiadiazol)-yl]-quinuclidin,
3-[5-(3-Isopropyl-1,2,4-thiadiazol)-yl]-quinuclidin,
3-[5-(3-(1-Hydroxy-1-phenylmethyl)-1,2,4-thiadiazol)-yl]-quinuclidin,
3-[5-(3-Benzoyl-1,2,4-thiadiazol)-yl]-quinuclidin,
3-[5-(3-(1,1-Diphenyl-1-hydroxymethyl)-1,2,4-thiadiazol)-yl]-quinuclidin,
3-[5-(3-(1,1-Diphenyl-1-fluormethyl)-1,2,4-thiadiazol)-yl]-quinuclidin,
exo- und endo-3-[5-(3-Isopropyl-1,2,4-thiadiazol)-yl]-1-azabicyclo[2.2.1]heptan,
(1R*,6R*)- und (1R*,6S*)-6-[5-(3-Cyclopropyl-1,2,4-thiadiazol)-yl]-2-azabicyclo[2.2.2]octan,
exo- und endo-3-[5-(3-n-Propyl-1,2,4-thiadiazol)-yl]-1-azabicyclo[2.2.1]heptan,
3-[5-(3-n-Propyl-1,2,4-thiadiazol)-yl]-quinuclidin;
(1R*,6R*)- und (1R*,6S*)-6-[5-(3-Isopropyl-1,2,4-thiadiazol)-yl]-2-azabicyclo[2.2.2]octan,
exo-3-[5-(3-Isopropyl-1,2,4-thiadiazol)-yl]-endo-5-hydroxy-1-azabicyclo-[2.2.1]heptan und
exo- und endo-3-[5-(3-Benzyl-1,2,4-thiadiazol)-yl]-1-azabicyclo[2.2.1]heptan und
aus den Salzen und Arzneimittelvorstufen davon.

14. Verfahren nach Anspruch 7, bei dem das Thiadiazol ausgewählt wird aus exo- und endo-3-[5-(3-Ethyl-1,2,4-thiadiazol)-yl]-1-azabicyclo[2.2.1]heptan und aus den Salzen und Arzneimittelvorstufen davon.

15. Verfahren zur Herstellung eines pharmazeutischen Präparats, umfassend eine Verbindung nach einem der Ansprüche 1 bis 14 in Verbindung mit einem pharmazeutisch verträglichen Trägerstoff.

16. Verfahren nach Anspruch 15, das außerdem die Einarbeitung eines peripheren cholinergen Antagonisten umfaßt.

17. Verwendung einer Verbindung nach einem der Ansprüche 1 bis 14 zur Herstellung eines Medikaments zur Behandlung neurologischer und mentaler Störungen oder zur Behandlung schwerer schmerzhafter Zustände.

18. Verfahren zur Herstellung von 3-[5-(3-Benzoyl-1,2,4-thiadiazol)-yl]-quinuclidin.

## Revendications (Revendications pour l'(les) Etat(s) contractant(s) suivant(s): AT, BE, CH, DE, FR, GB, IT, LI, LU, NL, SE)

1. Thiadiazole ou un de ses sels ou promédicaments, qui est substitué sur un de ses atomes de carbone cyclique par un système cyclique azacyclique ou azabicyclique non aromatique, contenant de 4 à 10 atomes de carbone, plus un atome d'azote à titre de seul hétéroatome ; et qui est substitué sur l'autre atome cyclique par un substituant de faible lipophilie, possédant une valeur f de rekker non supérieure à 1,5, ou un substituant hydrocarboné substitué ou non substitué choisi dans le groupe comprenant les groupes alkyle en C₁₋₁₅, alcényle en C₂₋₁₅, alcynyle en C₂₋₁₅, phényle, naphtyle, benzyle ou phényl(alkyle en C₁₋₃), étant entendu que,
lorsqu'un système cyclique azabicylique est 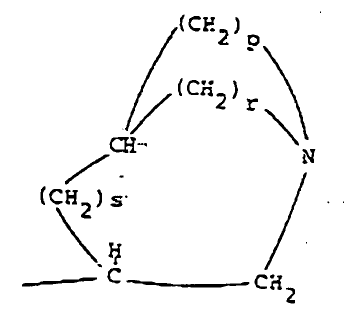
et (i) (p, r, s,) est (2,2,0) ou (2,2,1), alors soit le substituant de faible lipophilie est différent de H ou du groupe méthyle, soit le composé est en configuration endo ; ou
(ii) (p, r, s) est (2,1,0) (2,1,1) ou (3,1,0) alors, sauf dans le cas des 3-(5-(3-méthyl-1,2,4-thiadiazol)-yl)-1-azabicyclo(2.2.1)heptane ; 3-(5-(3-éthyl-1,2,4-thiadiazol)-yl)-1-azabicyclo(2.2.1)heptane ; 6-(5-(3-méthyl-1,2,4-thiodiazol)-yl)-1-azabicyclo-(3.2.1)octane ; soit le substituant de faible lipophilie est différent de H ou d'un groupe alkyle en C₁₋₂, soit le composé est en configuration endo.

2. Composé selon la revendication 1, dans lequel le cycle non aromatique est azacyclique.

3. Composé selon la revendication 2, représenté par la formule développée IA, IB ou IC : 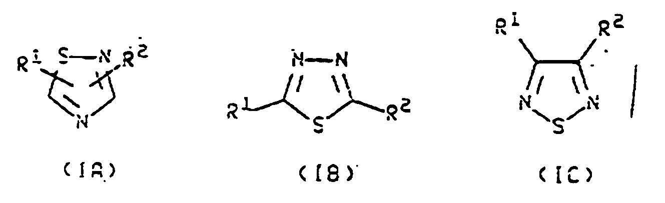 ou un de ses sels ou promédicaments tels que définis dans la revendication 1 ; dans laquelle
R¹ représente un systeme cyclique azacyclique non aromatique ;
et R² représente un atome d'hydrogène ou d'halogène ou un groupe -CF₃, -OR⁷, -NR⁷R⁸, -NHOR⁷, -NHNH₂, -CN, -CO₂R⁷, -CONR⁷R⁸, ou un substituant hydrocarboné ; où R⁷ et R⁸ représentent indépendamment un atome d'hydrogène ou un groupe alkyle en C₁₋₂.

4. Composé selon la revendication 2, représenté par la formule développée IA, IB ou IC : 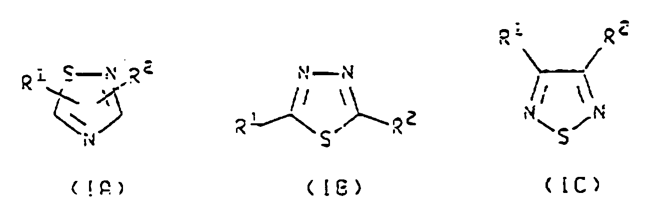 ou un de ses sels ou promédicaments, dans laquelle
R¹ représente un système cyclique azacyclique non aromatique ;
et R² représente un atome d'hydrogène ou d'halogène ou un groupe -CF₃, -OR⁷, NR⁷R⁸, -NHOR⁷, -NHNH₂, -CN, -CO₂R⁷, -CON⁷R⁸, alcényle en C₂₋₅, alcynyle en C₂₋₅, alkyle en C₁₋₂ ou alkyle en C₁₋₂ substitué par -OR⁷, -NR⁷R⁸, -SR⁷, -CO₂R⁷, -CONR⁷R⁸ ou un atome d'halogène ; où R⁷ et R⁸ représentent indépendamment un atome d'hydrogène ou un groupe alkyle en C₁₋₂.

5. Composé selon la revendication 4, choisi parmi les :
3-(5-(3-mèthyl-1,2,4-thiadiazol)-yl)pyrrolidine ;
1-méthyl-3-(5-(3-méthyl-1,2,4-thiadiazol)-yl)pyrrolidine ;
1-méthyl-3-(5-(3-amino-1,2,4-thiadiaxol)-yl)pyrrolidine ;
1-méthyl-3-(5-(3-amino-1,2,4-thiadiazol)-yl)-1,2,5,6-tétrahydropyridine ;
3-(5-(3-amino-1,2,4-thiadiazol)-yl)-1,2,5,6-tétrahydropyridine ;
3-(5-(3-éthyl-1,2,4-thiadiazol)-yl)-1,2,5,6-tétrahydropyridine ;
3-(5-(3-méthoxy-1,2,4-thiadiazol)-yl)-1,2,5,6-tétrahydropyridine ;
3-(5-(3-éthoxy-1,2,4-thiadiazol)-yl)-1,2,5,6-tétrahydropyridine ;
3-(5-(3-chloro-1,2,4-thiadiazol)-yl)-1,2,5,6-tétrahydropyridine ;
3-(5-(3-méthylamino-1,2,4-thiadiazol)-yl)-1,2,5,6-tètrahydropyridine ; et
3-(5-(3-éthylamino-1,2,4-thiadiazol)-yl)-1,2,5,6-tétrahydropyridine ;
1-méthyl-3-(5-(3-méthyl-1,2,4-thiadiazol)-yl)-1,2,5,6-tétrahydropyridine ;
3-(5-(3-méthyl-1,2,4-thiadiazol)-yl)-1,2,5,6-tétrahydropyridine ;
3-(5-(3-diméthylamino-1,2,4-thiadiazol)-yl)-1,2,5,6-tétrahydropyridine ;
et leurs sels et promédicaments.

6. Composé selon la revendication 4, choisi parmi les :
3-(5-(3-diméthylamino-1,2,4-thiadiazol)-yl)-1,2,5,6-tétrahydropyridine ;
3-(5-(3-n-propyl-1,2,4-thiadiazol)-yl)-1,2,5,6-tétrahydropyridine ;
3-(5-(3-cyclopropyl-1,2,4-thiadiazol)-yl)-1,2,5,6-tétrahydropyridine ;
3-(5-(3-benzyl-1,2,4-thiadiazol)-yl)-1,2,5,6-tétrahydropyridine ;
1-méthyl-3-(5-(3-n-propyl-1,2,4-thiadiazol)-yl)-1,2,5,6-tétrahydropyridine ;
1-méthyl-3-(5-(3-cyclopropyl-1,2,4-thiadiazol)-yl)-1,2,5,6-tètrahydropyridine ;
1-méthyl-3-(5-(3-benzyl-1,2,4-thiadiazol)-yl)-1,2,5,6-tétrahydropyridine ;
1-méthyl-3-(5-(3-isopropyl-1,2,4-thiadiazol)-yl)-1,2,5,6-tétrahydropyridine ; et
3-(5-(3-isopropyl-1,2,4-thiadiazol)-yl)-1,2,5,6-tétrahydropyridine ;
et leurs sels et promédicaments.

7. Composé selon la revendication 1, dans lequel le cycle non aromatique est azabicyclique.

8. Composé selon la revendication 7, représenté par la formule développée IA, IB ou IC : 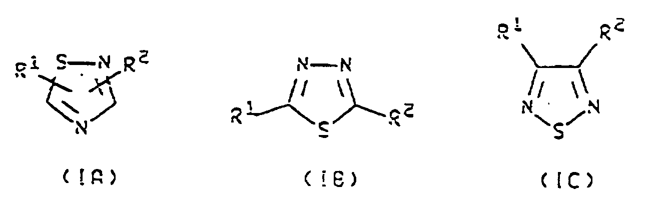 ou un de ses sels ou promédicaments tels que définis dans la revendication 1 ; dans laquelle
R¹ représente un système cyclique azabicyclique non aromatique ;
et R² un atome d'hydrogène ou d'halogène ou un groupe -CF₃, -OR⁷, -NR⁷R⁸, -NHOR⁷, -NHNH₂, CN, -CO₂R⁷, -CONRR⁷R⁸ ou un substituant hydrocarboné ; où R⁷ et R⁸ représentent indépendamment un atome d'hydrogène ou un groupe alkyle en C₁₋₂.

9. Composé selon la revendication 7 ou la revendication 8, dans lequel le système azabicyclique est choisi parmi : 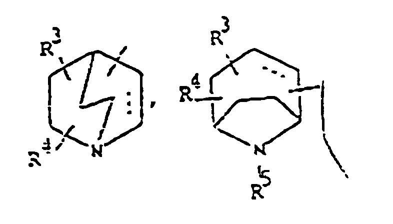 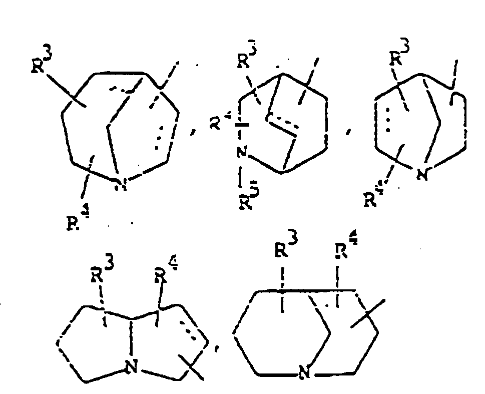 où la ligne en traits interrompus représente une liaison chimique facultative ;
les substituants R³ et R⁴ représentent indépendamment un atome d'hydrogène ou d'halogène ou un groupe alkyle en C₁₋₄, alcoxy en C₁₋₄, hydroxyle ou carboxyle ; ou R³ et R⁴, conjointement avec de l'atome de carbone auquel ils sont fixés, représentent le groupe carbonyle ;
le groupe R⁵ représente un atome d'hydrogène ou un groupe alkyle en C₁₋₄ ; et l'atome d'azote dans le système cyclique azabicyclique porte un doublet libre d'électrons.

10. Composé selon la revendication 7 ou 8, dans lequel le système azabicyclique est choisi parmi : 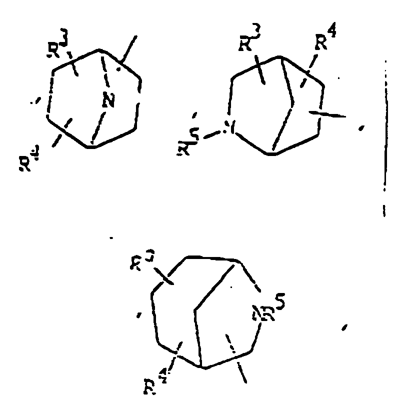 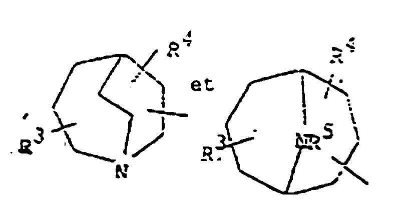 où la ligne en traits interrompus représente une liaison chimique facultative :
les substituants R³ et R⁴ représentent indépendamment un atome d'hydrogène ou d'halogene ou un groupe alkyle en C₁₋₄, alcoxy en C₁₋₄, hydroxyle ou carboxy ; ou R³ et R⁴, conjointement avec l'atome de carbone auquel ils sont fixés, représentent le groupe carbonyle ;
le groupe R⁵ reprèsente un atome d'hydrogène ou un groupe alkyle en C₁₋₄ ; et l'atome d'azote dans le système cyclique azabicylique porte un doublet libre d'électrons.

11. Composé selon l'une quelconque des revendications 7 à 10, représenté par la formule développée IA, IB ou IC : 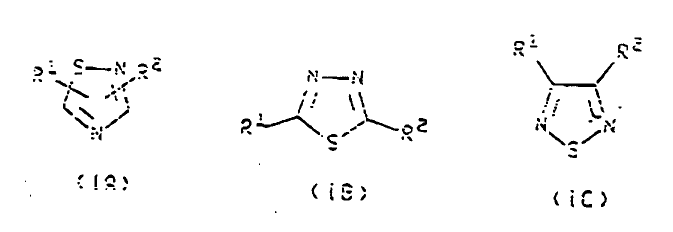 ou un de ses sels ou promédicaments ; dans laquelle
R¹ représente un systeme cyclique azabicylique aromatique ; et
R² représente un atome d'hydrogène ou d'halogène, ou un groupe -CF₃, -OR⁷, -NR⁷R⁸, -NHOR⁷, -NHNH₂, -CN, -CO₂R⁷, -CONR⁷R⁸, alcényle en C₂₋₅, alcynyle en C₂₋₅, alkyle en C₁₋₂ ou alkyle en C₁₋₂ substitué par un groupe -OR⁷, -NR⁷R⁸, -SR⁷, -CO₂R⁷, -CONR⁷R⁸ ou halogéno ; ou R⁷ et R⁸ représentent indépendament un atome d'hydrogène ou un groupe alkyle en C₁₋₂.

12. Composé selon la revendication 7, choisi parmi les:
3-(5-(3-méthyl-1,2,4-thiadiazol)-yl)-1-azabicyclo(2.2.1)heptane ;
3-(5-(3-amino-1,2,4-thiadiazol)-yl)-quinuclidine ;
6-(5-(3-méthyl-1,2,4-thiadiazol)-yl)-1-azabicyclo(3.2.1)octane ;
6-(5-(3-amino-1,2,4-thiadiazol)-yl)-1-azabicyclo(3.2.1)octane ;
3-(5-(3-amino-1,2,4-thiadiazol)-yl)-1-azabicyclo(2.2.1)heptane ;
5-(5-(3-méthyl-1,2,4-thiadiazol)-yl)quinuclidine-3-ol ;
5-(5-(3-amino-1,2,4-thiadiazol)-yl)-1-quinuclidin-3-ol ;
5-méthyl-3-(5-(3-méthyl-1,2,4-thiadiazol)-yl)quinuclidine ;
5-méthyl-3-(5-(3-amino-1,2,4-thiadiazol)-yl)quinuclidine ; et
leurs sels et promédicaments.

13. Composé selon la revendication 7, choisi parmi les :
3-(5-(3-phényl-1,2,4-thiadiazol)-yl)quinuclidine ;
3-(5-(3-cyclopropyl-1,2,4-thiadiazol)-yl)quinuclidine ;
exo- et endo-3-(5-(3-cyclopropyl-1,2,4-thiadiazol)-yl)-1-azabicyclo(2.2.1)heptane ;
3-(5-(3-benzyl-1,2,4-thiadiazol)-yl)quinuclidine ;
3-(5-(3-tert-butyl-1,2,4-thiadiazol)-yl)quinuclidine ;
3-(5-(3-iso-propyl-1,2,4-thiadiazol)-yl)quinuclidine ;
3-(5-(3-(1-hydroxy-1-phénylméthyl)-1,2,4-thiadiazol)-yl)quinuclidine ;
3-(5-(3-(benzoyl-1,2,4-thiadiazol)-yl)quinuclidine ;
3-(5-(3-(1,1-diphényl-1-hydroxyméthyl)-1,2,4-thiadiazol)-yl)quinuclidine ;
3-(5-(3-(1,1-diphényl-1-fluorométhyl)-1,2,4-thiadiazol)-yl)quinuclidine ;
exo- et endo3-(5-(3-iso-propyl-1,2,4-thiadiazol)-yl)-1-azabicyclo-(2.2.1)heptane ;
(1R*,6R*) et (1R*, 6S*) 6-(5-(3-cyclopropyl-1,2,4-thiadiazol)-yl)--2-azabicyclo(2.2.2)octane ;
exo- et endo-3-(5-(3-n-propyl-1,2,4-thiadiazol)-yl)-1-azabicyclo-(2.2.1)heptane ;
3-(5-(3-n-propyl-1,2,4-thiadiazol)-yl)quinuclidine ;
(1R*,6R*) et (1R*,6S*) 6-(5-(3-iso-propyl-1,2,4-thiadiazol)-yl)-2-azabicyclo(2.2.2)octane ;
exo-3-(5-(3-isopropyl-1,2,4-thiadiazol)-yl)-endo-5-hydroxyl-azabicyclo(2.2.1)heptane ; et
exo- et endo-3-(5-(3-benzyl-1,2,4-thiadiazol)-yl)-1-azabicyclo-(2.2.1)heptane ;
et leurs sels et promédicaments.

14. Composé selon la revendication 7, choisi parmi: l'exo- et l'endo-3-(5-(3-éthyl-1,2,4-thiadiazol)-yl)-1-azabicyclo-(2.2.1)heptane ;
et leurs sels et promédicaments.

15. Composition pharmaceutique comprenant un composé selon l'une quelconque des revendications 1 à 14, en association avec un véhicule pharmaceutiquement acceptable.

16. Composition pharmaceutique selon la revendication 16, qui comprend en outre un antagoniste cholinergique périphérique.

17. Emploi d'un composé selon l'une quelconque des revendications 1 à 14, pour la préparation d'un médicament pour le traitement des troubles neurologiques et mentaux ou pour le traitement des états douloureux graves.

18. Procédé pour la préparation d'un composé selon l'une quelconque des revendications 1 à 14, qui comprend ;
(A) la cyclisation d'un composé de formule III : 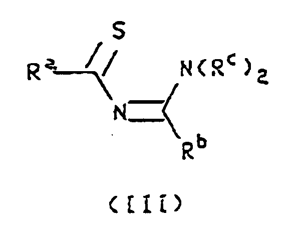 dans laquelle l'un d'entre R^{a} et R^{b} est un système cyclique azacyclique ou azabicyclique non aromatique, et l'autre est un substituant de faible lipophilie ou un substituant hydrocarboné tel que défini dans la revendication 1 ; et R^{c} est un atome d'hydrogène ou un groupe alkyle ;
(B) la cyclo-addition d'un sulfure de nitrile R^{a}-C ≡N⁺-S- avec un nitrile de formule R^{b}CN où R^{a} et R^{b} sont tels que définis ci-dessus ;
(C) la réaction d'un thiadiazole de formule IV : 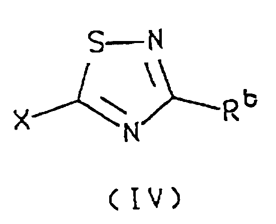 avec un réactif qui fournit un anion ⁻R^{a} où R^{a} et R^{b} sont tels que définis ci-dessus et X représente un atome d'halogène ;
(D) la réaction d'une diamine du type 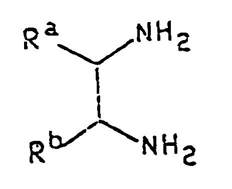 où R^{a} et R^{b} sont tels que définis ci-dessus, avec un chlorure de soufre ; ou
(E) la déshydratation d'un thiosemicarbazide de formule R^{x}CSNHNHCONR^{P}R^{q}, où R^{x} est un système cyclique azacyclique ou azabicyclique non aromatique et R^{P} et R^{q} représentent indépendamment un atome d'hydrogène ou un groupe alkyle.

19. 3-(5-(3-benzoyl-1,2,4-thiadiazol)-yl)quinuclidine et ses sels et promédicaments.

## Revendications (Revendications pour l'(les) Etat(s) contractant(s) suivant(s): ES, GR)

1. Procédé pour la préparation d'un thiadiazole ou un de ses sels ou promédicaments, thiadiazole qui est substitué sur un de ses atomes de carbone cyclique par un système cyclique azacyclique ou azabicyclique non aromatique, contenant de 4 à 10 atomes de carbone, plus un atome d'azote à titre de seul hétéroatome ; et qui est substituè sur l'autre atome cyclique par un substituant de faible lipophilie, possédant une valeur f de rekker non supérieure à 1,5, ou un substituant hydrocarboné substitué ou non substituè choisi dans le groupe comprenant les groupes alkyle en C₁₋₁₅, alcényle en C₂₋₁₅, alcynyle en C₂₋₁₅, phényle, naphtyle, benzyle ou phényl(alkyle en C₁₋₃),
étant entendu que,
lorsqu' un système cyclique azabicylique est 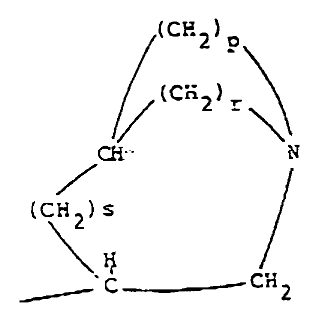
et (i) (p, r, s) est (2,2,0) ou (2,2,1), alors soit le substituant de faible lipophilie est différent de H ou du groupe méthyle, soit le composé est en configuration endo ; ou
(ii) (p, r, s) est (2,1,0), (2,1,1) ou (3,1,0) alors, sauf dans le cas des 3-(5-(3-méthyl-1,2,4-thiadiazol)-yl)-1-azabicyclo(2.2.1)heptane ; 3-(5-(3-éthyl-1,2,4-thiadiazol)-yl)-1-azabicyclo(2.2.1)heptane ; 6-(5-(3-méthyl-1,2,4-thiadiazol)-yl)-1-azabicyclo(3.2.1)octane ; soit le substituant de faible lipophilie est différent de H ou d'un groupe alkyle en C₁₋₂, soit le composé est en configuration endo ; le procédé comprenant
(A) la cyclisation d'un composé de formule III : 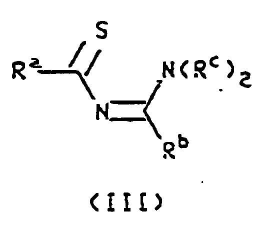 dans laquelle l'un d'entre R^{a} et R^{b} est un système cyclique azacyclique ou azabicyclique non aromatique, et l'autre est un substituant de faible lipophilie ou un substituant hydrocarboné tel que défini ci-dessus : et R^{c} est un atome d'hydrogène ou un groupe alkyle ; ou
(B) la cyclo-addition d'un sulfure de nitrile R^{a}-C =N⁺-S- avec un nitrile de formule R^{b}CN où R^{a} et R^{b} sont tels que définis ci-dessus ; ou
(C) la réaction d'un thiadiazole de formule IV : 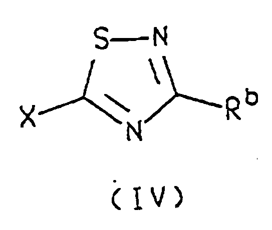 avec un réactif qui fournit un anion ⁻R^{a} où R^{a} et R^{b} sont tels que définis ci-dessus et X représente un atome d'halogène ; ou
(D) la réaction d'une diamine du type 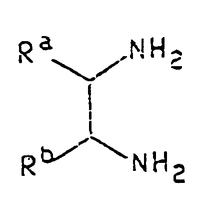 où R^{a} et R^{b} sont tels que définis ci-dessus, avec un chlorure de soufre; ou
(E) la déshydratation d'un thiosemicarbazide de formule R^{x}CSNHNHCONR^{P}R^{q} où R^{x} est un système cyclique azacyclique ou azebicyclique non aromatique tel que défini ci-dessus et R^{P} et R^{q} représentent un atome d'hydrogène ou un groupe alkyle.

2. Procédé selon la revendication 1, dans lequel le cycle non aromatique est azacyclique.

3. Procédé selon la revendication 2, dans lequel le thiadiazole est représenté par la formule développée IA, IB ou IC : 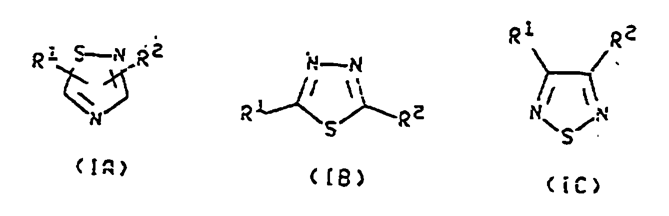 ou un de ses sels ou promédicaments tels que définis dans la revendication 1 ; dans laquelle
R¹ représente un système cyclique azacyclique non aromatique ;
et R² représente un atome d'hydrogène ou d'halogène ou un groupe -CF₃, -OR⁷, -NR⁷R⁸, -NHOR⁷, -NHNH₂, -CN, -CO₂R⁷, -CONR⁷R⁸, ou un substituant hydrocarboné ; où R⁷ et R⁸ représentent indépendamment un atome d'hydrogène ou un groupe alkyle en C₁₋₂.

4. Procédé selon la revendication 2, dans lequel le thiadiazole est représenté par la formule développée IA, IB ou IC : 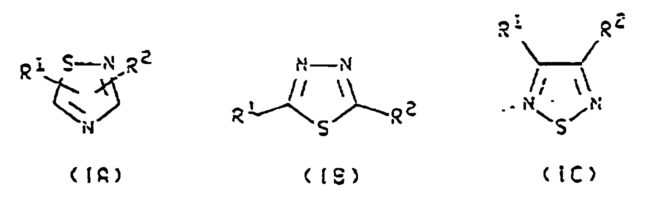 ou un de ses sels ou promédicaments, dans laquelle
R¹ représente un système cyclique azacyclique non aromatique ;
et R² représente un atome d'hydrogène, ou d'halogène ou un groupe -CF₃, -OR⁷, NR⁷R⁸, -NHOR⁷, -NHNH₂, -CN, -CO₂R⁷, -CON⁷R⁸, alcényle en C₂₋₅, alcynyle en C₂₋₅, alkyle en C₁₋₂ ou alkyle en C₁₋₂ substitué par -OR⁷, -NR⁷R⁸, -SR⁷, -CO₂R⁷, -CONR⁷R⁸ ou un atome d'halogène ; où R⁷ et R⁸ représentent indépendamment un atome d'hydrogène ou un groupe alkyle en C₁₋₂.

5. Procédé selon la revendication 4, dans lequel le thiadiazole est choisi parmi les :
3-(5-(3-méthyl-1,2,4-thiadiazol)-yl)pyrrolidine ;
1-méthyl-3-(5-(3-méthyl-1,2,4-thiadiazol)-yl)pyrrolidine ;
1-méthyl-3-(5-(3-amino-1,2,4-thiadiazol)-yl)pyrrolidine ;
1-méthyl-3-(5-(3-amino-1,2,4-thiadiazol)-yl)-1,2,5,6-tétrahydropyridine ;
3-(5-(3-amino-1,2,4-thiadiazol)-yl)-1,2,5,6-tétrahydrprridine ;
3-(5-(3-éthyl-1,2,4-thiadiazol)-yl)-1,2,5,6-tétrahydropyridine ;
3-(5-(3-méthoxy-1,2,4-thiadiazol)-yl)-1,2,5,6-tétrahydropyridine ;
3-(5-(3-éthoxy-1,2,4-thiadiazol)-yl)-1,2,5,6-tètrahydropyridine ;
3-(5-(3-chloro-1,2,4-thiadiazol)-yl)-1,2,5,6-tétrahydropyridine ;
3-(5-(3-méthylamino-1,2,4-thiadiazol)-yl)-1,2,5,6-tétrahydropyridine ; et
3-(5-(3-éthylamino-1,2,4-thiadiazol)-yl)-1,2,5,6-tétrahydropyridine ;
1-méthyl-3-(5-(3-méthyl-1,2,4-thiadiazol)-yl)-1,2,5,6-tétrahydropyridine ;
3-(5-(3-méthyl-1,2,4-thiadiazol)-yl)-1,2,5,6-tétrahydropyridine ;
3-(5-(3-diméthylamino-1,2,4-thiadiazol)-yl)-1,2,5,6-tétrahydropyridine ;
et leurs sels et promédicaments.

6. Procédé selon la revendication 4, dans lequel le thiadiazole est choisi parmi les :
3-(5-(3-diméthylamino-1,2,4-thiadiazol)-yl)-1,2,5,6-tétrahydropyridine ;
3-(5-(3-n-propyl-1,2,4-thiadiazol)-yl)-1,2,5,6-tétrahydropyridine ;
3-(5-(3-cyclopropyl-1,2,4-thiadiazol)-yl)-1,2,5,6-tétrahydropyridine ;
3-(5-(3-benzyl-1,2,4-thiadiazol)-yl)-1,2,5,6-tétrahydropyridine ;
1-méthyl-3-(5-(3-n-propyl-1,2,4-thiadiazol)-yl)-1,2,5,6-tétrahydropyridine ;
1-méthyl-3-(5-(3-cyclopropyl-1,2,4-thiadiazol)-yl)-1,2,5,6-tétrahydropyridine ;
1-méthyl-3-(5-(3-benzyl-1,2,4-thiadiazol)-yl)-1,2,5,6-tétrahydropyridine ;
1-méthyl-3-(5-(3-isopropyl-1,2,4-thiadiazol)-yl)-1,2,5,6-tétrahydropyridine ; et
3-(5-(3-isopropyl-1,2,4-thiadiazol)-yl)-1,2,5,6-tétrahydropyridine ;
et leurs sels et promédicaments.

7. Procédé selon la revendication 1, dans lequel le cycle non aromatique est azabicyclique.

8. Procédé selon la revendication 7, dans lequel le thiadiazole est représenté par la formule développée IA, IB ou IC : 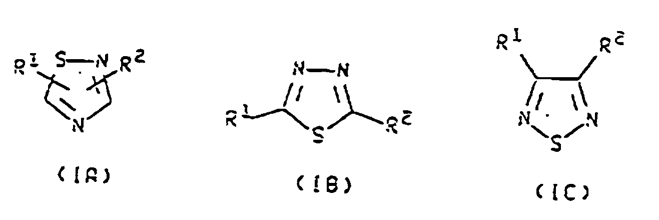 ou un de ses sels ou promédicaments tels que définis dans la revendication 1 ; dans laquelle
R¹ représente un système cyclique azabicyclique non aromatique ;
et R² un atome d'hydrogène ou d'halogène ou un groupe -CF₃, -OR⁷, -NR⁷R⁸, -NHOR⁷, -NHNH₂, -CN, -CO₂R⁷, -CONRR⁷R⁸ ou un substituant hydrocarboné ; où R⁷ et R⁸ représentent indépendamment un atome d'hydrogène ou un groupe alkyle en C₁₋₂.

9. Procédé selon la revendication 7 ou 8, dans lequel le système azabicyclique est choisi parmi : 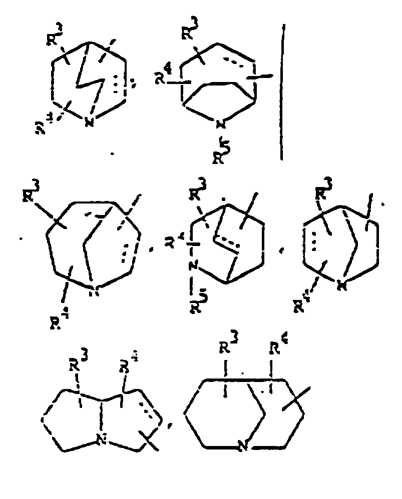 où la ligne en traits interrompus représente une liaison chimique facultative ;
les subetituants R³ et R⁴ représentent indépendamment un atome d'hydrogène ou d'halogène ou un groupe alkyle en C₁₋₄, alcoxy en C₁₋₄, hydroxyle ou carboxyle ; ou R³ et R⁴, conjointement avec de l'atome de carbone auquel ils sont fixés,représentent le groupe carbonyle ;
le groupe R⁵ représente un atome d'hydrogène ou un groupe alkyle en C₁₋₄ ; et l'atome d'azote dans le système cyclique azabicyclique porte un doublet libre d'électrons.

10. Procédé selon la revendication 7 ou 8, dans lequel le système azabicyclique est choisi parmi : 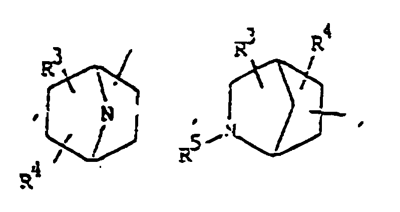 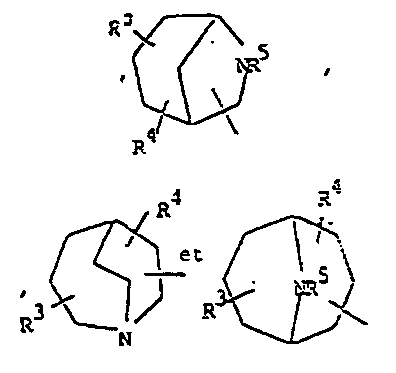 où la ligne en traits interrompus représente une liaison chimique facultative ;
les substituants R³ et R⁴ représentent indépendamment un atome d'hydrogène ou d'halogène ou un groupe alkyle en C₁₋₄, alcoxy en C₁₋₄, hydroxyle ou carboxy ; ou R³ et R⁴, conjointement avec l'atome de carbone auquel ils sont fixés, représentent le groupe carbonyle ;
le groupe R⁵ représente un atome d'hydrogène ou un groupe alkyle en C₁₋₄ ; et l'atome d'azote dans le système cyclique azabicylique porte un doublet libre d'électrons.

11. Procédé selon l'une quelconque des revendications 7 à 10, dans lequel le thiadiaiole est représenté par la formule développée IA, IB ou IC : 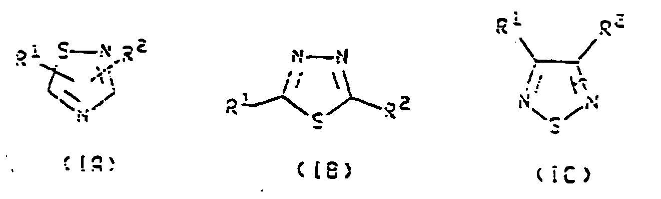 ou un de ses sels ou promédicaments ; dans laquelle
R¹ représente un systeme cyclique azabicylique aromatique ; et
R² reprèsente un atome d'hydrogène ou d'halogène, ou un groupe -CF₃, -OR⁷, -NR⁷R⁸, -NHOR⁷, -NHNH₂, -CN, -CO₂R⁷, -CONR⁷R⁸, alcényle en C₂₋₅, alcynyle en C₂₋₅, alkyle en C₁₋₂ ou alkyle en C₁₋₂ substitué par un groupe -OR⁷, -NR⁷R⁸, -SR⁷, -CO₂R⁷, -CONR⁷R⁸ ou halogéno ; où R⁷ et R⁸ représentent indépendamment un atome d'hydrogène ou un groupe alkyle en C₁₋₂.

12. Procédé selon la revendication 7, dans lequel le thiadiazole est choisi parmi les :
3-(5-(3-méthyl-1,2,4-thiadiazol)-yl)-1-azabicyclo(2.2.1)heptane ;
3-(5-(3-Amino-1,2,4-thiazole)-yl)quinuclidine ;
6-(5-(3-méthyl-1,2,4-thiadiazol)-yl)-1-azabicyclo(3.2.1)octane ;
6-(5-(3-amino-1,2,4-thiadiazol)-yl)-1-azabicyclo(3.2.1)octane ;
3-(5-(3-amino-1,2,4-thiadiazol)-yl)-1-azabicyclo(2.2.1)heptane ;
5-(5-(3-méthyl-1,2,4-thiadiazol)-yl)quinuclidine-3-ol ;
5-(5-(3-amino-1,2,4-thiadiazol)-yl)-1-quinuclidin-3-ol ;
5-méthyl-3-(5-(3-méthyl-1,2,4-thiadiazol)-yl)quinuclidine ;
5-méthyl-3-(5-(3-amino-1,2,4-thiadiazol)-yl)quinuclidine ; et
et leurs sels et promédicaments.

13. Procédé selon la revendication 7, dans lequel le thiadiazole est choisi parmi les :
3-(5-(3-phényl-1,2,4-thiadiazol)-yl)quinuclidine ;
3-(5-(3-cyclopropyl-1,2,4-thiadiazol)-yl)quinuclidine ;
exo- et endo-3-(5-(3-cyclopropyl-1,2,4-thiadiazol)-yl)-1-azabicyclo(2.2.1)heptane ;
3-(5-(3-benzyl-1,2,4-thiadiazol)-yl)quinuclidine ;
3-(5-(3-tert-butyl-1,2,4-thiadiazol)-yl)quinuclidine ;
3-(5-(3-iso-propyl-1,2,4-thiadiazol)-yl)quinuclidine ;
3-(5-(3-(1-hydroxy-1-phénylméthyl)-1,2,4-thiadiazol)-yl)quinuclidine ;
3-(5-(3-(benzoyl-1,2,4-thiadiazol)-yl)quinuclidine ;
3-(5-(3-(1,1-diphényl-1-hydroxyméthyl)-1,2,4-thiadiazol)-yl)quinuclidine ;
3-(5-(3-(1,1-diphényl-1-fluorométhyl)-1,2,4-thiadiazol)-yl)quinuclidine ;
exo- et endo3-(5-(3-iso-propyl-1,2,4-thiadiazol)-yl)-1-azabicyclo-(2.2.1)heptane ;
(1R*,6R*) et (1R*,6S*) 6-(5-(3-cyclopropyl-1,2,4-thiadiazol)-yl)-2-azabicyclo(2.2.2)octane ;
exo- et endo-3-(5-(3-n-propyl-1,2,4-thiadiazol)-yl)-1-azabicyclo-(2.2.1)heptane ;
3-(5-(3-n-propyl-1,2,4-thiadiazol)-yl)quinuclidine ;
(1R*,6R*) et (1R*,6S*) 6-(5-(3-iso-propyl-1,2,4-thiadiazol)-yl)-2-azabicyclo(2.2.2)octane ;
exo-3-(5-(3-isopropyl-1,2,4-thiadiazol)-yl)-endo-5-hydroxyl-azabicyclo(2.2.1)heptane ; et
exo- et endo-3-(5-(3-benzyl-1,2,4-thiadiazol)-yl)-1-azabicyclo-(2.2.1)heptane ;
et leurs sels et promédicaments.

14. Procédé selon la revendication 7, dans lequel le thiadiazole est choisi parmi les:
exo- et endo-3-(5-(3-éthyl-1,2,4-thiadiazol)-yl)-1-azabicyclo-(2.2.1)heptane ;
et leurs sels et promédicaments.

15. Procédé pour la préparation d'une composition pharmaceutique qui consiste à amener un composé préparé conformément à l'une quelconque des revendications 1 à 14, en association avec un véhicule pharmaceutiquement acceptable.

16. Procédé selon la revendication 15, qui comprend en outre l'incorporation d'un antagoniste cholinergique périphérique.

17. Utilisation d'un composé selon l'une quelconque des revendicatîons 1 à 14, pour la préparation d'un médicament pour le traitement des troubles neurologiques et mentaux ou pour le traitement des états douloureux graves.

18. Procédé selon la revendication 1, pour la préparation de la 3-(5-(3-benzoyl-1,2,4-thiadiazol)-yl)quinuclidine.
